(19) Europäisches Patentamt
European Patent Office
Office européen des brevets

(11) **EP 1 097 138 B1**

(12) # FASCICULE DE BREVET EUROPEEN

(45) Date de publication et mention de la délivrance du brevet: **07.12.2005 Bulletin 2005/49**

(21) Numéro de dépôt: **99931343.0**

(22) Date de dépôt: **13.07.1999**

(51) Int Cl.[7]: **C07D 215/22**, A61K 31/47, C07D 215/36, C07D 405/06, C07D 455/04, C07D 215/38, C07D 401/12, C07D 215/26

(86) Numéro de dépôt international: **PCT/FR1999/001716**

(87) Numéro de publication internationale: **WO 2000/003990 (27.01.2000 Gazette 2000/04)**

(54) **COMPOSITIONS PHARMACEUTIQUES COMPRENANT DES 2-QUINOLONES**

2-CHINOLONEN ENTHALTENDE ARZNEIMITTEL

PHARMACEUTICAL COMPOSITIONS COMPRISING 2-QUINOLONES

(84) Etats contractants désignés:
**AT BE CH CY DE DK ES FI FR GB GR IE IT LI LU MC NL PT SE**
Etats d'extension désignés:
**LT LV RO**

(30) Priorité: **15.07.1998 FR 9809060**

(43) Date de publication de la demande: **09.05.2001 Bulletin 2001/19**

(73) Titulaire: **LABORATOIRE L. LAFON**
**94701 Maisons Alfort (FR)**

(72) Inventeurs:
- **JOSEPH, Benoît, I.C.O.A. CNRS, Univ. d'Orléans 45067 Orléans Cedex 2 (FR)**
- **DARRO, Francis, Univ. Bruxelles, Faculté Médecine 1070 Bruxelles (BE)**
- **GUILLAUMET, Gérald, I.C.O.A. CNRS Univ. d'Orléans 45067 Orléans Cedex 2 (FR)**
- **KISS, Robert, Univ. Bruxelles, Faculté de Médeci. 1070 Bruxelles (BE)**
- **FRYDMAN, Armand, Laboratoire L. Lafon 94701 Maisons Alfort (FR)**

(74) Mandataire: **Bernasconi, Jean Raymond et al**
**c/o Cabinet Lavoix,**
**2, Place d'Estienne d'Orves**
**75441 Paris Cedex 09 (FR)**

(56) Documents cités:
**EP-A- 0 024 638**
**WO-A-93/11115**
**WO-A-94/02145**
**US-A- 5 726 184**

- **CHEMICAL ABSTRACTS, vol. 82, no. 3, 20 janvier 1975 (1975-01-20) Columbus, Ohio, US; abstract no. 16708r, KAMETANI, TETSUJI ET AL: "Quinoline derivatives." XP002095829 -& DATABASE CHEMICAL ABSTRACTS [Online] CA 82:16708, XP002095885 & JP 07 476876 A (JAPAN CHEMIPHA CO., LTD.)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 097 138 B1

## Description

La présente invention concerne des compositions pharmaceutiques comprenant des 2-quinolones ou des composés dérivés.

EP-A-0 024 638 décrit déjà l'utilisation de certaines. 2-quinolones en thérapeutique pour augmenter la synthèse du glucose. WO-A-93/11115 décrit l'utilisation de 2-quinolones, essentiellement à substitution 7-chloro comme antagonistes des récepteurs NMA. En outre, WO-A-94/02145 décrit l'utilisation de 2-aryl-4-quinolones comme antitumoraux.

Un cancer est un désordre des gènes somatiques au cours duquel des dysfonctionnements génétiques s'amplifient au fur et à mesure que le processus tumoral progresse de l'état de lésion précancéreuse à celui dé transformation maligne, la tumeur cancéreuse devenant métastasique et souvent résistante aux médicaments cytotoxiques.

En dépit des efforts très importants conduits dans tous les pays développés, en particulier à travers des programmes de recherche expérimentale et clinique, la mortalité due aux différents cancers (tumeurs solides et néoplasies hématologiques) demeure inacceptablement élevée. Dans de nombreux pays, la mortalité par cancer est au second rang, juste après les maladies cardio-vasculaires.

En termes de cancers nouvellement diagnostiqués, la répartition entre tumeurs solides et néoplasies hématologiques (moëlle osseuse, sang, système lymphatique) montre que 9 cancers sur 10 sont des tumeurs solides. Au contraire de ce qui est observé en oncologie hématologique (succès thérapeutiques dans 40 à 90 % des cancers des cellules du sang), seulement un petit nombre de tumeurs solides avancées ou disséminées répondent aux seuls traitements chimiothérapeutiques. C'est en partie pour cette raison que la mortalité globale par cancer a cru aux U.S.A. entre 1973 et 1992.

Il n'est malheureusement pas sûr que cette tendance pourra s'inverser seulement par l'apparition, à côté de l'arsenal chimiothérapeutique établi, de nouveaux médicaments antitumoraux tels que les taxanes (paclitaxel et docetaxel) qui interfèrent avec la formation des microtubules (W.P. Mc Guire et al., Am. Intern. Med., 1989), les inhibiteurs de topoisomérases 1 dérivés de la camptothécine (topotecan et irinotecan), la vinorelbine (nouvel alcaloïde issu de la pervenche), la gemcitabine. (nouvel antimétabolique cytotoxique), le raltitrexed (inhibiteur de la thymidylate synthétase) et la miltefosine (premier représentant de la famille des alkyl-lysophospholipides). Ces traitements s'ajoutent, soit en première intention, soit en seconde intention, aux médicaments dont l'activité spécifique est maintenant bien reconnue comme la doxorubicine, le cisplatine, là vincristine, le méthotréxate, le 5-fluorouracile.

Un des plus difficiles problèmes actuels de la chimiothérapie anticancéreuse est dû au fait que de nombreuses populations de cellules malignes présentent une résistance importante aux substances cytotoxiques établies. Le plus souvent cette situation résulte de l'existence de gènes de multi-résistance ou de la fréquence de mutations génétiques chez certains types de tumeurs. Ainsi, le traitement des cancers nécessite de nouvelles approches, complémentaires de celles actuellement mises en oeuvre, et destinées à mieux lutter contre l'extension et l'hétérogénéité de la charge tumorale et l'acquisition de la résistance "multi-drogues cytotoxiques".

Parmi ces nouvelles approches, certaines sont déjà prometteuses. C'est le cas de l'induction de l'apoptose, l'inhibition de l'angiogénèse tumorale et des processus métastasiques sans parler de la thérapie génique ou de l'immunothérapie.

Les inventeurs se sont intéressés à une approche différente. L'objectif recherché était de rendre la population de cellules tumorales plus sensible aux traitements anticancéreux de référence afin d'atteindre un double bénéfice :

1) augmenter l'activité cytotoxique donc l'efficacité et
2) diminuer la fréquence et la sévérité de certains effets secondaires grâce à la réduction de posologie qui pourrait suivre l'induction de l'augmentation de l'efficacité anti-tumorale.

C'est cette stratégie qui est à l'origine de la découverte de compositions capables d'induire une augmentation très significative de l'activité cytotoxique de médicaments anticancéreux éprouvés. Ces compositions ont la capacité soit de stimuler le recrutement de cellules clonogènes au sein de la tumeur rendant celle-ci plus sensible au traitement conventionnel par des agents cytotoxiques, soit d'inhiber la prolifération de cellules clonogènes, contribuant ainsi à la régression de la tumeur.

La présente invention a ainsi pour objet l'utilisation, dans le traitement des cancers avec au moins un antitumoral choisi parmi les agents cytotoxiques, d'un composé ayant une activité sur la prolifération de cellules clonogènes dans les tumeurs, choisi parmi les composés de formule :

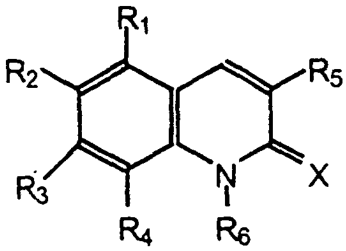 (I) et 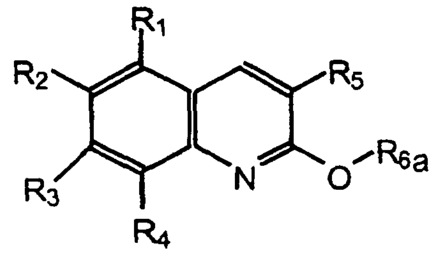 (Ia)

dans laquelle :

X est choisi parmi =O, =S et =N-NH-$R_7$, $R_7$ étant un groupe phényle ou pyridinyle, $R_1$, $R_2$, $R_3$ et $R_4$ sont choisis indépendamment l'un de l'autre parmi H, OH, un groupe alkyl en $C_1$-$C_4$, un groupe alkoxy en $C_1$-$C_4$, un groupe -OCO-$R_8$, $R_8$ étant un groupe alkyle en $C_1$-$C_4$, au moins l'un des substituants $R_1$, $R_2$, $R_3$ ou $R_4$ étant autre que H, et $R_2$ et $R_3$ pouvant former ensemble un groupe méthylènedioxy,
$R_5$ est un groupe phényle ou un groupe phényle 1 à 3 fois substitué par des groupes choisis parmi H, OH, un groupe alkoxy en $C_1$-$C_4$, un groupe -OCO$R_8$, un groupe phényl(alkoxy en $C_1$-$C_4$), un groupe -O-$SO_2$-$R'_8$, $R'_8$ étant un groupe alkyle en $C_1$-$C_4$ ou un groupe $CF_3$, et un groupe dérivé d'un ose,
$R_6$ est choisi parmi un groupe alkyle en $C_1$-$C_4$, un groupe -CO-$R_9$ et un groupe -A-$R_{10}$,
$R_{6a}$ est choisi parmi un groupe alkyle en $C_1$-$C_4$, un groupe -CO-$R_9$ et un groupe -A-$R_{10}$,
$R_9$ étant un groupe alkyle en $C_1$-$C_4$,
A étant un groupe alkylène en $C_1$-$C_4$,
$R_{10}$ étant choisi parmi les groupes hétérocycliques à 5 ou 6 chaînons ayant 1 à 4 hétéroatomes choisis parmi l'oxygène, le soufre et l'azote, le groupe CN, un groupe - COO$R_{11}$, -CON$R_{12}R_{13}$, un groupe -N$R_{14}R_{15}$, et un groupe -CO$R_{16}$,
$R_{11}$, $R_{12}$, $R_{13}$, $R_{14}$, $R_{15}$ et $R_{16}$ étant indépendamment choisis parmi un atome d'hydrogène, un groupe alkyle en $C_1$-$C_4$ et un groupe phényl(alkyle en $C_1$-$C_4$),
$R_4$ et $R_6$ pouvant en outre former ensemble un groupe -CO-$CH_2$-$CH_2$-.

Les agents cytotoxiques peuvent être utilisés à leur dose habituelle et, dans ce cas, leur efficacité est améliorée, ou à des doses plus faibles compte tenu de l'augmentation de leur efficacité antitumorale.
Dans une forme de réalisation préférée est utilisé un composé de formule (I) dans laquelle :

- $R_1$ est un groupe alkoxy en $C_1$-$C_4$
- $R_2$ est un atome d'hydrogène
- $R_3$ est un groupe alkoxy en $C_1$-$C_4$
- $R_4$ est un atome d'hydrogène,

et en particulier un composé de formule (I) dans laquelle:

- $R_5$ est un groupe 4-(alkoxy en $C_1$-$C_4$)phényle,
  et tout particulièrement un composé de formule (I) dans laquelle :

- $R_1$ est un groupe méthoxy,
- $R_3$ est un groupe méthoxy, et
- $R_5$ est un groupe 4-méthoxyphényle.

Il a également été découvert qu'au moins certains des composés de formule (I) avaient une activité antitumorale par eux-mêmes.
La présente invention a également pour objet une composition ayant une activité sur la prolifération de cellules clonogènes dans les tumeurs en interférant sur la génération de cellules clonogènes, soit par stimulation de la prolifération et recrutement, soit par inhibition de la prolifération, et qui comprend une quantité efficace d'un composé de formule :

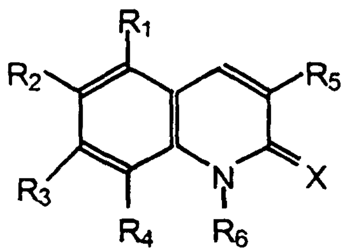 (I) et 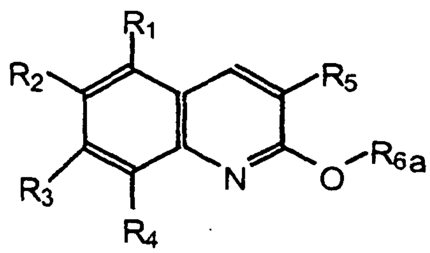 (Ia)

dans laquelle :

X est choisi parmi =O, =S et =N-NH-$R_7$, $R_7$ étant un groupe phényle ou pyridinyle,

$R_1$, $R_2$, $R_3$ et $R_4$ sont choisis indépendamment l'un de l'autre parmi H, OH, un groupe alkyle en $C_1$-$C_4$, un groupe alkoxy en $C_1$-$C_4$, un groupe -OCO-$R_8$, $R_8$ étant un groupe alkyle en $C_1$-$C_4$, au moins l'un des substituants $R_1$, $R_2$, $R_3$ ou $R_4$ étant autre que H, et $R_2$ et $R_3$ pouvant former ensemble un groupe méthylènedioxy,

$R_5$ est un groupe phényle ou un groupe phényle 1 à 3 fois substitué par des groupes choisis parmi H, OH, un groupe alkoxy en $C_1$-$C_4$, un groupe -OCO$R_8$, un groupe phényl(alkoxy en $C_1$-$C_4$), un groupe -O-$SO_2$-$R'_8$, $R'_8$ étant un groupe alkyle en $C_1$-$C_4$ ou un groupe $CF_3$,

$R_6$ est choisi parmi H, un groupe alkyle en $C_1$-$C_4$, un groupe -CO-$R_9$ et un groupe -A-$R_{10}$,

$R_{6a}$ est choisi parmi, un groupe -CO-$R_9$, et un groupe -A-$R_{10}$,

$R_9$ étant un groupe alkyle en $C_1$-$C_4$,

A étant un groupe alkylène en $C_1$-$C_4$,

$R_{10}$ étant choisi parmi les groupes hétérocycliques à 5 ou 6 chaînons ayant 1 à 4 hétéroatomes choisis parmi l'oxygène, le soufre et l'azote, le groupe CN, un groupe - COO$R_{11}$, -CON$R_{12}R_{13}$, un groupe -N$R_{14}R_{15}$, et un groupe -CO$R_{16}$,

$R_{11}$, $R_{12}$, $R_{13}$, $R_{14}$, $R_{15}$ et $R_{16}$ étant indépendamment choisis parmi un atome d'hydrogène, un groupe alkyle en $C_1$-$C_4$ et un groupe phényl(alkyle en $C_1$-$C_4$),

$R_4$ et $R_6$ pouvant en outre former ensemble un groupe -CO-$CH_2$-$CH_2$-.

La présente invention a également pour objet des composés nouveaux, à savoir des composés de formule :

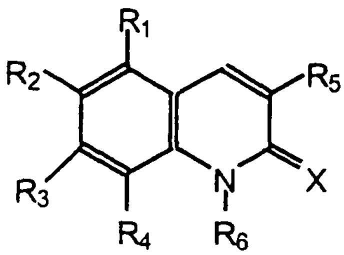 (I) et 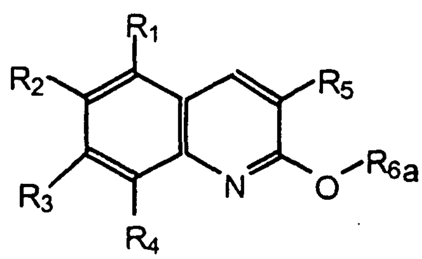 (Ia)

dans laquelle :

X est choisi parmi =O, =S et =N-NH-$R_7$, $R_7$ étant un groupe phényle ou pyridinyle,

$R_1$, $R_2$, $R_3$ et $R_4$ sont choisis indépendamment l'un de l'autre parmi H, OH, un groupe alkyle en $C_1$-$C_4$, un groupe alkoxy en $C_1$-$C_4$, un groupe -OCO-$R_8$, $R_8$ étant un groupe alkyle en $C_1$-$C_4$, au moins l'un des substituants $R_1$, $R_2$, $R_3$ ou $R_4$ étant autre que H, et $R_2$ et $R_3$ pouvant former ensemble un groupe méthylènedioxy.

$R_5$ est un groupe phényle 1 à 3 fois substitué par des groupes choisis parmi OH, un groupe alkoxy en $C_1$-$C_4$, un groupe -OCO$R_8$, un groupe phényl(alkoxy en $C_1$-$C_4$), un groupe -O-$SO_2$-$R'_8$, $R'_8$ étant un groupe alkyle en $C_1$-$C_4$ ou un groupe $CF_3$,

$R_6$ est choisi parmi un groupe alkyle en $C_1$-$C_4$, un groupe -CO-$R_9$ et un groupe -A-$R_{10}$,

$R_{6a}$ est choisi parmi un groupe -CO-$R_9$, et un groupe -A-$R_{10}$,

$R_9$ étant un groupe alkyle en $C_1$-$C_4$,

A étant un groupe alkylène en $C_1$-$C_4$,
$R_{10}$ étant choisi parmi les groupes hétérocycliques à 5 ou 6 chaînons ayant 1 à 4 hétéroatomes choisis parmi l'oxygène, le soufre et l'azote, le groupe CN, un groupe - $COOR_{11}$, -$CONR_{12}R_{13}$, un groupe -$NR_{14}R_{15}$, et un groupe -$COR_{16}$,
$R_{11}$, $R_{12}$, $R_{13}$, $R_{14}$, $R_{15}$ et $R_{16}$ étant indépendamment choisis parmi un atome d'hydrogène, un groupe alkyle en $C_1$-$C_4$ et un groupe phényl(alkyle en $C_1$-$C_4$),
$R_4$ et $R_6$ pouvant en outre former ensemble un groupe -CO-$CH_2$-$CH_2$-,

Dans le traitement chimiothérapeutique des cancers par des agents cytotoxiques, les composés de formule (I) et (Ia) peuvent être administrés au début des traitements chimiothérapeutiques soit en une fois, soit sur plusieurs jours au début de ces traitements (par exemple pendant 5 à 7 jours) et, en fonction du protocole chimiothérapeutique, au début de chaque cycle de traitement (par exemple pendant 2 à 5 jours) au cours de chaque cure.

Les composés de formule (I) et (Ia) sont avantageusement administrés par perfusion (généralement en 1 à 3 heures) à des doses de 5 à 50 mg/kg/jour ou 200 à 2000 mg/$m^2$/jour.

Afin d'obtenir un effet maximal sur la production (inhibition ou stimulation) de cellules clonogènes, les composés de formule (I) et (Ia) doivent être administrés de telle manière que les concentrations tissulaires obtenues soient les plus élevées qu'il est possible d'envisager.

Pour les protocoles de traitement dans les phases aiguës des cures, la voie intraveineuse est à privilégier en utilisant :

- des solutés de perfusion prêts à l'emploi (poches, flacons ...) destinés à être administrés tels quels par perfusion intraveineuse à l'aide d'une ligne de perfusion et selon le débit recommandé :

- des lyophilisats à remettre en solution pour la perfusion intraveineuse à l'aide des solutés pharmaceutiques connus de l'homme de l'art ;
- pour les traitements d'entretien, il est également possible d'envisager la voie orale lorsque le traitement de la chimiothérapie privilégie l'administration de cytostatiques par voie orale. A cette fin, pourront être utilisés des lyocs (pour absorption orale ou *perlinguale),* des comprimés à libération instantanée ou retardée, les solutions orales, les suspensions, les granulés, les gélules ...

Les agents cytotoxiques peuvent être choisis parmi :

i) des agents intercalants, notamment la doxorubicine (Adriamycine), la daunorubicine, l'épirubicine, l'idarubicine, la zorubicine, l'aclarubicine, la pirarubicine, l'acridine, la mitoxanthrone, l'actinomycine D, l'acétate d'eptilinium ;
ii) des agents alkylants choisis parmi les dérivés du platine (cisplatine, carboplatine, oxaliplatine) ;
iii) un composé choisi parmi les autres groupes d'agents alkylants :

- cyclophosphamide, ifosfamide, chlormétrine, melphalan, chlorambucil, estramustine,
- busulfan, mitomycine C,
- nitrosourées : BCNU (carmustine), CCNU (lomustine), fotémustine, streptozotocine,
- triazènes ou dérivés : procarbazine, dacarbazine,
- pipobroman,
- éthylène-imines : altretamine, triéthylène-thiophosphoramide,

iv) un composé choisi parmi les autres groupes d'agents anti-métaboliques :

- antifoliques : méthotrexate, raltitrexed,
- antipyrimidiques : 5-fluorouracil (5-FU), cytarabine (Ara-C),
- hydroxyurée
- antipuriques : purinéthol, thioguanine, pentostatine, cladribine
- inducteurs de la synthèse de nucléosides cytotoxiques : gemcitabine,

v) un composé choisi parmi les autres groupes d'agents tubulo-affins :

- vinca-alcaloïdes désorganisant le fuseau mitotique : vincristine, vinblastine, vindésine, navelbine
- agents bloquant la dépolymérisation du fuseau mitotique : paclitaxel, docetaxel
- agents induisant des cassures de l'ADN par inhibition de la topoisomérase II: étoposide, téniposide
- inhibiteurs de la topoisomérase I induisant des coupures de l'ADN : topotécan, irinotécan,

vi) un agent scindant, fragmentant l'ADN, telle la bléomycine,
vii) un des composés suivants ; plicamycine, L-asparaginase, mitoguazone, dacarbazine,
viii) un stéroïde progestatif anticancéreux : médroxy-progestérone, mégestrol,
ix) un stéroïde oestrogénique anticancéreux : diéthylstilbestrol ; fosfestrol tétrasodique,
x) un anti-oestrogène : tamoxifène, droloxifène, raloxifène, amino-gluthétimide,
xi) un anti-androgène stéroïdien (ex cyprotérone) ou un anti-androgène non stéroïdien (flutamide, nilutamide).

En particulier, les composés de formule (I) et (Ia) peuvent être associés à tous les traitements par les agents cytotoxiques majeurs utilisés dans les polychimiothérapies des tumeurs solides tels :

- la doxorubicine
- les agents alkylants : oxazophorines (cyclophosphamide, ifosfamide, chlorambucil, melphalan)
- les nitrosourées
- la mitomycine C
- les anti-métabolites comme le méthotrexate, le 5-FU, l'Ara-C, la capécitabine
- les agents interférant avec la tubuline : vinca-alcaloïdes (vincristine, vinblastine, vindésine, navelbine), les taxoïdes (paclitaxel, docétaxel), les dérivés des épipodophyllotoxines (étoposide, téniposide)
- la bléomycine
- les inhibiteurs de la topoisomérase I : topotécan, irinotécan.

De même, les composés de formule (I) et (Ia) peuvent être associés aux traitements par les agents cytotoxiques majeurs utilisés en oncohématologie pour le traitement des cancers du sang :

- maladie de Hodgkin : cyclophosphamide, mechloréthamine, chlorambucil, melphalan, ifosfamide, étoposide, doxorubicine, daunorubicine ;
- leucémies aiguës : méthotrexate, 6-mercaptopurine, cytarabine, vinblastine, vincristine, doxorubicine, daunorubicine, L-asparaginase ;
- lymphomes malins non hodgkiniens : mechloréthamine, chlorambucil, cyclophosphamide, melphalan, ifosfamide, methotrexate, cytarabine, vinblastine, vincristine, étoposide, doxorubicine, daunorubicine, carmustine, iomustine, cisplatine ;
- leucémies lymphoïdes chroniques : méchlorétamine, chlorambucil, cyclophosphamide, melphalan, ifosfamide.

D'une manière générale, les composés de formule (I) peuvent être préparés selon les schémas réactionnels suivants :

SCHEMA I

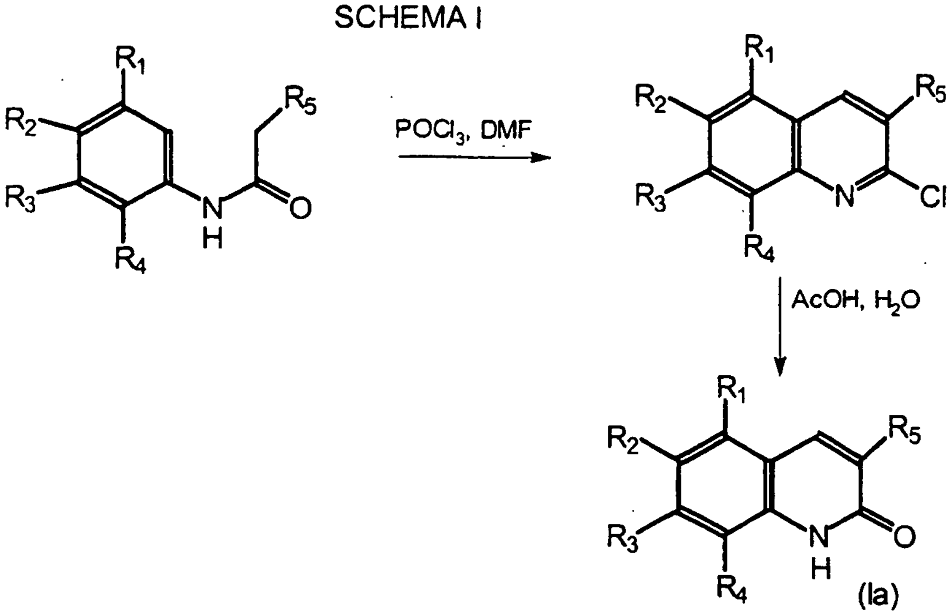


(Ia)

SCHEMA II

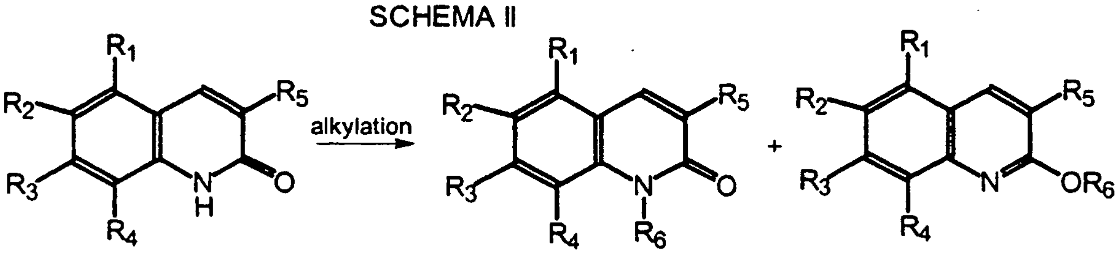


Comme réactif alkylant, on peut utiliser un réactif de type $XR_6$ où X = I, Br, Cl.

En variante, on peut utiliser un composé $CH_2$ = CH-R pour fixer un groupe $R_6$ = $-CH_2-CH_2-R$ (correspondant au groupe $-A-R_{10}$ précédemment défini).

SCHEMA III

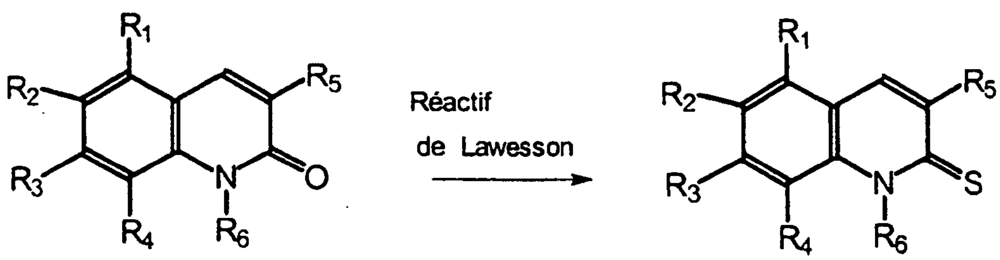

SCHEMA IV

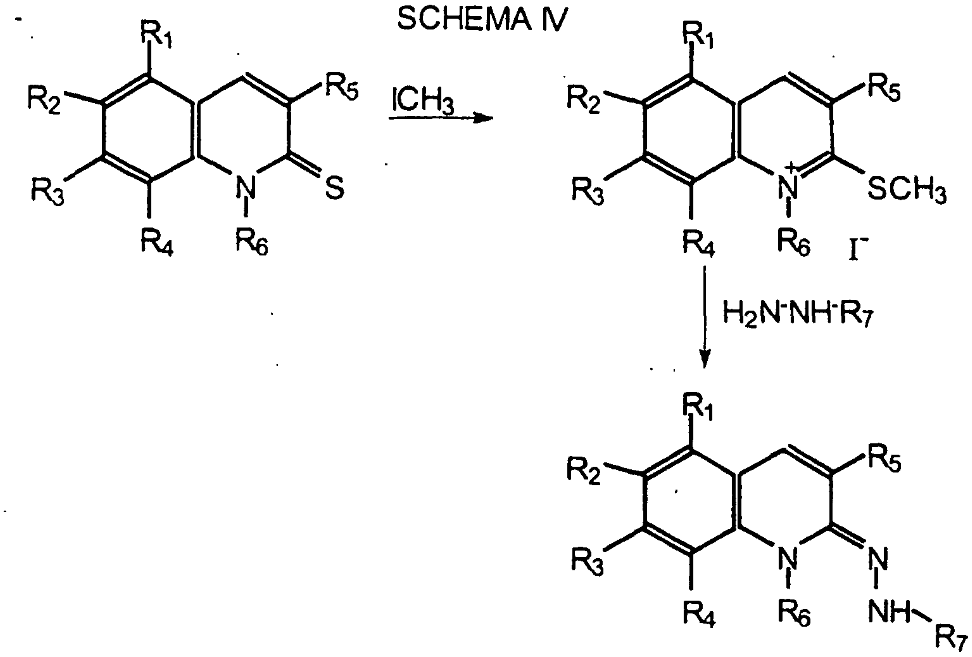


En outre, il est possible de transformer une partie ou la totalité des groupes alkoxy en groupes hydroxy selon des méthodes connues. De même les groupes, hydroxy peuvent être transformés en ester ou en sulfonate selon les méthodes connues.

De même, il est possible de convertir selon des méthodes connues un groupe -A-$COOR_{11}$ dans laquelle $R_{11}$ est un groupe alkyle ou phénylalkyle en un groupe -A-COOH et de convertir un groupe -A-COOH en un groupe -A-$CONR_{12}R_{13}$.

Les composés dans lesquels $R_4$ et $R_6$ forment un groupe -CO-$CH_2$-$CH_2$- peuvent être obtenus par cyclisation d'un composé dans lequel $R_4$ = H et $R_6$ = -$CH_2$-$CH_2$-COOH.

**<u>EXEMPLE 1 :</u> 5,7-Diméthoxy-3-(4-méthoxyphényl)-1,2-dihydro-2-quinolinone (composé 1)**

**a) *N*-(3,5-Diméthoxyphényl)-2-(4-méthoxyphényl)acétamide (Composé 2)**

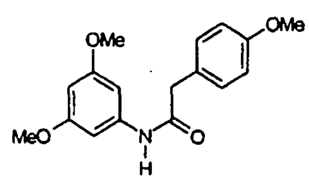

Sous atmosphère d'azote, 500 mg (3,3 mmol) de 3,5-diméthoxyaniline sont solubilisés dans du toluène (7 ml) à 0°C. Une solution de chlorure de 4-méthoxyphénylacétyle (0,5 ml, 3,3 mmol) dans 5 ml de toluène est ajoutée goutte à goutte au milieu. Le mélange réactionnel est agité à température ambiante pendant 1 h, puis le milieu est hydrolysé par une solution froide d'hydrogénocarbonate de sodium. Le système biphasique est agité vigoureusement pendant 30 min, puis la phase organique est recueillie. La phase aqueuse est extraite par de l'acétate d'éthyle (deux fois). La phase organique est séchée sur $MgSO_4$, puis évaporée. Le résidu obtenu est cristallisé dans l'éther de pétrole pour conduire à 810 mg (82%) du composé **2**.

- PF 135-137°C (toluène)
- IR (KBr) n 3292, 1658, 1615 $cm^{-1}$
- $^1H$ RMN (250 MHz, $CDCl_3$): d 3.66 (s, 2H, $CH_2$), 3.74 (s, 6H, $OCH_3$), 3.82 (s, 3H, $OCH_3$), 6.20 (t, 1H, J = 2.2 Hz, $H_{Ar}$), 6.62-6.66 (m, 2H, $H_{Ar}$), 6.95 (d, 2H, J = 7.5 Hz, $H_{Ar}$), 6.97 (s large, 1H, NH), 7.23 (d, 2H, J = 7.5 Hz, $H_{Ar'}$).

- $^{13}C$ RMN (62.90 MHz, $COCl_3$): d 44.0, 55.3, 55.4 (2), 96.7, 97.9 (2), 114.4 (2), 126.2, 130.7 (2), 139.4, 159.0, 161.0 (2), 169.5.
- SM (ionspray): 302 $(M+1)^+$

**b) 2-Chloro-5,7-diméthoxy-3-(4-méthoxyphényl)-1,2-dihydroquinoline (Composé 3)**

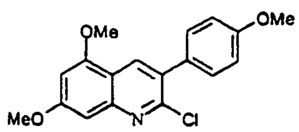

Sous atmosphère d'azote et à -30°C, 0,31 ml (4,0 mmol, 1,5 eq) de N,N-diméthylformamide sont additionnés goutte à goutte à 1,75 ml (20,0 mmol, 7,5 eq) de $POCl_3$. Le milieu est agité pendant 15 min à -30°C, puis 810 mg d'amide 2 (2,7 mmol) sont additionnés. Sous agitation, le mélange réactionnel est ramené à température ambiante, puis la réaction est chauffée à 75°C pendant 2,5 h. A la fin de la réaction, cette solution est versée sur de la glace pilée, neutralisée par une solution d'ammoniaque à 30%, puis extraite par du dichlorométhane. La phase organique est séchée sur $MgSO_4$, puis évaporée. Le résidu obtenu est purifié par chromatographie sur colonne de silice (éluant: AcOEt/EP 3:7) pour donner 270 mg (30%) du composé **3**.

- PF 156-157°C (toluène)
- $^1H$ RMN (250 MHz, $CDCl_3$): d 3.87 (s, 3H, $OCH_3$), 3.93 (s, 3H, $OCH_3$), 3.95 (s, 3H, $OCH_3$), 6.52 (d, 1 H, J = 2.0 Hz, $H_{Ar}$), 6.97-7.01 (m, 3H, $H_{Ar}$), 6.95 (d, 2H, J = 8.7 Hz, $H_{Ar}$), 8.35 (s, 1 H, $H_{Ar}$).
- $^{13}C$ RMN (62.9 MHz, $CDCl_3$): d 55.3, 55.7, 55.8, 98.6, 98.9, 113.6 (2), 115.8, 125.9, 130.5, 131.0 (2), 133.8, 149.0, 150.5, 156.0, 159.4, 162.1.
- SM (ionspray): m/z 330 $(M+1)^+$, 332 $(M+3)^+$

**c) 5,7-Diméthoxy-3-(4-méthoxyphényl)-1,2-dihydro-2-quinolinone (Composé 1)**

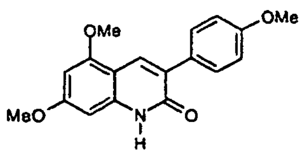

**(CRL8246)**

Le composé 3 (250 mg, 0,76 mmol) en solution dans l'acide acétique (1,2 ml, 26,25 mmol par mmol de 3) et l'eau (0,04 ml, 2,77 mmol par mmol de 3) est agitée à reflux pendant 3 h. L'acide acétique est évaporé. Le résidu obtenu est repris dans l'eau, neutralisé par une solution d'hydroxyde de sodium à 25%, pour être finalement extrait avec du dichlorométhane. La phase organique est séchée sur $MgSO_4$, puis évaporée sous pression réduite. Le résidu est repris dans de l'acétate d'éthyle, cette opération entraînant la cristallisation du produit final. Les cristaux ainsi obtenus sont filtrés pour donner 200 mg (85%) du composé 1.

Le rendement global de la synthèse mise en oeuvre pour obtenir le composé 1 est de 21%.

- PF 254-255°C (AcOEt)
- IR (KBr) n 1664, 1628, 1573, 1518 $cm^{-1}$
- $^1H$ RMN (250 MHz, DMSO-$d_6$): d 3,78 (s, 3H, $OCH_3$), 3.81 (s, 3H, $OCH_3$), 3.89 (s, 3H, $OCH_3$), 6.35 (d, 1H, J = 2.0 Hz, $H_{Ar}$), 6.45 (d, 1H, J = 2.0 Hz, $H_{Ar}$), 6.95 (d, 2H, J = 7.5 Hz, $H_{Ar}$), 7.66 (d, 2H, J = 7.5 Hz, $H_{Ar}$), 7.96 (s, 1H, $H_{Ar}$), 11.76 (s large, 1H, NH).
- $^{13}C$ RMN (62.90 MHz, DMSO-$d_6$): d 55.1, 55.4, 55.9, 90.0, 93.0, 104.6, 113.3 (2), 126.5, 129.0, 129.6 (2), 130.2; 140.5, 156.6, 158.7, 161.5, 161.9.

- SM (ionspray): m/z 312 $(M+1)^+$
- *Anal.* calculé pour $C_{18}H_{17}NO_4$: C, 69.44; H, 5.50; N, 4.50. Trouvé: C, 69.29; H, 5.40; N, 4.55.

**<u>EXEMPLE 2 :</u> 5,7-Diméthoxy-3-(4-hydroxyphényl)-1,2-dihydro-2-quinolinone (Composé 4)**

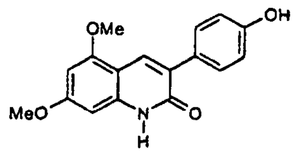

**(CRL8284)**

Sous atmosphère inerte, 530 mg (1,70 mmol) de composé 1 sont solubilisés dans 15 ml d'acide acétique. Une solution commerciale de HBr 48% dans l'eau (2,65 ml) est additionnée goutte à goutte (réaction exothermique) au mélange réactionnel. La solution finale est portée à reflux sous agitation pendant 5 h. Après refroidissement, la réaction est diluée par addition d'eau, puis neutralisée avec une solution d'hydroxyde de sodium à 10% (pH= 6-7). Le produit est extrait par du dichlorométhane (deux fois) La phase organique est séchée sur $MgSO_4$, puis évaporée sous pression réduite. Le résidu est purifié par chromatographie sur colonne de silice (éluant $CH_2Cl2$/MeOH 9:1) pour donner 175 mg (35%) du composé **4**.

- PF 275-276°C (AcOEt)
- IR (KBr) n 1628, 1604, 1558, 1518 $cm^{-1}$
- $^1H$ RMN (250 MHz, DMSO-$d_6$): d 3.80 (s, 3H, $OCH_3$), 3.89 (s, 3H, $OCH_3$), 6.35 (d, 1H, J = 2.5 Hz, $H_{Ar}$), 6.43 (d, 1H, J = 2.5 Hz, $H_{Ar}$), 6.78 (d, 2H, J = 7.5 Hz, $H_{Ar}$), 7.55 (d, 2H, J = 7.5 Hz, $H_{Ar}$), 7.92 (s, 1H, $H_{Ar}$), 9.48 (s, 1 H, OH), 11.72 (s large, 1 H, NH)
- $^{13}C$ RMN (62.90 MHz, DMSO-$d_6$): d 55.4, 55.9, 90.0, 93.0, 104.7, 114.8 (2), 126.9, 127.4, 129.6, 129.8 (2), 140.4, 156.6, 156.9, 161.6, 161.8.
- SM (ionspray): m/z 298 $(M+1)^+$
- *Anal*. calculé pour $C_{17}H_{15}NO_4$: C, 68.68; H, 5.09; N, 4.71. Trouvé: C, 68.90; H, 5.09; N, 4.0.

**<u>EXEMPLE 3 :</u> 5,7-Dihydroxy-3-(4-hydroxyphényl)-1,2-dihydro-2-quinolinone (Composé 5)**

14

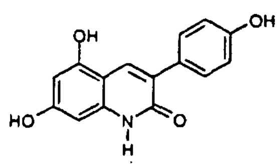

**(CRL8311)**

Sous atmosphère inerte, 1,0 g (3,2 mmol) de composé 1 est dissous dans 15 ml d'acide acétique. Une solution commerciale de HBr 48% dans l'eau (5 ml) est additionnée goutte à goutte (réaction exothermique) au mélange réactionnel. La solution finale est agitée à reflux pendant 3 jours. Après refroidissement, la réaction est diluée par addition d'eau, puis neutralisée avec une solution d'hydroxyde de sodium à 10% (pH= 6-7). Le produit est extrait par du dichlorométhane (deux fois). La phase organique est séchée sur $MgSO_4$, puis évaporée sous pression réduite. Le résidu obtenu est purifié par chromatographie sur colonne de silice (éluant $CH_2Cl_2$/MeOH 9:1) pour donner 320 mg (37%) du composé **5**.

- PF > 280°C
- $^1H$ RMN (250 MHz, DMSO-$d_6$): d 6.11 (d, 1H, J = 2.0 Hz, $H_{Ar}$), 6.18 (d, 1H, J = 2.0 Hz, $H_{Ar}$), 6.76 (d, 2H, J = 8.6 Hz, $H_{Ar}$), 7.52 (d, 2H, J = 8.6 Hz, $H_{Ar}$), 7.89 (s, 1 H, $H_{Ar}$), 9.42 (s, 1H, OH), 9.84 (s, 1H, OH), 10.21 (s, 1 H, OH), 11.46 (s large, 1 H, NH).

- $^{13}C$ RMN (62.90 MHz, DMSO-$d_6$): d 91.3, 96.4, 103.5, 114.7 (2), 125.1, 127.8, 129.4 (2), 130.4, 140.7, 155.3, 156.6, 160.1, 161.8.
- SM (ionspray): m/z 270 $(M+1)^+$
- *Anal.* calculé pour $C_{15}H_{11}NO_4$: C, 66.91; H, 4.12; N, 5.20. Trouvé: C, 66.80; H, 4.00; N, 5.0.

**EXEMPLE 4 : 5,7-Diméthoxy-3-(4-méthoxyphényl)-1,2-dihydro-2-quinolinethione (Composé 6)**

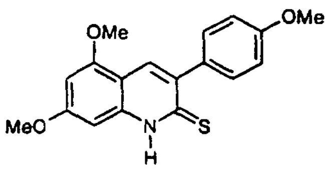

**(CRL8271)**

Sous atmosphère inerte, 100 mg (0,32 mmol) de composé 1 sont solubilisés dans 15 ml de toluène (dissolution à chaud). 260 mg (0,64 mmol, 2 eq) de réactif de Lawesson sont additionnés au mélange réactionnel. La solution finale est agitée à reflux pendant 18 h. Après refroidissement, le toluène est évaporé. Le résidu obtenu est purifié par chromatographie sur colonne de silice (éluant $CH_2Cl_2$/AcOEt 9:1 ) pour donner 81 mg (77%) du composé **6**.

- PF 229-230°C ($Et_2O$)
- IR (KBr) 1636, 1610, 1524 $cm^{-1}$
- $^1H$ RMN (250 MHz, DMSO-$d_6$): d 3,78 (s, 3H, $OCH_3$), 3.83 (s, 3H, $OCH_3$), 3.89 (s, 3H, $OCH_3$), 6.49 (d, 1 H, J = 1.9 Hz, $H_{Ar}$), 6.79 (d, 1 H, J = 1.9 Hz, $H_{Ar}$), 6.92 (d, 2H, J = 8.7 Hz, $H_{Ar}$), 7.49 (d, 2H, J = 8.7 Hz, $H_{Ar}$), 7.78 (s, 1H, $H_{Ar}$), 13.50 (s large, 1H, NH).
- $^{13}C$ RMN (62.90 MHz, $CDCl_3$): d 55.1, 55.6, 56.1, 90.2, 95.2, 108.9, 112.9 (2), 128.2, 130.7 (2), 132.0, 136.3, 140.9, 156.4, 158.5, 162.5, 180.5.
- SM (ionspray): m/z 328 $(M+1)^+$
- *Anal.* calculé pour $C_{18}H_{17}NO_3S$: C, 66.03; H, 5.23; N, 4.28. Trouvé: C, 66.30; H, 5.30; N, 4.35.

**EXEMPLE 5 : 5,7-Diméthoxy-3-(4-méthoxyphényl)-1-méthyl-1,2-dihydro-2-quinolinone (Composé 7)**

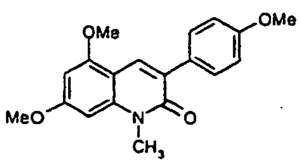

**(CRL8244)**

Sous atmosphère d'azote, 600 mg (1,93 mmol) de composé 1 sont solubilisés dans 30 ml de *N,N*-diméthylformamide (DMF) anhydre. A 0°C, 93 mg (3,86 mmol, 2 eq) de NaH, préalablement lavés avec de l'éther de pétrole, sont additionnés par petites portions à la solution réactionnelle (réaction exothermique). Une solution d'iodure de méthyle (0,48 ml, 7,72 mmol, 4 eq) diluée dans 5 ml de DMF est ajoutée au milieu. La réaction est chauffée pendant 18 h à 90°C. Après refroidissement, de l'eau est versée sur le mélange réactionnel puis la solution est agitée pendant 15 min. Le solide obtenu est recueilli par filtration sur verre fritté puis rincé par de l'eau. Le solide est dissous dans le $CH_2Cl_2$ et lavé par deux fois par de l'eau. La phase organique obtenue est séchée sur $MgSO_4$, puis évaporée sous pression réduite. Le résidu obtenu est purifié par chromatographie sur colonne de silice ($CH_2Cl_2$/AcOEt 8:2) pour donner 408 mg (68%) de composé **7** et 157 mg (25%) de dérivé **7a**.

*Composé* **7**:

- PF 125°C (AcOEt/EP)
- IR (KBr) n 1635, 1596, 1590, 1514 $cm^{-1}$
- $^1H$ RMN (250 MHz, $CDCl_3$): d 3.73 (s, 3H, $NCH_3$), 3.83 (s, 3H, $OCH_3$), 3.91 (s, 6H, $OCH_3$), 6.30 (d, 1H, J = 2.0 Hz, $H_{Ar}$), 6.37 (d, 1H, J = 2.0 Hz, $H_{Ar}$), 6.94 (d, 2H, J = 7.5 Hz, $H_{Ar}$), 7.67 (d, 2H, J = 7.5 Hz, $H_{Ar}$), 8.12 (s, 1 H, $H_{Ar}$).
- $^{13}C$ RMN (62.90 MHz, $CDCl_3$): d 30.3, 55.3, 55.5, 55.8, 90.2, 92.6, 106.1, 113.5 (2), 127.0, 130.0, 130.1 (2), 130.2, 141.7, 157.6, 159.1, 162.2 (2).
- SM (ionspray): m/z 326 $(M+1)^+$
- *Anal.* calculé pour $C_{19}H_{19}NO_4$: C, 70.14; H, 5.89; N, 4.30. Trouvé: C, 70.00; H, 5.73; N, 4.24.

*Composé* ***7a:***

**2,5,7-Triméthoxy-3-(4-méthoxyphényl)quinoline**

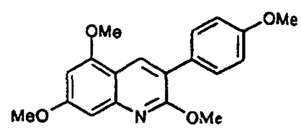

- PF 106-107°C (AcOEt/EP)
- IR (KBr) n 1621, 1515, 1265 $cm^{-1}$
- $^1H$ RMN (250 MHz, $COCl_3$): d 3.86 (s, 3H, $OCH_3$), 3.94 (s, 6H, $OCH_3$), 4.08 (s, 3H, $OCH_3$), 6.40 (d, 1H, J = 1.8 Hz, $H_{Ar}$), 6.85 (d, 1H, J = 1.8 Hz, $H_{Ar}$), 6.97 (d, 2H, J = 8.8 Hz, $H_{Ar}$), 7.57 (d, 2H, J = 8.8 Hz, $H_{Ar}$), 8.26 (s, 1 H, $H_{Ar}$).
- $^{13}C$ RMN (62.90 MHz, $COCl_3$): d 53.5, 55.3, 55.5, 55.6, 95.9, 98.5, 112.8, 113.6 (2), 122.1, 129.6, 130.5 (2), 132.3, 147.9, 156.3, 158.9, 160.6, 161.3.
- SM (ionspray): m/z 326 $(M+1)^+$
- *Anal.* calculé pour $C_{19}H_{19}NO_4$: C, 70.14; H, 5.89; N, 4.30. Trouvé: C, 69.89; H, 5.81; N, 4.10.

**<u>EXEMPLE 6 :</u> 5,7-Diméthoxy-3-(4-hydrooyphényl)-1-méthyl-1,2-dihydro-2-quinolinone (Composé 8)**

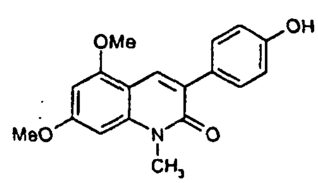

Sous atmosphère inerte, 408 mg (1,3 mmol) de composé 7 sont solubilisés dans 15 ml d'acide acétique. Une solution commerciale de HBr 48% dans l'eau (2 ml) est additionnée goutte à goutte (réaction exothermique) au mélange réactionnel. La solution finale est agitée à reflux pendant 5 h. Après refroidissement, la réaction est diluée par addition d'eau, puis neutralisée avec une solution d'hydroxyde de sodium à 10% (pH= 6-7). Le produit est extrait par du dichlorométhane (deux fois). La phase organique est séchée sur $MgSO_4$, puis évaporée sous pression réduite. Le résidu obtenu est purifié par chromatographie sur colonne de silice (éluant $CH_2Cl_2$/AcOEt 7:3) pour donner 220 mg (56%) du composé **8**.

- PF 204-205°C (AcOEt)
- $^1H$ RMN (250 MHz, DMSO-$d_6$): d 3.63 (s, 3H, $NCH_3$), 3,89 (s, 3H, $OCH_3$), 3.90 (s, 3H, $OCH_3$), 6.46 (d, 1H, J = 1.9

Hz, $H_{Ar}$), 6.54 (d, 1H, J = 1.9 Hz, $H_{Ar}$), 6.76 (d, 2H, J = 8.5 Hz, $H_{Ar}$), 7.48 (d, 2H, J = 8.5 Hz, $H_{Ar}$), 7.93 (s, 1H, $H_{Ar}$), 9.47 (s, 1 H, OH).

- $^{13}C$ RMN (62.90 MHz, DMSO-$d_6$): d 30.5, 56.1, 56.5, 91.3, 93.4, 105.3, 115.2 (2), 126.5, 128.4, 129.2, 130.2 (2), 141.7, 157.4 (2), 161.4, 162.5.
- SM: m/z 312 $(M+1)^+$
- *Anal.* calculé pour $C_{18}H_{17}NO_4$: C, 69.44; H, 5.50; N, 4.50. Trouvé: C, 69.65; H, 5.59; N, 4.60.

**EXEMPLE 7 : 5,7·Dihydroxy-3-(4·hydroxyphényl)-1-méthyl-1,2-dihydro-2-quinolinone (Composé 9)**

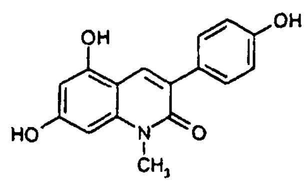

**(CRL8321)**

**1. Méthode A:** Sous atmosphère inerte, 200 mg (0,61 mmol) de composé 7 sont solubilisés dans 15 ml d'acide acétique. Une solution commerciale de HBr 48% dans l'eau (1 ml) est additionnée goutte à goutte (réaction exothermique) au mélange réactionnel. La solution finale est agitée à reflux pendant 3 jours. Après refroidissement, la réaction est diluée par addition d'eau, puis neutralisée avec une solution d'hydroxyde de sodium à 10% (pH= 6-7). Le produit est extrait par du dichlorométhane (deux fois). La phase organique est séchée sur $MgSO_4$, puis évaporée sous pression réduite. Le résidu obtenu est purifié par chromatographie sur colonne de silice (éluant $CH_2Cl_2$/MeOH 9:1) pour donner 61 mg (35%) du composé **9.**

**2. Méthode B**: Sous atmosphère inerte, 1,0 g (3,1 mmol) de composé **7** est solubilisé dans 15 ml de dichlorométhane. A 0°C, 1,81 ml (19,0 mmol, 6 eq) de tribromure de bore sont additionnés goutte à goutte (réaction exothermique) au mélange réactionnel. La solution finale est agitée à température ambiante pendant 18 h. La réaction est hydrolysée par addition (goutte à goutte) d'eau, puis neutralisée avec une solution d'hydroxyde de sodium à 10% (pH= 6-7). Le produit est extrait par du dichlorométhane (deux fois). La phase organique est séchée sur $MgSO_4$, puis évaporée sous pression réduite. Le résidu obtenu est purifié sur colonne de silice (éluant $CH_2Cl_2$/MeOH 9:1) pour donner 688 mg (76%) du composé 9.

- PF > 280°C (AcOEt)
- $^1H$ RMN (250 MHz, DMSOd-$_6$): d 3,53 (s, 3H, $CH_3$), 6.25 (s, 2H, $H_{Ar}$), 6.76 (d, 2H, J = 8.8 Hz, $H_{Ar}$), 7.47 (d, 2H, J = 8.8 Hz, $H_{Ar}$), 7.92 (s, 1H, $H_{Ar}$), 9.42 (s, 1H, OH), 10.00 (s, 1 H, OH), 10.35 (s, 1 H, OH).
- $^{13}C$ RMN (62.90 MHz, DMSOd-$_6$): d 29.7, 91.8, 96.5, 103.5, 114.7 (2), 124.3, 128.4, 129.7 (3), 141.7, 155.9, 156.7, 160.6, 161.1.
- SM (ionspray): m/z 284 $(M+1)^+$
- *Anal.* calculé pour $C_{16}H_{13}NO_4$: C, 67.84; H, 4.63; N, 4.94. Trouvé: C, 67.68; H, 4.46; N, 4.78.

**EXEMPLE 8 : 5,7-Diméthoxy-3-(4-méthoxyphényl)-1-méthyl-1,2-dihydro-2-quinolinethione (Composé 10)**

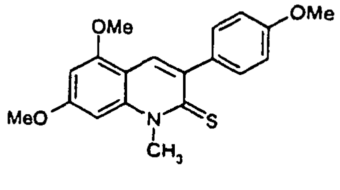

**(CRL8245)**

Sous atmosphère d'azote, 500 mg (1,5 mmol) de composé 7 sont solubilisés dans 30 ml de toluène. A ce mélange réactionnel est ajouté 870 mg (2,1 mmol, 1,4 eq) de réactif de Lawesson. La réaction est portée à reflux pendant 12 h. Après refroidissement, le solvant est évaporé. Le résidu obtenu est purifié par chromatographie sur

colonne de silice (AcOEt/EP 3:7) pour donner 376 mg (72%) du composé **10**.

- PF 176-177°C (AcOEt/EP)
- IR (KBr) 1613, 1570, 1512 $cm^{-1}$
- $^1H$ RMN (250 MHz, $CDCl_3$): d 3,84 (s, 3H, $OCH_3$), 3.91 (s, 3H, $OCH_3$), 3.95 (s, 3H, $OCH_3$). 4.39 (s, 3H, $NCH_3$). 6.39 (d, 1H, J = 2.0 Hz, $H_{Ar}$), 6.56 (d, 1 H, J = 2.0 Hz, $H_{Ar}$), 6.93 (d, 2H, J = 7.5 Hz, $H_{Ar}$), 7.43 (d, 2H, J = 7.5 Hz, $H_{Ar}$), 7.97 (s, 1 H, $H_{Ar}$).
- $^{13}C$ RMN (62.90 MHz, $CDCl_3$): d 39.5, 55.2, 55.6, 55.9, 91.1, 94.6, 109.9, 113.1 (2), 126.6, 130.8 (2), 134.3, 138.6, 142.7, 157.6, 158.8, 162.7, 184.5.
- SM: m/z 342 $(M+1)^+$
- *Anal*. calculé pour $C_{19}H_{19}NO_3S$. C, 66.84; H, 5.61; N, 4.10. Trouvé: C, 66.70; H, 5.53; N, 4.03.

**<u>EXEMPLE 9 :</u> 2-[5,1-Diméthoxy-3-(4-hydroxyphényl)-2-oxo-1,2-dihydro-1-quinolinyl]acétate d'éthyle (Composé 11)**

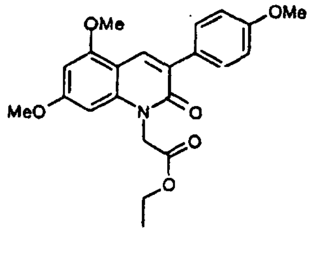

(CRL8314)

Sous atmosphère d'azote, 1,0 g (3,2 mmol) de composé 1 est solubilisé dans 30 ml de *N*,*N*-diméthylformamide (DMF) anhydre. A 0°C, 115 mg (4,8 mmol, 1,5 eq) de NaH, préalablement lavés dans de l'éther de pétrole, sont additionnés par petites portions à la solution réactionnelle (réaction exothermique). Une solution de bromoacétate d'éthyle (0,72 ml, 6,4 mmol, 2 eq) dans 5 ml de DMF est ajoutée au milieu. La réaction est chauffée pendant 2-3 h à 90°C. Après refroidissement, de l'eau est versée sur le mélange réactionnel, puis celui-ci est agitée pendant 15 min. La solution est extraite par de l'acétate d'éthyle (deux fois). La phase organique est séchée sur $MgSO_4$, puis évaporée sous pression réduite. Le résidu obtenu est purifié par chromatographie sur colonne de silice ($CH_2Cl_2$/AcOEt 7:3) pour donner 890 mg (70%) de composé 11 et 318 mg (25%) de dérivé **11a.**

*Composé **11:***

- PF 160-161°C (AcOEt/EP)
- IR (KBr) 1735, 1647, 1609 $cm^{-1}$
- $^1H$ RMN (250 MHz, $CDCl_3$): d 1.6 (t, 3H, J = 7.1 Hz, $COOCH_2CH_3$), 3.83 (s, 3H, $OCH_3$), 3.87 (s, 3H, $OCH_3$), 3.92 (s, 3H, $OCH_3$), 4.22 (q, 2H, J = 7.1 Hz, $COOCH_2CH_3$), 5.10 (s, 2H, $CH_2CO$), 6.14 (d, 1H, J = 2.0 Hz, $H_{Ar}$), 6.30 (d, 1H, J = 2.0 Hz, $H_{Ar}$), 6.94 (d, 2H, J = 7.5 Hz, $H_{Ar}$), 7.69 (d, 2H, J = 7.5 Hz, $H_{Ar}$), 8.18 (s, 1H, $H_{Ar}$).
- $^{13}C$ RMN (62.90 MHz, $COCl_3$): d 14.2, 44.8, 55.3, 55.5, 55.9, 61.6, 90.0, 92.8, 106.2, 113.5 (2), 126.6, 129.6, 130.1 (2), 131.0, 141.1, 157.8, 159.2, 161.9, 162.5, 168.4.
- SM (ionspray): m/z 398 $(M+1)^+$
- *Anal*. calculé pour $C_{22}H_{23}NO_6$: C, 66.49; H, 5.83; N, 3.52. Trouvé: C, 66.60; H, 6.03; N, 3.75.

*Composé **11a:***

**2 -([5,7-Diméthoxy-3-(4-méthoxyphényl) -2-quinolinyl]oxy)acétate d'éthyle**

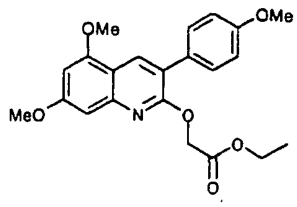

- PF 95-96°C (AcOEt/EP)
- IR (KBr) n 1754, 1622, 1516, 1265 $cm^{-1}$
- $^1H$ RMN (250 MHz, $CDCl_3$): d 1.27 (t, 3H, J = 7.1 Hz, $CH_3$), 3,86 (s, 3H, $OCH_3$), 3.91 (s, 3H, $OCH_3$), 3.93 (s, 3H, $OCH_3$). 4.24 (q, 2H , J = 7.1 Hz, $OCH_2$), 5.05 (s, 2H, N $CH_2$), 6.40 (d, 1H, J = 2.0 Hz, $H_{Ar}$), 6.76 (d, 1H, J = 2.0 Hz, $H_{Ar}$), 6.98 (d, 2H, J = 9.0 Hz, $H_{Ar}$), 7.68 (d, 2H, J = 9.0 Hz, $H_{Ar}$), 8.30 (s, 1 H, $H_{Ar}$).
- $^{13}C$ RMN (62.90 MHz, $CDCl_3$): d 14.2, 55.3, 55.5, 55.7, 60.9, 62.7, 96.2, 98.6, 113.4, 113.7 (2), 121.7, 129.2, 130.6 (2), 132.8, 147.3, 156.3, 158.8, 159.1, 161.4, 169.5.
- SM (ionspray): m/z 398 $(M+1)^+$
- *Anal.* calculé pour $C_{22}H_{23}NO_6$: C, 66.49; H, 5.83; N, 3.52. Trouvé: C, 66.63; H, 5.90; N, 3.60.

**EXEMPLE 10 : 3-[5,7-Diméthoxy-3-(4-hydroxyphényl)-2-oxo-1,2-dihydro-1-quinolinyl]propanoate de méthyle (Composé 12)**

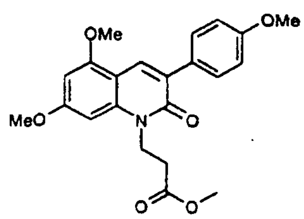

**(CRL8318)**

Sous atmosphère d'azote, 1,0 g (3,2 mmol) de composé 1 est solubilisé dans 20 ml de *N*,*N*-diméthylformamide (DMF) anhydre en présence de 2,9 ml d'acrylate de méthyle (32,0 mmol, 10 eq). A 0°C, 2-3 gouttes de triton B sont additionnées à la solution réactionnelle. Le mélange est agité pendant 4 h à température ambiante. Le DMF et l'acrylate de méthyle sont évaporés sous pression réduite. Le résidu obtenu est repris dans l'acétate d'éthyle et lavé deux fois par de l'eau. La phase organique obtenue est séchée sur $MgSO_4$, puis évaporée sous pression réduite. Le produit brut est purifié par chromatographie sur colonne de silice ($CH_2Cl_2$/AcOEt 8:2) pour donner 1,06 g (83%) du composé **12**.

- PF 100-101°C (AcOEt)
- IR (KBr) 1725, 1638 $cm^{-1}$
- $^1H$ RMN (250 MHz, $CDCl_3$): d 2.78 (t, 2H, J = 8.0 Hz, $CH_2$), 3.68 (s, 3H, $COOCH_3$), 3.80 (s, 3H, $OCH_3$), 3.88 (s, 6H, $OCH_3$), 4.57 (t, 2H, J = 8.0 Hz, $CH_2$), 6.26 (d, 1H, J = 2.0 Hz, $H_{Ar}$), 6.46 (d, 1H, J = 2.0 Hz, $H_{Ar}$), 6.92 (d, 2H, J = 8.8 Hz, $H_{Ar}$), 7.66 (d, 2H, J = 8.8 Hz, $H_{Ar}$), 8.11 (s, 1H, $H_{Ar}$).
- $^{13}C$ RMN (62.90 MHz, $CDCl_3$): d 31.9, 39.1, 51.8, 55.2, 55.5, 55.7, 89.8, 92.6, 106.2, 113.4 (2), 126.5, 129.5, 129.9 (2), 130.3, 140.5, 157.6, 159.0, 161.7, 162.4, 172.0.
- SM (ionspray): m/z 398 $(M+1)^+$
- *Anal.* calculé pour $C_{22}H_{23}NO_6$: C, 66.49; H, 5.83; N, 3.52. Trouvé: C, 66.55; H, 5.70; N, 3.50.

**EXEMPLE 11 : 2-[5,7-Diméthoxy-3-(4-méthoxyphényl)-2-oxo-1,2-dihydro-1-quinolinyl]acétate de benzyle (Composé 13)**

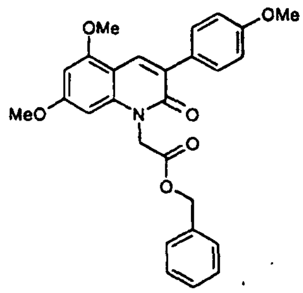

Sous atmosphère d'azote, 300 mg (0.96 mmol) de composé 1 sont solubilisés dans 10 ml de *N,N*-diméthylformamide (DMF) anhydre. A 0°C, 35 mg (1,40 mmol, 1,5 eq) de NaH, préalablement lavés dans de l'éther de pétrole, sont additionnés par petites portions à la solution réactionnelle (réaction exothermique). Une solution de bromoacétate de benzyle (0,31 ml, 1,90 mmol, 2 eq) diluée dans 5 ml de DMF est ajoutée au milieu. La réaction est chauffée pendant 2 h à 90°C. Après refroidissement, de l'eau est versée sur le mélange réactionnel, puis ce dernier est agitée pendant 15 min. La solution est extraite par du dichlorométhane (deux fois). La phase organique est séchée sur $MgSO_4$, puis évaporée sous pression réduite. Le résidu obtenu est purifié par chromatographie sur colonne de silice (éluant $CH_2Cl_2$) pour donner 317 mg (72%) de composé **13** et 88 mg (20%) de dérivé **13a.**

*Composé **13:***

- PF 199-200°C (lavage $Et_2O$)
- IR (KBr) 1748, 1642, 1617 $cm^{-1}$
- $^1H$ RMN (250 MHz, $CDCl_3$): d 3,68 (s, 3H, $OCH_3$), 3.84 (s, 3H, $OCH_3$), 3.92 (s, 3H, $OCH_3$), 5.16 (s, 2H, $CH_2$), 5.21 (s, 2H, $CH_2$), 6.03 (d, 1H, J = 2.0 Hz, $H_{Ar}$), 6.27 (d, 1H, J = 2.0 Hz, $H_{Ar}$), 6.94 (d, 2H, J = 7.5 Hz, $H_{Ar}$), 7.25-7.32 (m, 5H, $H_{Ar}$), 7.68 (d, 2H, J = 7.5 Hz, $H_{Ar}$), 8.17 (s, 1 H, $H_{Ar}$).
- $^{13}C$ RMN (62.90 MHz, $CDCl_3$): d 44.7, 55.3, 55.4, 55.8, 67.1, 89.8, 93.0, 106.2, 113.5 (2), 126.5, 128.3 (2), 128.4, 128.5 (2), 129.5, 130.1 (2), 131.1, 135.3, 141.0, 157.8, 159.2, 161.8, 162.4, 168.3.
- SM (ionspray): m/z 460 $(M+1)^+$

***Composé 13a:***

**2-([5,7-Diméthoxy-3-(4-méthoxyphényl)·2-quinolinyl]oxy)acétate de benzyle**

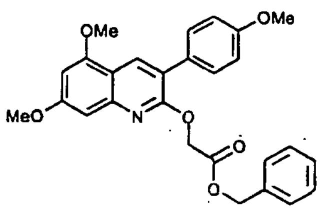

- PF 114-115°C (éther)
- IR (KBr) n 1761, 1621, 1517 $cm^{-1}$
- $^1H$ RMN (250 MHz, $CDCl_3$): d 3,87 (s, 3H, $OCH_3$), 3.91 (s, 3H, $OCH_3$), 3.95 (s, 3H, $OCH_3$), 5.17 (s, 2H, $OCH_2$), 5.27 (s, 2H, $OCH_2$), 6.44 (d, 1H, J = 2.0 Hz, $H_{Ar}$), 6.76 (d, 2H, J = 2.0 Hz, $H_{Ar}$), 7.00 (d, 2H, J = 8.0 Hz, $H_{Ar}$),

7.26-7.38 (m, 5H, $H_{Ar}$), 7.71 (d, 2H, J = 8.0 Hz, $H_{Ar}$), 8.36 (s, 1H, $H_{Ar}$).

- $^{13}$C RMN (62.90 MHz, $CDCl_3$): d 55.2, 55.4, 55.5, 62.6, 66.4, 96.2, 98.5, 113.4, 113.6 (2), 121.6, 128.0 (2), 128.4 (3), 129.0, 130.5 (2), 132.7, 135.6, 147.2, 156.1, 158.6, 159.0, 161.3, 169.3.
- SM (ionspray): m/z 460 $(M+1)^+$

**<u>EXEMPLE 12</u> : Acide 2-[5,7-diméthoxy-3-(4-méthoxyphényl)-2-oxo-1,2-dihydro-1-quinolinyl)acétique (Composé 14)**

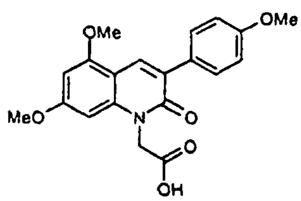

**(CRL8317)**

Dans un ballon, 1,0 g (2,2 mmol) d'ester benzylique **13** est solubilisé dans le dioxane (30 ml). Le palladium sur charbon à 10% (100 mg) est additionné à la solution réactionnelle. La réaction de débenzylation est effectuée au moyen d'un appareil de Parr sous 40 psi d'hydrogène à température ambiante pendant 4 h. Le milieu réactionnel est filtré sur célite et le filtrat est évaporé sous pression réduite. Le produit cristallin obtenu est lavé par de l'éther pour donner 764 mg (95%) du composé **14**.

- PF 179-180°C (lavage $Et_2O$)
- IR (KBr) 1732, 1614, 1583 $cm^{-1}$
- $^1$H RMN (250 MHz, DMSO-$d_6$): d 3.77 (s, 3H, $OCH_3$), 3.85 (s, 3H, $OCH_3$). 3.91 (s, 3H, $OCH_3$), 5.02 (s, 2H, $CH_2$), 6.46 (s, 1H, $H_{Ar}$), 6.47 (s, 1 H, $H_{Ar}$), 6.93 (d, 2H, J = 9.0 Hz, $H_{Ar}$), 7.62 (d, 2H, J = 9.0 Hz, $H_{Ar}$), 8.03 (s, 1 H, $H_{Ar}$).
- $^{13}$C RMN (62.90 MHz, DMSO-$d_6$): d: 44.9, 55.5, 56.1, 56.6, 91.3, 93.3, 105.3, 113.8 (2), 125.7, 129.4, 130.1 (2), 130.4, 141.3, 157.6, 159.1, 161.3, 162.8, 170.1.
- SM (ionspray): m/z 370 $(M+1)^+$
- *Anal.* calculé pour $C_{20}H_{19}NO_6$: C, 65.03; H, 5.18; N, 3.79. Trouvé: C, 65.00; H, 5.25; N, 3.85.

**<u>EXEMPLE 13</u> : 3-[5,7-Diméthoxy-3-(4-méthoxyphényl)-2-oxo-1,2-dihydro-1-quinolinyl]propanoate de benzyle (Composé 15)**

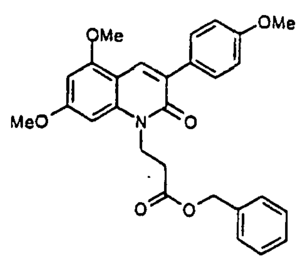

Sous atmosphère d'azote, 150 mg (0,48 mmol) de composé **1** sont solubilisés dans 10 ml de *N,N*-diméthylformamide (DMF) anhydre en présence de 782 mg (4,8 mmol, 10 eq) d'acrylate de benzyle. A 0°C, 2-3 gouttes de triton B sont additionnées à la solution réactionnelle. La solution est agitée pendant 18 h à température ambiante. Les solvants sont évaporés sous pression réduite. Le résidu obtenu est repris dans l'acétate d'éthyle et lavé deux fois par de l'eau. La phase organique obtenue est séchée sur $MgSO_4$, puis évaporée sous pression réduite. Le produit brut est purifié par chromatographie sur colonne de silice (AcOEt/EP 4:6) pour donner 200 mg (88%) du composé **15**.

- PF 124-125°C ($Et_2O$/EP)

- IR (KBr) 1731, 1635, 1600 $cm^{-1}$
- $^1H$ RMN (250 MHz, $CDCl_3$): d 2.86 (t, 2H, J = 8.0 Hz, $COCH_2$), 3,84 (s, 3H, $OCH_3$), 3.85 (s, 3H, $OCH_3$), 3.92 (s, 3H, $OCH_3$), 4.63 (t, 2H, J = 8.0 Hz, $NCH_2$), 5.14 (s, 2H, $CH_2Ph$), 6.29 (d, 1H, J = 2.0 Hz, $H_{Ar}$), 6.49 (d, 1H, J = 2.0 Hz, $H_{Ar}$), 6.94 (d, 2H, J = 7.5 Hz, $H_{Ar}$), 7.30-7.35 (m, 5H, $H_{Ar}$), 7.68 (d, 2H, J = 7.5 Hz, $H_{Ar}$), 8.13 (s, 1 H, $H_{Ar}$).
- $^{13}C$ RMN (62.90 MHz, $CDCl_3$): d 32.2, 39.1, 55.2, 55.4, 55.7, 66.5, 89.7, 92.6, 106.2, 113.4 (2), 126.5, 128.1 (2), 128.2, 128.4 (2), 129.5, 129.9 (2), 130.4, 135.5, 140.5, 157.6, 159.0, 161.7, 162.4, 171.3. SM (ionspray): m/z 474 $(M+1)^+$

**<u>EXEMPLE 14 :</u> Acide 3-[5,7-diméthoxy-3-(4-méthoxyphényl)-2-oxo-1,2-dihydro-1-quinolinyl]propanoïque (Composé 16)**

26

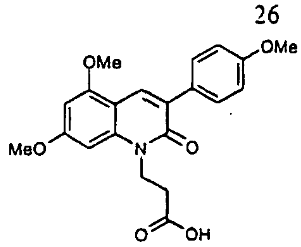

**(CRL8319)**

Dans un ballon, 1,0 g (2,1 mmol) d'ester benzylique **15** est solubilisé dans le dioxane (30 ml). Le palladium sur charbon à 10% (100 mg) est additionné à la solution réactionnelle. La réaction de débenzylation est effectuée au moyen d'un appareil de Parr sous 40 psi d'hydrogène pendant 48 h. Le milieu réactionnel est filtré sur célite et le filtrat est évaporé sous pression réduite pour donner, après lavage à l'éther, 780 mg (97%) du composé **16**.

- PF 194-195°C (lavage $Et_2O$)
- IR (KBr) 1724, 1637, 1612, 1604 $cm^{-1}$
- $^1H$ RMN (250 MHz, DMSO-$d_6$): d 2.60 (t, 2H, J = 7.5 Hz, $COCH_2$), 3.78 (s, 3H, $OCH_3$), 3.90 (s, 3H, $OCH_3$), 3.92 (s, 3H, $OCH_3$), 4.48 (t, 2H, J = 7.5 Hz, $NCH_2$), 6.48 (d, 1H, J = 1.8 Hz, $H_{Ar}$), 6.62 (s large, 1H, $H_{Ar}$), 6.95 (d, 2H, J = 8.8 Hz, $H_{Ar}$), 7.62 (d, 2H, J = 8.8 Hz, $H_{Ar}$), 8.00 (s, 1 H, $H_{Ar}$).
- $^{13}C$ RMN (62.90 MHz, DMSO-$d_6$): d: 32.0, 38.9, 55.1, 55.7, 56.1, 90.6, 93.0, 105.0, 113.3 (2), 125.6, 129.3, 129.5, 129.8 (2), 140.5, 157.2, 158.7, 160.6, 162.4, 172.5.
- SM: m/z 384 $(M+1)^+$
- *Anal.* calculé pour $C_{21}H_{21}NO_6$: C, 65.79; H, 5.52; N, 3.65. Trouvé: C, 65.60; H, 5.51; N, 3.70.

**<u>EXEMPLE 15</u> : *N,N*-Diéthyl-3-[5,7-diméthoxy-3-(4-méthoxyphényl)-2-oxo-1,2-dihydro-1-quinolinyl]propanamide (Composé 17)**

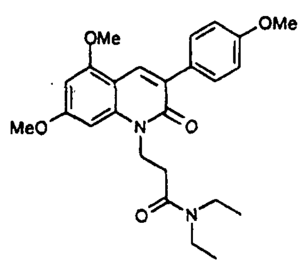

**(CRL8283)**

Sous atmosphère d'azote, 1,0 g (2,6 mmol) de composé **16** est solubilisé dans 25 ml de *N,N*-diméthylformamide (DMF) anhydre. A 0°C, 353 mg (2,6 mmol) d'hydroxybenzotriazole et 540 mg (2,6 mmol) de cyclohexylcarbodiimide sont additionnés à la solution réactionnelle. La réaction est agitée 10 minutes à 0°C, avant d'ajouter 0,26 ml (2,6

mmol) de diéthylamine. La solution finale est agitée 2 h à 0°C, puis 24 h à température ambiante. La dicyclohexylurée est éliminée par filtration. Le filtrat est extrait par de l'acétate d'éthyle (deux fois). La phase organique recueillie est lavée plusieurs fois à l'eau. La phase organique est séchée sur $MgSO_4$, puis évaporée sous pression réduite. Le résidu obtenu est purifié par chromatographie sur colonne de silice ($CH_2Cl_2$/AcOEt 7:3) pour donner 680 mg (60%) du composé **17**.

- PF 137-138°C (lavage AcOEt)
- IR (KBr) 1636, 1617 $cm^{-1}$
- $^1$H RMN (250 MHz, $CDCl_3$): d 1.07-1.21 (m, 6H, $CH_3$), 2.78 (t, 2H, J = 8.0 Hz, $COCH_2$), 3.30 (q, 2H, J = 7.0 Hz, $NCH_2$), 3.39 (q, 2H, J = 7.0 Hz, $NCH_2$), 3,84 (s, 3H, $OCH_3$), 3.91 (s, 3H, $OCH_3$), 3.94 (s, 3H, $OCH_3$), 4.65 (t, 2H, J = 8.0 Hz, $NCH_2$), 6.29 (d, 1H, J = 2.0 Hz, $H_{Ar}$), 6.73 (d, 1H, J = 2.0 Hz, $H_{Ar}$), 6.94 (d, 2H, J = 7.5 Hz, $H_{Ar}$), 7.67 (d, 2H, J = 7.5 Hz, $H_{Ar}$), 8.15 (s, 1 H, $H_{Ar}$).
- $^{13}$C RMN (62.90 MHz, $CDCl_3$): d; 13.1, 14.4, 31.0, 40.1, 40.5, 42.2, 55.3, 55.8 (2), 89.9, 93.1, 106.3, 113.5 (2), 126.6, 129.7, 130.0 (2), 130.5, 140.9. 157.6, 159.1, 162.1, 162.6, 169.9.
- SM (ionspray): m/z 439 $(M+1)^+$
- *Anal.* calculé pour $C_{25}H_{30}N_2O_5$: C, 68.47; H, 6.90; N, 6.39. Trouvé: C, 68.27; H, 6.80; N, 6.40.

**<u>EXEMPLE 16</u> : *N,N*-Diéthyl-2-[5,7-diméthoxy-3-(4-méthoxyphényl)-2-oxo-1,2-dihydro-1-quinolinyl]acétamide (Composé 18)**

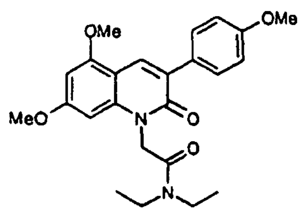

**(CRL8315)**

Sous atmosphère d'azote, 1,0 g (3,2 mmol) de composé 1 est solubilisé dans 30 ml de *N,N*-diméthylformamide (DMF) anhydre. A 0°C, 115 mg (4,8 mmol, 1,5 eq) de NaH, préalablement lavés dans de l'éther de pétrole, sont additionnés par petites portions à la solution réactionnelle (réaction exothermique). Une solution de 2-chloro-N,N-diéthylacétamide (0,88 ml, 6,4 mmol, 2 eq) diluée dans 5 ml de DMF est ajoutée au milieu. La réaction est chauffée pendant 3 h à 90°C. Après refroidissement, de l'eau est versée sur le mélange réactionnel. La solution réactionnelle est extraite par de l'acétate d'éthyle (deux fois). La phase organique obtenue est séchée sur $MgSO_4$, puis évaporée sous pression réduite. Le résidu obtenu est purifié par chromatographie sur colonne de silice ($CH_2Cl_2$/AcOEt 7:3) pour donner 820 mg (61%) de composé **18** et 408 mg (30%) de dérivé **18a**.

*Composé **18:***

- PF 178-179°C (AcOEt)
- IR (KBr) 1642, 1617, 1601 $cm^{-1}$
- $^1$ H RMN (250 MHz, $CDCl_3$): d 1.10-1.23 (m, 6H, $CH_3$), 3.38-3.49 (m, 4H, $CH_2$), 3.83 (s, 3H, $OCH_3$), 3.86 (s, 3H, $OCH_3$), 3.91 (s, 3H, $OCH_3$), 5.17 (s, 2H, $NCH_2CO$), 6.28 (d, 1H, J = 2.0 Hz, $H_{Ar}$), 6.34 (d, 1H, J = 2.0 Hz, $H_{Ar}$), 6.93 (d, 2H, J = 7.0 Hz, $H_{Ar}$), 7.66 (d, 2H, J = 7.0 Hz, $H_{Ar}$), 8.17 (s, 1H, $H_{Ar}$).
- $^{13}$C RMN (62.90 MHz, $CDCl_3$): d 13.0, 14.2, 40.9, 41.6, 45.3, 55.3, 55.5, 55.8, 90.8, 92.8, 106.3, 113.4 (2), 126.6, 129.9, 130.1 (2), 131.0, 141.7, 157.6, 159.0, 161.9, 162.3, 166.3.
- SM (ionspray): m/z 425 $(M+1)^+$
- *Anal.* calculé pour $C_{24}H_{28}N_2O_5$: C, 67.91; H, 6.65; N, 6.60. Trouvé: C, 67.62; H, 6.44; N, 6.50.

*Composé **18a**:*

***N,N*-Diéthyl-2-([5,7-diméthoxy-3-(4-méthoxyphényl)-2-quinolinyl]oxy)acetamide (18a)**

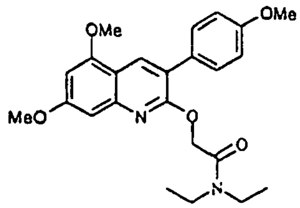

- PF 146-147°C (AcOEt/EP)
- IR (KBr) n 1654, 1624, 1517 $cm^{-1}$
- $^{1}H$ RMN (250 MHz, $CDCl_3$): d 1.14 (t, 3H, J = 7.5 Hz , $CH_3$), 1.28 (t, 3H, J = 7.5 Hz, $CH_3$), 3.36-3.47 (m, 4H; $NCH_2$), 3,85 (s, 3H, $OCH_3$), 3.91 (s, 3H, $OCH_3$), 3.93 (s, 3H, $OCH_3$), 5.17 (s, 2H, $NCH_2$), 6.38 (d, 1 H, J = 2.0 Hz, $H_{Ar}$), 6.73 (d, 1H, J = 2.0 Hz, $H_{Ar}$), 6.97 (d, 2H, J = 9.0 Hz, $H_{Ar}$), 7.75 (d, 2H, J = 9.0 Hz, $H_{Ar}$), 8.28 (s, 1 H, $H_{Ar}$).
- $^{13}C$ RMN (62.90 MHz, $CDCl_3$): d 12.9, 14.2, 40.2, 55.2, 55.4, 55.6, 63.0, 95.8, 98.4, 113.3, 113.5 (2), 121.9, 129.2, 130.6 (2), 132.5, 147.3, 156.2, 158.9, 161.1, 167.3.
- SM (ionspray): m/z 425 $(M+1)^+$
- *Anal.* calculé pour $C_{24}H_{28}N_2O_5$: C, 67.91; H, 6.65; N, 6.60. Trouvé: C, 67.85; H, 6.70; N, 6.51.

**<u>EXEMPLE 17</u> : [5,7-Diméthoxy-3-(4-méthoxyphényl)-2-oxo-1,2-dihydro-1-quinolinyl]acetonitrile (Composé 19)**

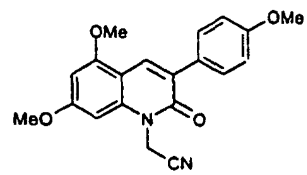

**(CRL8255)**

Sous atmosphère d'azote, 1,0 g (3,2 mmol) de composé 1 est solubilisé dans 30 ml de *N,N*-diméthylformamide (DMF) anhydre. A 0°C, 115 mg (4,8 mmol, 1,5 eq) de NaH, préalablement lavés dans de l'éther de pétrole, sont additionnés par petites portions à la solution réactionnelle (réaction exothermique). Une solution de bromoacétonitrile (0,45 ml, 6,4 mmol, 2 eq) diluée dans 5 ml de DMF est ajoutée au milieu. La réaction est chauffée pendant 3 h à 90°C. Après refroidissement, de l'eau est versée sur le mélange réactionnel. La solution réactionnelle est extraite par de l'acétate d'éthyle (deux fois). La phase organique est séchée sur $MgSO_4$, puis évaporée sous pression réduite. Le résidu obtenu est purifié par chromatographie sur colonne de silice ($CH_2Cl_2$/AcOEt 7:3) pour donner 683 mg (61%) de composé **19** et 336 mg (30%) de dérivé **19a**.

Composé **19**:

- PF 208-209°C (AcOEt)
- IR (KBr) 2216, 1660, 1607 $cm^{-1}$
- $^{1}H$ RMN (250 MHz, $CDCl_3$): d 3.84 (s, 3H, $OCH_3$), 3.94 (s, 3H, $OCH_3$), 3.95 (s, 3H, $OCH_3$), 5.29 (s, 2H, $CH_2$), 6.36 (s, 2H, $H_{Ar}$), 6.95 (d, 2H, J = 8.8 Hz, $H_{Ar}$), 7.65 (d, 2H, J = 8.8 Hz, $H_{Ar}$), 8.17 (s, 1 H, $H_{Ar}$).
- $^{13}C$ RMN (62.90 MHz, $CDCl_3$): d 30.4, 55.3, 55.8, 56.0, 90.0, 93.5, 106.2, 113.6 (2), 114.8, 126.3, 129.0, 130.0 (2), 131.7, 139.8, 158.1, 159.4, 161.1, 163.0.
- SM (ionspray): m/z 351 $(M+1)^+$
- *Anal.* calculé pour $C_{20}H_{18}N_2O_4$: C, 68.56; H, 5.18; N, 8.00. Trouvé: C, 68.30; H, 5.00; N, 7.90.

*Composé* ***19a:***

**2-([5,7-Diméthoxy-3-(4-méthoxyphényl)-2-quinolinyl]oxy)acetonitrile (Composé 19a)**

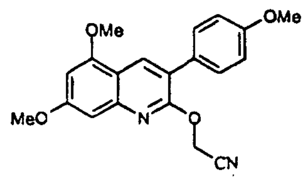

- PF 149-150°C (éther)
- IR (KBr) n 1623, 1586, 1516, 1265 $cm^{-1}$
- $^1H$ RMN (250 MHz, $CDCl_3$): d 3.87 (s, 3H, $OCH_3$), 3.95 (s, 6H, $OCH_3$), 5.17 (s, 2H, $OCH_2$), 6.45 (d, 1H, J = 2.0 Hz, $H_{Ar}$), 6.87 (d, 1 H, J = 2.0 Hz, $H_{Ar}$), 7.99 (d, 2H, J = 9.0 Hz, $H_{Ar}$), 7.54 (d, 2H, J = 9.0 Hz, $H_{Ar}$), 8.34 (s, 1 H, $H_{Ar}$).
- $^{13}C$ RMN (62.90 MHz, $CDCl_3$): d 50.1, 55.3, 55.6, 55.7, 96.9, 98.6, 113.8 (2), 113.9, 116.1, 121.3, 128.4, 130.5 (2), 133.5, 147.1, 156.3, 157.2, 129.3, 161.8.
- SM (ionspray): m/z 351 $(M+1)^+$
- *Anal.* calculé pour $C_{20}H_{18}N_2O_4$: C, 68.56; H, 5.18; N, 8.00. Trouvé: C, 68.42; H, 5.03; N, 7.88.

**<u>EXEMPLE 18</u> : 3-[5,7-Diméthoxy-3-(4-méthoxyphényl)-2-oxo-1,2-dihydro-1 - quinolinyl]propanenitrile (Composé 20)**

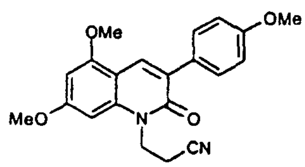

**(CRL8247)**

Sous atmosphère d'azote, 500 mg (1,6 mmol) de composé 1 et 0,8 ml (12 mmol) d'acrylonitrile sont solubilisés dans 10 ml de N,N-diméthylformamide (DMF) anhydre. A 0°C, 2 gouttes de triton B sont additionnées à la solution réactionnelle. La réaction est suivie par plaque CCM. En fin de réaction, les solvants sont évaporés. Le résidu est repris dans de l'acétate d'éthyle et lavé plusieurs fois par de l'eau. La phase organique est séchée sur $MgSO_4$ puis évaporée sous pression réduite. Le résidu obtenu est purifié par chromatographie sur colonne de silice ($CH_2Cl_2$/AcOEt 7:3) pour donner 365 mg (63%) du composé **20**.

- PF 156-157°C (AcOEt/EP)
- IR (KBr) 2241, 1639 $cm^{-1}$
- $^1H$ RMN (250 MHz, $COCl_3$): d 2.88 (t, 2H, J = 7.1 Hz, $CH_2CN$), 3.84 (s, 3H, $OCH_3$), 3.93 (s, 3H, $OCH_3$), 3.94 (s, 3H, $OCH_3$). 4.59 (t, 2H, J = 7.1 Hz, $NCH_2$), 6.33 (d, 1H, J = 1.8 Hz, $H_{Ar}$), 6.45 (d, 1 H, J = 1.8 Hz, $H_{Ar}$), 6.95 (d, 2H, J = 9.0 Hz, $H_{Ar}$), 7.66 (d, 2H, J = 9.0 Hz, $H_{Ar}$), 8.17 (s, 1 H, $H_{Ar}$).
- $^{13}C$ RMN (62.90 MHz, $COCl_3$): d; 15.9, 39.4, 55.3, 55.7, 55.9, 89.9, 93.0, 106.4, 113.6 (2), 117.7, 126.6, 129.2, 130.0 (2), 131.1, 140.5, 158.0, 159.3, 161.8, 162.7.
- SM (ionspray): m/z 365 $(M+1)^+$
- *Anal.* calculé pour $C_{21}H_{20}N_2O_4$: C, 69.22; H, 5.53; N, 7.69. Trouvé: C, 69.40; H, 5.40; N, 7.80.

**EXEMPLE 19 : 1-[2-(1*H*-1,2,3,4-Tétrazol-5-yl)éthyl]-5,7-diméthoxy-3-(4-méthoxyphényl)-1,2-dihydro-2-quinolinone (Composé 21)**

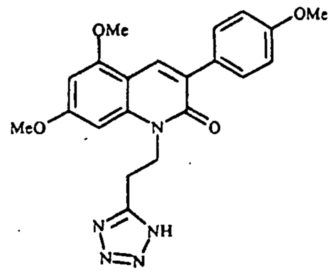

**(CRL8256)**

Sous atmosphère d'argon, 350 mg (0.90 mmol) de composé **20** et 0,42 ml (1,53 mmol) d'azoture de tributylétain sont solubilisés dans 20 ml de toluène anhydre. La solution réactionnelle est agitée à 105°C pendant 65 h. Après refroidissement, le solvant est évaporé sous pression réduite. Le résidu obtenu est purifié par chromatographie sur colonne de silice ($CH_2Cl_2$/MeOH 9:1) pour donner 333 mg (85%) du composé **21**.

- PF 234-235°C (lavage $Et_2O$)
- IR (KBr) 1618, 1594 $cm^{-1}$
- $^1H$ RMN (250 MHz, DMSO-$d_6$): d 3.33 (t, 2H, J = 6.0 Hz, $CH_2$), 3.79 (s, 3H, $OCH_3$), 3.89 (s, 3H, $OCH_3$), 3.93 (s, 3H, $OCH_3$), 4.67 (t, 2H, J = 6.0 Hz, $CH_2$), 6.48 (s, 1 H, $H_{Ar}$), 6.52 (s, 1H, $H_{Ar}$), 6.96 (d, 2H, J = 9.0 Hz, $H_{Ar}$), 7.59 (d, 2H, J = 9.0 Hz, $H_{Ar}$), 8.01 (s, 1 H, $H_{Ar}$).
- $^{13}C$ RMN (62.90 MHz, DMSO-$d_6$): d 21.4, 40.8, 55.1, 55.6, 56.1, 90.4, 93.0, 105.0, 113.3(2), 125.5, 129.3, 129.6, 129.7 (2), 140.4, 153.7, 157.2, 158.7, 160.7, 162.4.
- SM (ionspray): m/z 408 $(M+1)^+$
- *Anal.* calculé pour $C_{21}H_{20}N_5O_4$: C, 61.91; H, 5.20; N, 17.19. Trouvé: C, 62.00; H, 5.19; N, 17.30.

**EXEMPLE 20 : 1-[3-(Diméthylamino)propyl]-5,7-diméthoxy-3-(4-méthoxyphényl)-1,2-dihydro-2-quinolinone (Composé 22)**

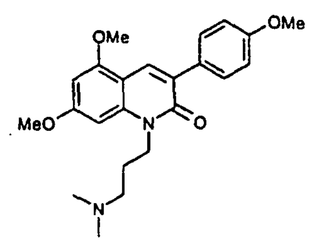

**(CRL8254)**

Sous atmosphère d'azote, 1,0 g (3,2 mmol) de composé 1 est solubilisé dans 20 ml de *N,N*-diméthylformamide (DMF) anhydre. A 0°C, 115 mg de NaH (4,8 mmol, 1,5 eq), préalablement lavés dans de l'éther de pétrole, sont additionnés par petites portions à la solution réactionnelle (réaction exothermique). Une solution de chlorure de 3-diméthylaminopropyle (777 mg, 7,3 mmol, 2,25 eq) dans 5 ml de DMF est ajoutée au milieu. La réaction est chauffée pendant 2-3 h à 90°C. Après refroidissement, de l'eau est versée sur le mélange réactionnel, puis ce dernier est agitée pendant 15 min. La solution est extraite par de l'acétate d'éthyle (deux fois). La phase organique est séchée sur $MgSO_4$, puis évaporée sous pression réduite. Le résidu obtenu est purifié par chromatographie sur colonne de silice ($Et_2O$/MeOH 8:2, puis $CH_2Cl_2$/MeOH 9:1) pour donner 887 mg (70%) de composé 22 et 317 mg (25%) de dérivé **22a**.

*Composé* ***22***:

- PF 94-95°C (lavage $Et_2O$)
- IR (KBr) 1635, 1617, 1598 $cm^{-1}$
- $^1H$ RMN (250 MHz, $CDCl_3$): d 1.91-2.04 (m, 2H, $CH_2$), 2.28 (s, 6H, $CH_3$), 2.46 (t, 2H, J = 7.2 Hz, $CH_2$), 3.83 (s, 3H, $OCH_3$), 3.91 (s, 3H, $OCH_3$), 3.93 (s, 3H, $OCH_3$), 4.36 (t, 2H, J = 7.2 Hz, $CH_2$), 6.29 (d, 1 H, J = 2.0 Hz, $H_{Ar}$), 6.54 (d, 1H, J = 2.0 Hz, $H_{Ar}$), 6.94 (d, 2H, J = 8.8 Hz, $H_{Ar}$), 7.68 (d, 2H, J = 8.8 Hz, $H_{Ar}$), 8.13 (s, 1H, $H_{Ar}$).
- $^{13}C$ RMN (62.90 MHz, $CDCl_3$): d 25.5, 41.8, 45.6 (2), 55.4, 55.5, 55.8, 57.1, 90.3, 92.6, 106.4, 113.5 (2), 126.9, 130.0, 130.1 (2), 130.3, 141.1, 157.6, 159.1, 162.0, 162.3.
- SM: m/z 397 $(M+1)^+$
- *Anal.* calculé pour $C_{23}H_{28}N_2O_4$: C, 69.68; H, 7.12; N, 7.07. Trouvé: C, 69.40; H, 6.97; N, 7.15.

*Composé* ***22a***:

***N,N*-Diméthyl-2-[5,7-diméthoxy-3·(4-méthoxyphényl)-2-quinolyl]oxy-1-propanamine (Composé 22a)**

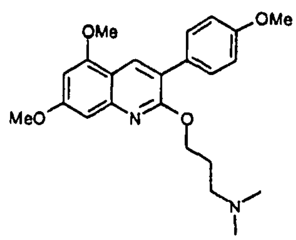

- PF 54-55°C (éther)
- IR(KBr)n 1621, 1584, 1515 $cm^{-1}$
- $^1H$ RMN (250 MHz, $CDCl_3$): d 1.96-2.07 (m, 2H, $CH_2$), 2.26 (s, 6H, $NCH_3$), 2.47 (t, 2H, J = 6.5 Hz, $NCH_2$), 3,84 (s, 3H, $OCH_3$), 3.92 (s, 6H, $OCH_3$), 4.53 (t, 2H, J = 6.5 Hz, $OCH_2$), 6.39 (d, 1H, J = 2.2 Hz, $H_{Ar}$), 6.82 (d, 1H, J = 2.2 Hz, $H_{Ar}$), 6.96 (d, 2H, J = 8.8 Hz, $H_{Ar}$), 7.57 (d, 2H, J = 8.8 Hz, $H_{Ar}$), 8.26 (s, 1 H, $H_{Ar}$).
- $^{13}C$ RMN (62.90 MHz, $CDCl_3$): d 27.0, 45.4 (2), 55.2, 55.5 (2), 56.6, 64.2, 95.8, 98.5, 112.7, 113.4 (2), 121.9, 129.6, 130.5 (2), 132.1, 147.9, 156.2, 158.8, 160.2, 161.2.
- SM (ionspray): m/z 397 $(M+1)^+$
- *Anal.* calculé pour $C_{23}H_{28}N_2O_4$: C, 69.68; H, 7.12; N, 7.07. Trouvé: C, 69.53; H, 6.92; N, 7.16.

<u>EXEMPLE 21 :</u> **1-[2-(Diméthylamino)éthyl]-5,7-diméthoxy-3-(4-méthoxyphényl)-1,2-dihydro-2-quinolinone (Composé 23)**

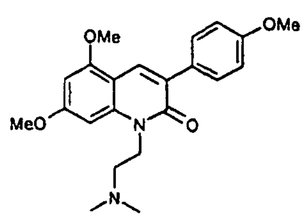

**(CRL8316)**

Sous atmosphère d'azote, 250 mg (0,8 mmol) de composé 1 sont solubilisés dans 15 ml de *N,N*-diméthylformamide (DMF) anhydre. A 0°C, 29 mg (1,2 mmol, 1,5 eq) de NaH, préalablement lavés dans de l'éther de pétrole, sont additionnés par petites portions à la solution réactionnelle (réaction exothermique). Une solution de chlorure de 2-diméthylaminoéthyle (230 mg, 2,5 mmol, 3 eq) dans 5 ml de DMF est ajoutée au milieu. La réaction est chauffée pendant 2-3 h à 90°C. Après refroidissement, de l'eau est versée sur le mélange réactionnel, puis ce dernier est agité pendant 15 min. La solution est extraite par de l'acétate d'éthyle (deux fois). La phase organique est séchée sur $MgSO_4$, puis évaporée sous pression réduite. Le résidu obtenu est purifié par chromatographie sur colonne de silice ($Et_2O$/AcOEt 3:7, puis $CH_2Cl_2$/MeOH 9:1) pour donner 205 mg (67%) de composé **23** et 92 mg (30%) de dérivé **23a.**

*Composé **23***:

- PF 113-114°C (lavage $Et_2O$)
- IR (KBr) 1645, 1617, 1604 $cm^{-1}$
- $^1H$ RMN (250 MHz, $CDCl_3$): d 2.39 (s, 6H, $CH_3$), 2.65 (t, 2H, J = 7.8 Hz, $CH_2$), 3.82 (s, 3H, $OCH_3$), 3.90 (s, 3H, $OCH_3$), 3.91 (s, 3H, $OCH_3$), 4.44 (t, 2H, J = 7.8 Hz, $CH_2$), 6.28 (d, 1H, J = 2.0 Hz, $H_{Ar}$), 6.49 (d, 1H, J = 2.0 Hz, $H_{Ar}$), 6.94 (d, 2H, J = 8.8 Hz, $H_{Ar}$), 7.67 (d, 2H, J = 8.8 Hz, $H_{Ar}$, 8.12 (s, 1 H, $H_{Ar}$).
- $^{13}C$ RMN (62.90 MHz, $CDCl_3$): d 41.6, 45.8 (2), 55.3, 55.6, 55.8 (2), 90.1, 92.7, 106.4, 113.5 (2), 126.9, 129.8, 130.1 (2), 130.4, 141.1, 157.7, 159.1, 161.9, 162.4.
- SM (ionspray): m/z 383 $(M+1)^+$
- *Anal.* calculé pour $C_{22}H_{26}N_2O_4$: C, 69.09; H, 6.85; N, 7.32. Trouvé: C, 69.37; H, 6.98; N, 7.51.

*Composé **23a***:

**N,N-Dimethyl-2-[5,7 -diméthoxy-3-(4-méthoxyphényl)-2-quinolyl]oxy-1-éthanamine**

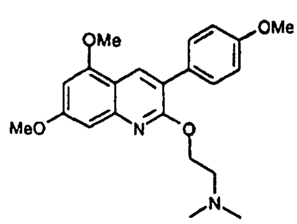

- PF 49-50°C (lavage éther)
- IR (KBr) n 1621, 1584, 1515 $cm^{-1}$
- $^1H$ RMN (250 MHz, $CDCl_3$): d 2.31 (s, 6H, $NCH_3$), 2.77 (t, 2H, J = 6.0 Hz, $NCH_2$), 3,85 (s, 3H, $OCH_3$), 3.93 (s, 6H, $OCH_3$), 4.62 (t, 2H, J = 6.0 Hz, $OCH_2$), 6.39 (d, 1 H, J = 2.0 Hz, $H_{Ar}$), 6.81 (d, 1H, J = 2.0 Hz, $H_{Ar}$), 6.94 (d, 2H, J = 8.8 Hz, $H_{Ar}$), 7.58 (d, 2H, J = 8.8 Hz, $H_{Ar}$), 8.25 (s, 1H, $H_{Ar}$).
- $^{13}C$ RMN (62.90 MHz, $CDCl_3$): d 45.7 (2), 55.3, 55.5 (2), 57.8, 63.8, 95.9, 98.5, 112.9, 113.4 (2), 122.0, 129.5, 130.6 (2), 132.3, 147.8, 156.3, 158.9, 160.0, 161.3.
- SM (ionspray): m/z 383 (M+1)+
- *Anal.* calculé pour $C_{22}H_{26}NO_4$: C, 69.09; H, 6.85; N, 7.32. Trouvé: C, 69.30; H, 6.70; N, 7.29.

**EXEMPLE 22 : 8,10-Diméthoxy-6-(4-méthoxyphényl)-2,3-dihydro-1*H*,5*H*-pyrido[3,2,1-*ij*]quinoline-1,5-dione (Composé 24)**

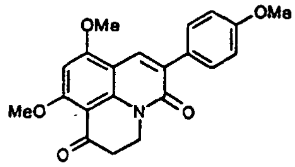

(CRL8285)

Sous atmosphère d'azote, 63 mg de $P_2O_5$ et 500 mg de PPA sont introduits dans un ballon puis le mélange est agité pendant 1 h à 120°C. Le composé 16 (100 mg, 0,26 mmol) est additionné, puis la réaction est agitée pendant 45 min à 120°C. Après refroidissement, une solution de soude 2N est additionnée jusqu'à l'obtention d'un pH= 6-7. Le produit brut est extrait par le $CH_2Cl_2$, (deux fois). La phase organique est séchée sur $MgSO_4$, puis évaporée sous pression réduite. Le résidu obtenu est purifié par chromatographie sur colonne de silice ($CH_2Cl_2$/MeOH 98:2) pour donner 62 mg (65%) du composé **24**.

- PF 240-241°C ($CH_2Cl_2$/EP)
- IR (KBr) 1678, 1649, 1634 $cm^{-1}$
- $^1H$ RMN (250 MHz, $CDCl_3$): d 2.83 (t, 2H, J = 7.0 Hz, $COCH_2$), 3.85 (s, 3H, $OCH_3$), 4.04 (s, 6H, $OCH_3$), 4.54 (d, 2H, J = 7.0 Hz, $NCH_2$), 6.32 (s, 1H, $H_{Ar}$), 6.96 (d, 2H, J = 7.5 Hz, $H_{Ar}$), 7.68 (d, 2H, J = 7.5 Hz, $H_{Ar}$), 8.12 (s, 1H, $H_{Ar}$).
- $^{13}C$ RMN (62.90 MHz, $CDCl_3$): d 37.6, 40.3, 55.3, 56.1, 56.5, 88.9, 103.5, 104.8, 113.6 (2), 127.6, 129.0, 129.8, 130.0 (2), 142.9, 159.4, 161.6, 161.7, 163.7, 190.3.
- SM (ionspray): m/z 366 $(M+1)^+$
- *Anal.* calculé pour $C_{21}H_{19}NO_5$: C, 69.03; H, 5.24; N, 3.83. Trouvé: C, 68.80; H, 5.34; N, 4.00.

**EXEMPLE 23 :**

**a) Iodure de 5,7-diméthoxy-3-(4-méthoxyphényl)-1-méthyl-2-(méthylsulfanyl)quinolium (Composé 25)**

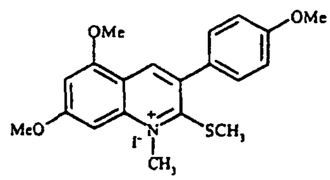

Sous atmosphère d'azote, 682 mg (2.0 mmol) de composé 10 sont solubilisés dans 30 ml de THF anhydre. A température ambiante, 3,5 ml d'iodure de méthyle (56 mmol, 28 eq) dilués dans 5 ml de THF sont additionnés, puis la réaction est agitée pendant 18 h sous atmosphère inerte. Le précipité observé en fin de réaction est filtré sur verre fritté (lavage avec THF) pour donner 761 mg (79%) du composé 25.

- PF 156-157°C (lavage THF)
- $^1H$ RMN (250 MHz, $CDCl_3$): d 2.44 (s, 3H, $SCH_3$), 3.88 (s, 3H, $OCH_3$), 4.02 (s, 3H, $OCH_3$), 4.28 (s, 3H, $OCH_3$), 5.03 (s, 3H, $NCH_3$). 6.70 (d, 1H, J = 1,5 Hz, $H_{Ar}$), 7.03 (d, 2H, J = 8.5 Hz, $H_{Ar}$), 7.37 (s large, 1H, $H_{Ar}$), 7.45 (d, 2H, J = 8.5 Hz, $H_{Ar}$), 8.71 (s, 1H, $H_{Ar}$).
- $^{13}C$ RMN (62.90 MHz, $CDCl_3$): d 20.8, 46.5, 55.4, 56.7, 58.5, 92.9, 101.0, 114.4 (2), 117.6, 125.8, 128.9, 130.7 (2), 135.9, 138.8, 144.2, 157.4, 160.3, 167.7.

**Ce composé est rapidement utilisé dans l'étape suivante.**

**b) 5,7-Diméthoxy-3-(4-méthoxyphényl)-1-méthyl-1,2-dihydro-2-quinolinone-2-phénylhydrazone (Composé 26)**

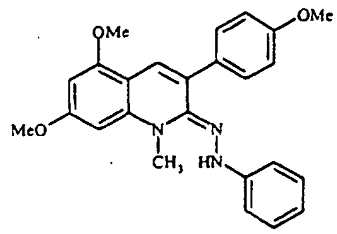

**(CRL8270)**

Dans un tube scellé, 200 mg (0,4 mmol) de composé 25 et 0,28 ml (2,8 mmol, 7 eq) de phénylhydrazine sont solubilisés dans 5 ml d'éthanol anhydre. Le mélange réactionnel est chauffé 18 h à 90°C. Après refroidissement, l'éthanol est évaporé sous pression réduite. Le résidu est repris dans du $CH_2Cl_2$, puis lavé deux fois avec une solution d'hydrogénocarbonate de sodium. La phase organique est séchée sur $MgSO_4$, puis évaporée sous pression réduite. Le résidu est repris dans le méthanol où le produit final précipite. Ce dernier est isolé par filtration (lavage MeOH) pour donner 102 mg (60%) du composé **26**.

- PF 148-149°C (lavage MeOH)
- IR (KBr) 3340, 1602, 1599 $cm^{-1}$
- $^1H$ RMN (250 MHz, $CDCl_3$): d 3.58 (s, 3H, $CH_3$), 3.85 (s, 3H, $OCH_3$), 3.86 (s, 3H, $OCH_3$), 3.88 (s, 3H, $OCH_3$), 6.04 (s, 1 H, $H_{Ar}$), 6.14 (s, 1H, $H_{Ar}$), 6.48 (s large, 1H, NH), 6.56-6.67 (m, 3H, $H_{Ar}$), 6.94 (d, 2H, J = 8.8 Hz, $H_{Ar}$), 7.11 (t, 2H, J = 7.7 Hz, $H_{Ar}$), 7.28 (s, 1 H, $H_{Ar}$), 7.28-7.34 (m, 2H, $H_{Ar}$).
- $^{13}C$ RMN (62.90 MHz, $CDCl_3$): d 33.3, 55.4 (2), 55.6, 89.6 (2), 111.6, 113.8 (2), 117.8, 125.9, 128.3, 128.9 (3), 129.5 (2), 130.5, 146.2, 157.2, 159.2 (2), 162.3.
- SM (ionspray): m/z 416 $(M+1)^+$
- *Anal*. calculé pour $C_{25}H_{25}N_3O_3$: C, 72.27; H, 6.06; N, 10.11. Trouvé: C, 72.01; H, 5.86; N, 10.02.

**EXEMPLE 24 : 5,7-Diméthoxy-3-(4-méthoxyphényl)-1-méthyl-1,2-dihydro-2-quinolinone-2-(2-pyridinyl)hydrazone (Composé 27)**

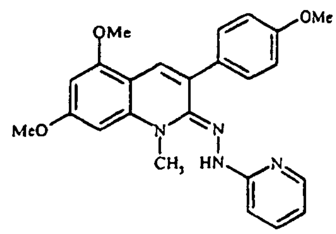

**(CRL8266)**

Dans un tube scellé, 150 mg (0,31 mmol) de composé **25** et 237 mg (2,2 mmol, 7 eq) de 2-hydrazinopyridine sont solubilisés dans 5 ml d'éthanol anhydre. La mélange réactionnel est chauffé 18 h à 90°C. Après refroidissement, l'éthanol est évaporé sous pression réduite. Le résidu est repris dans du $CH_2Cl_2$ puis lavé deux fois avec une solution d'hydrogénocarbonate de sodium. La phase organique est séchée sur $MgSO_4$ puis évaporée sous pression réduite. Le résidu est repris dans le méthanol ou le produit final précipite. Ce dernier est filtré (lavage MeOH) pour donner 75 mg (58%) du composé orangé **27**.

- PF 182-183°C (lavage MeOH)
- IR (KBr) 3353, 1628, 1593 $cm^{-1}$
- $^1H$ RMN (250 MHz, $CDCl_3$): d 3.61 (s, 3H, $NCH_3$), 3.87 (s, 6H, $OCH_3$), 3.90 (s, 3H, $OCH_3$), 6.07 (s, 1H, $H_{Ar}$), 6.17 (s, 1H, $H_{Ar}$), 6.51-6.55 (m, 1H, $H_{pyr}$), 6.94-7.01 (m, 3H, $H_{Ar}$ + $H_{pyr}$), 7.14 (s large, 1H, NH), 7.32-7.36 (m, 3H, $H_{Ar}$) 7.48 (t, 1H, J = 7.3 Hz, $H_{pyr}$), 7.91 (d, 1 H, J = 4.3 Hz, $H_{pyr}$).

- $^{13}C$ RMN (62.90 MHz, $CDCl_3$): d 33.2, 55.3, 55.4, 55.6, 89.7 (2), 104.7, 105.7, 113.4, 114.1 (2), 122.9, 129.0, 129.2 (2), 130.1, 137.5, 140.7, 143.7. 147.8, 157.3, 157.4, 159.2, 162.4.
- SM (ionspray): m/z 417 $(M+1)^+$
- *Anal.* calculé pour $C_{24}H_{24}N_4O_3$: C, 69.21; H, 5.81; N, 13.45. Trouvé: C, 69.50; H, 6.04; N, 13.28.

**EXEMPLE 25**

**a) N-(2-Méthoxyphényl)-2-(4-méthoxyphényl)acétamide (Composé 30)**

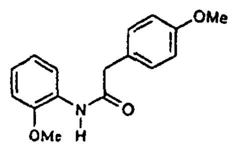

Sous atmosphère d'azote, 0,46 ml (4,1 mmol) d'o-anisidine sont dilués dans du toluène (7 ml) à 0°C. Une solution de chlorure de 4-méthoxyphénylacétyle (0,63 ml, 4,1 mmol) dans 5 ml de toluène est ajoutée goutte à goutte au milieu. Le mélange réactionnel est agité à température ambiante pendant 1 h, puis le milieu est hydrolysé par une solution froide d'hydrogénocarbonate de sodium. Le système biphasique est agité vigoureusement pendant 30 min, puis la phase organique est recueillie. La phase aqueuse est extraite par de l'acétate d'éthyle (deux fois). La phase organique est séchée sur $MgSO_4$, puis évaporée sous pression réduite. Le résidu obtenu est repris dans de l'éther de pétrole, cette opération entraînant la précipitation du dérivé recherché. Les cristaux, ainsi formés, sont recueillis par filtration pour donner 1,0 g (91 %) du composé **30**.

- PF 47-48°C (toluène)
- IR (KBr) n 3375, 1667, 1597 $cm^{-1}$
- $^1H$ RMN (250 MHz, $CDCl_3$): d 3,68 (s, 2H, $CH_2$), 3.72 (s, 3H, $OCH_3$), 3.82 (s, 3H, $OCH_3$), 6.79 (dd 1 H, J = 1.5, 8.0 Hz, $H_{Ar}$), 6.89-7.03 (m, 4H, $H_{Ar}$), 7.25 (d, 2H, J = 8.8 Hz, $H_{Ar}$), 7.79 (s large, 1H, NH), 8.33 (dd, 1H, J = 1,5, 8.0 Hz, $H_{Ar}$).
- $^{13}C$ RMN (62.90 MHz, $CDCl_3$): d 44.2, 55.3, 55.7, 109.9, 114.4 (2), 119.5, 121.0, 123.6, 126.6, 127.6, 130.7 (2), 147.8, 158.9, 169.3.
- SM (ionspray): m/z 272 $(M+1)^+$

**b) 8-Méthoxy-3-(4-méthoxyphényl)-1,2-dihydro-2-quinolinone (Composé 31)**

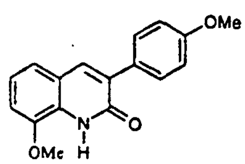

Sous atmosphère d'azote et à -30°C, 0,31 ml (4,0 mmol, 1,5 eq) de *N,N*-diméthylformamide sont additionnés goutte à goutte à 1,75 ml (19 mmol, 7,5 eq) de $POCl_3$. Le milieu est agité pendant 15 min à -30°C, puis 723 mg d'amide 30 (2,7 mmol) sont additionnés. Sous agitation, le mélange réactionnel est ramené à température ambiante, puis la réaction est chauffée à 75°C pendant 1,5 h. A la fin de la réaction, cette solution est versée sur de la glace pilée, neutralisée par une solution d'ammoniaque à 30%, puis extraite par du dichlorométhane. La phase organique est séchée sur $MgSO_4$, puis évaporée. Le résidu obtenu est dissous dans 4,75 ml d'acide acétique glacial et 0,15 ml d'eau, puis la solution finale est chauffée à reflux pendant 3 h. L'acide acétique est évaporé. Le résidu est solubilisé dans

l'eau, neutralisé par une solution d'hydroxyde de sodium à 25%, puis finalement extrait avec du dichlorométhane. La phase organique est séchée sur $MgSO_4$, puis évaporée sous pression réduite. Le résidu est repris dans de l'acétate d'éthyle, cette opération entraînant la précipitation du produit final. Les cristaux ainsi obtenus sont filtrés pour donner 75 mg (10%) du composé **31**.

Le rendement global de la synthèse mise en oeuvre pour obtenir le composé 31 est de 9%.

- PF 148-149°C (AcOEt)
- IR (KBr) n 1652, 1625, 1606, 1541 $cm^{-1}$
- $^1H$ RMN (250 MHz, DMSO-$d_6$): d 3,79 (s, 3H, $OCH_3$), 3.90 (s, 3H, $OCH_3$), 6.99 (d, 2H, J = 8.8 Hz, $H_{Ar}$), 7.09-7.15 (m, 2H, $H_{Ar}$), 7.29 (dd, 1H, J = 3.2, 6.0 Hz, $H_{Ar}$), 7.74 (d, 2H, J = 8.8 Hz, $H_{Ar}$), 8.03 (s, 1H, $H_{Ar}$), 10.90 (s large, 1 H, NH).
- $^{13}C$ RMN (62.90 MHz, DMSO-$d_6$): d 55.2, 56.0, 110.5, 113.4 (2), 119.6, 120.0, 121.8, 127.9, 128.5, 129.9 (2), 131.6, 136.4, 145.3, 159.1, 160.7.
- SM (ionspray): m/z 282 $(M+1)^+$
- *Anal*. calculé pour $C_{17}H_{15}NO_3$: C, 72.58; H, 5,37; N, 4.98. Trouvé: C, 72.50; H, 5.50; N, 4.83.

**EXEMPLE 26**

**a) *N*-(2,5-Diméthoxyphényl)-2-(4-méthoxyphényl)acétamide (Composé 32)**

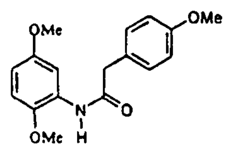

Sous atmosphère d'azote, 1,0 g (6,5 mmol) de 2,5-diméthoxyaniline est solubilisé dans du toluène (7 ml) à 0°C. Une solution de chlorure de 4-méthoxyphénylacétyle (1,0 ml, 6,5 mmol) diluée dans 5 ml de toluène est ajoutée goutte à goutte au milieu. Le mélange réactionnel est agité à température ambiante pendant 1 h, puis le milieu est hydrolysé par une solution froide d'hydrogénocarbonate de sodium. Le système biphasique est agité vigoureusement pendant 30 min, puis la phase organique est recueillie. La phase aqueuse est extraite par de l'acétate d'éthyle (deux fois). La phase organique est séchée sur $MgSO_4$, puis évaporée sous pression réduite. Le résidu obtenu est repris dans un minimum d'éther de pétrole, cette opération entraînant la précipitation du produit final. Les cristaux formés sont filtrés sur verre fritté pour donner 1,83 g (93%) du composé **32**.

- PF 89-90°C (toluène)
- $^1H$ RMN (250 MHz, $CDCl_3$): d 3,67 (s, 3H, $OCH_3$), 3.68 (s, 2H, $CH_2$), 3.75 (s, 3H, $OCH_3$), 3.81 (s, 3H, $OCH_3$), 6.53 (dd, 1H, J = 3.0, 9.0 Hz, $H_{Ar}$), 6.71 (d, 1H, J = 9.0 Hz, $H_{Ar}$), 6.92 (d, 2H, J = 8.8 Hz, $H_{Ar}$), 7.25 (d, 2H, J = 8.8 Hz, $H_{Ar}$), 7.82 (s large, 1H, NH), 8.80 (d, 1 H, J = 3.0 Hz, $H_{Ar}$).
- $^{13}C$ RMN (62.90 MHz, $CDCl_3$): d 44.2, 55.3, 55.7, 56.3. 105.5, 108.6, 110.9, 114.4 (2), 126.4, 128.3, 130.6 (2), 142.0, 153.9, 158.9, 169.3.
- SM (ionspray): m/z 302 $(M+1)^+$

**b) 5,8-Diméthoxy-3-(4-méthoxyphényl)-1,2-dihydro-2-quinolinone (Composé 33)**

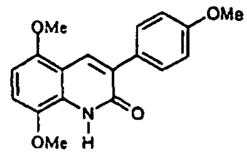

**(CRL8336)**

Sous atmosphère d'azote et à -30°C, 0,31 ml (4,0 mmol, 1,5 eq) de N,N-diméthylformamide sont additionnés goutte à goutte à 1,75 ml (20 mmol, 7,5 eq) de $POCl_3$. Le milieu est agité pendant 15 min à -30°C, puis 813 mg d'amide **32** (2,7 mmol) sont additionnés. Sous agitation, le mélange réactionnel est ramené à température ambiante, puis la réaction est chauffée à 75°C pendant 1,5 h. Une fois ce laps de temps écoulé, la solution est versée sur de la glace pilée, neutralisée par une solution d'ammoniaque à 30%, puis extraite par du dichlorométhane. La phase organique est séchée sur $MgSO_4$, puis évaporée. Le résidu obtenu est repris dans 4,75 ml d'acide acétique glacial et 0,15 ml d'eau, puis la solution finale est chauffée à reflux pendant 3 h. L'acide acétique est évaporé. Le résidu est solubilisé dans l'eau, neutralisé par une solution d'hydroxyde de sodium à 25%, puis finalement extrait avec du dichlorométhane. La phase organique est séchée sur $MgSO_4$, puis évaporée sous pression réduite. Le résidu est repris dans de l'acétate d'éthyle, cette opération entraînant la précipitation du composé final. Les cristaux ainsi obtenus sont filtrés pour donner 378 mg (45%) du composé **33**.

Le rendement global de la synthèse chimique pour obtenir le composé 33 est de 42%.

- PF 186-187°C (AcOEt)
- IR (KBr) n 1639, 1571, 1515 $cm^{-1}$
- $^1H$ RMN (250 MHz, $CDCl_3$): d 3,85 (s, 3H, $OCH_3$), 3.90 (s, 3H, $OCH_3$), 3.92 (s, 3H, $OCH_3$). 6.49 (d, 1H, J = 8.7 Hz, $H_{Ar}$), 6.84 (d, 1 H, J = 8.7 Hz, $H_{Ar}$), 6.97 (d, 2H, J = 8.8 Hz, $H_{Ar}$), 7.74 (d, J = 8.8 Hz, $H_{Ar}$), 8.19 (s, 1 H, $H_{Ar}$), 9.25 (s large, 1 H, NH).
- $^{13}C$ RMN (62.90 MHz, $CDCl_3$): d 55.2, 55.8, 56.2, 101.1, 109.7, 111.4, 113.6 (2), 128.7, 128.8, 130.0 (2), 131.4, 131.7, 139.4, 149.8, 159.5, 161.3.
- SM (ionspray): m/z 312 $(M+1)^+$
- *Anal.* calculé pour $C_{18}H_{17}NO_4$: C, 69.44; H, 5.50; N, 4.50. Trouvé: C, 69.71; H, 5.59; N, 4.70.

**<u>EXEMPLE 27 :</u> 5,7-Diméthoxy-3-phényl-1,2-dihydro-2-quinolinone (Composé 34)**

**a) *N*-(3,5-Diméthoxyphényl)-2-phénylacétamide (Composé 35)**

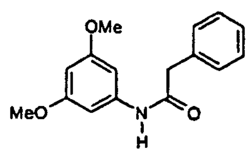

Sous atmosphère d'azote, 1,0 g (6,5 mmol) de 3,5-diméthoxyaniline sont solubilisés dans du toluène (14 ml) à 0°C. Une solution de chlorure de phénylacétyle (0,86 ml, 6,5 mmol) dans 10 ml de toluène est ajoutée goutte à goutte au milieu. Le mélange réactionnel est agité à température ambiante pendant 1 h, puis le milieu est hydrolysé par une solution froide d'hydrogénocarbonate de sodium. Le système biphasique est agité vigoureusement pendant 30 min, puis la phase organique est recueillie. La phase aqueuse est extraite par de l'acétate d'éthyle (deux fois). La phase organique est séchée sur $MgSO_4$, puis évaporée. Le résidu obtenu est cristallisé dans le toluène pour conduire à 1,55 g (87%) du composé **35**.

- PF 109-111 °C (toluène)
- IR (KBr) n 3286, 1657, 1616 $cm^{-1}$
- $^1H$ RMN (250 MHz, $CDCl_3$): d 3.70 (s, 2H, $CH_2$), 3.74 (s, 6H, $OCH_3$), 6.21 (t, 1H, J = 2.2 Hz, $H_{Ar}$), 6.66 (d, 2H, J = 2.2 Hz, $H_{Ar}$), 7.09 (s large, 1H, NH), 7.30-7.40 (m, 5H, $H_{Ar}$).
- $^{13}C$ RMN (62.90 MHz, $COCl_3$): d 44.9, 55.4 (2), 96.8, 97.9 (2), 127.7, 129.2 (2), 129.5 (2), 134.3, 139.4, 161.0 (2), 169.1.
- SM (ionspray): 272 $(M+1)^+$

**b) 2-Chloro-5,7-diméthoxy-3-phényl-1,2-dihydroquinoline (Composé 36)**

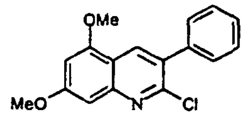

Sous atmosphère d'azote et à -30°C, 0,64 ml (8,3 mmol, 1,5 eq) de N,N-diméthylformamide sont additionnés goutte à goutte à 3,8 ml (41,0 mmol, 7,5 eq) de $POCl_3$. Le milieu est agité pendant 15 min à -30°C, puis 1,5 g d'amide 35 (5,5 mmol) sont additionnés. Sous agitation, le mélange réactionnel est ramené à température ambiante, puis la réaction est chauffée à 75°C pendant 2,5 h. A la fin de la réaction, cette solution est versée sur de la glace pilée, neutralisée par une solution d'ammoniaque à 30%, puis extraite par du dichlorométhane. La phase organique est séchée sur $MgSO_4$, puis évaporée. Le résidu obtenu est purifié par chromatographie sur colonne de silice (éluant: AcOEt/EP 3:7) pour donner 800 mg (49%) du composé 36.

- PF 148-150°C
- $^1H$ RMN (250 MHz, $CDCl_3$): d 3.93 (s, 6H, $OCH_3$), 6.51 (d, 1 H, J = 2.2 Hz, $H_{Ar}$), 6.97 (d, 1 H, J = 2.2 Hz, $H_{Ar}$), 7.40-7.54 (m, 5H, $H_{Ar}$), 8.36 (s, 1 H, $H_{Ar}$).
- $^{13}C$ RMN (62.9 MHz, $CDCl_3$): d 55.6, 55.8, 98.6, 98.8, 115.6, 127.9, 128.1 (2), 129.7 (2), 131.2, 133.9, 138.1, 149.2, 150.2, 156.1, 162.3.
- SM (ionspray): m/z 301 $(M+1)^+$, 303 $(M+3)^+$

**c) 5,7-Diméthoxy-3-phényl-1,2-dihydro-2-quinolinone (Composé 34)**

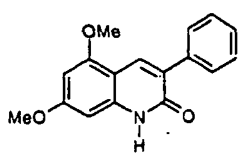

**(CRL8330)**

Sous atmosphère d'azote, 1,52 g (9,9 mmol) de 3,5-diméthoxyaniline et 2,30 g (12 mmol, 1,2 eq) d'a-formylphénylacétate d'éthyle sont mélangés dans un ballon. Le milieu est agité pendant 1 h à température ambiante. Une solution de polyphosphate de triméthylsilyle (PPSE), fraîchement préparée à partir de 4,56 g (0,03 mol) de $P_2O_5$, 10,9 ml (0,17 mol) d'hexaméthyldisiloxane et 50 ml de 1,2-dichloroéthane, est additionnée. Le mélange final est porté à 100°C pendant 2 h. Le chauffage est stoppé, puis de la glace est ajoutée au mélange réactionnel. Ce dernier est ensuite neutralisé par adjonction d'une solution saturée d'hydrogénocarbonate de sodium (addition par petites portions, réaction exothermique). Le produit est extrait par du dichlorométhane (grande quantité à utiliser). La phase organique est séchée sur $MgSO_4$, puis évaporée sous pression réduite. Le résidu est repris dans l'acétate d'éthyle, cette opération entraînant la précipitation du composé désiré. Le produit final est isolé par filtration sur verre fritté. Après concentration partielle du filtrat, le produit final précipite de nouveau. Le solide est de nouveau isolé par filtration, cette opération est répétée plusieurs fois. Le composé 34 (444 mg) est obtenu avec un rendement de 16%.

- PF 257-258°C (AcOEt)
- IR (KBr) n 1668, 1631, 1569, 1514 $cm^{-t}$
- $^1H$ RMN (250 MHz, DMSO-$d_6$): d 3.81 (s, 3H, $OCH_3$), 3.89 (s, 3H, $OCH_3$), 6.36 (d, 1 H, J = 1.8 Hz, $H_{Ar}$), 6.45 (d, 1H, J = 1.8 Hz, $H_{Ar}$), 7.28-7.42 (m, 3H, $H_{Ar}$), 7.69 (d, 2H, J = 7.0 Hz, $H_{Ar}$), 8.00 (s, 1H, $H_{Ar}$), 11.81 (s large, 1H, NH).
- $^{13}C$ RMN (62.90 MHz, DMSO-$d_6$): d 55.5, 56.0, 90.0, 93.1, 104.5, 126.9, 127.3, 127.9 (2), 128.4 (2), 131.4, 136.7, 140.8, 156.8, 161.4, 162.2.
- SM (ionspray): m/z 282 $(M+1)^+$
- *Anal.* calculé pour $C_{17}H_{15}NO_3$: C, 72.58; H, 5.37; N, 4.98. Trouvé: C, 72.29; H, 5.20; N, 5.10.

## EXEMPLE 28

### a) *N*-(2-Méthoxyphényl)-2-phénylacétamide (Composé 37)

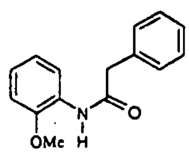

Sous atmosphère d'azote, 1,37 ml (0,01 mmol) d'o-anisidine sont dissous dans du toluène (14 ml) à 0°C. Une solution de chlorure de phénylacétyle (1,62 ml, 0,01 mmol) dans 5 ml de toluène est ajoutée goutte à goutte au milieu. Le mélange réactionnel est agité à température ambiante pendant 1 h, puis le milieu est hydrolysé par une solution froide d'hydrogénocarbonate de sodium. Le système biphasique est agité vigoureusement pendant 30 min, puis la phase organique est recueillie. La phase aqueuse est extraite par de l'acétate d'éthyle (deux fois). La phase organique est séchée sur $MgSO_4$, puis évaporée sous pression réduite. Le résidu obtenu est dissous dans un minimum de toluène, cette opération entraînant la précipitation du produit final. Après filtration sur verre fritté, 2,7 g (92%) du composé **37** sont isolés.

- PF 80-81 °C (toluène) [PF 85°C; C. Yamagami *et al Chem. Pharm. Bull.* **1984, 32,** 5003-5009]
- IR (KBr) n 3287, 1652, 1598 $cm^{-1}$
- $^1H$ RMN (250 MHz, $CDCl_3$): d 3.72 (s, 3H, $CH_3$), 3.76 (s, 2H, $CH_2$). 6,81 (dd, 1H, J = 8.0, 1.8 Hz, $H_{Ar}$), 6.91-7.05 (m, 2H, $H_{Ar}$), 7.21-7.37 (m, 4H, $H_{Ar}$), 7.80 (s large, 1H, NH), 8.35 (dd, 1 H, J = 8.0, 1.8 Hz, $H_{Ar}$).
- $^{13}C$ RMN (62.90 MHz, $CDCl_3$): d 45.2, 55.7, 109.9, 119.5, 121.1, 123.7, 127.4, 127.6, 129.0 (2), 129.6 (2), 134.6, 147.8, 168.8.
- SM (ionspray): m/z 242 $(M+1)^+$

### b) 8-Méthoxy-3-phényl-1,2-dihydro-2-quinolinone (Composé 38)

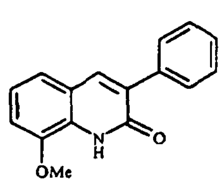

**(CRL8339)**

Sous atmosphère d'azote et à -30°C, 1,3 ml (1,68 mmol, 1,5 eq) de N,N-diméthylformamide sont additionnés goutte à goutte à 7,3 ml (78 mmol, 7 eq) de $POCl_3$. Le milieu est agité pendant 15 min à -30°C, puis 2,7 g d'amide 37 (0,01 mmol) sont additionnés. Sous agitation, le mélange réactionnel est ramené à température ambiante, puis la réaction est chauffée à 75°C pendant 1,5 h. A la fin de la réaction, cette solution est versée sur de la glace pilée, neutralisée par une solution d'ammoniaque à 30%, puis extraite par du dichlorométhane. La phase organique est

séchée sur $MgSO_4$, puis évaporée. Le résidu est purifié par chromatographie sur colonne de silice (AcOEt/EP 3:7) pour donner 650 mg (21%) de dérivé chloré. Après dissolution de ce dernier dans 3,8 ml d'acide acétique glacial et 0,12 ml d'eau, la solution finale est chauffée à reflux pendant 3 h. L'acide acétique est évaporé. Le résidu est solubilisé dans l'eau, neutralisé par une solution d'hydroxyde de sodium à 25%, puis finalement extrait avec du dichlorométhane. La phase organique est séchée sur $MgSO_4$, puis évaporée sous pression réduite. Le résidu est repris dans de l'acétate d'éthyle, cette opération entraînant la cristallisation du produit final. Les cristaux ainsi obtenus sont filtrés pour donner 237 mg (40%) du composé 38.

Le rendement global de la synthèse mise en oeuvre pour obtenir le composé 38 est de 8%.

- PF 188-189°C (AcOEt)
- IR (KBr) n 1646, 1607, 1569 $cm^{-1}$
- $^1H$ RMN (250 MHz, DMSO-$d_6$): d 3.92 (s, 3H, $OCH_3$), 7.13-7.16 (m, 2H, $H_{Ar}$), 7.30-7.46 (m, 4H, $H_{Ar}$), 7.74-7.77 (m, 2H, $H_{Ar}$), 8.09 (s, 1H, $H_{Ar}$), 10.98 (s large, 1H, NH).
- $^{13}C$ RMN (62.90 MHz, DMSO-$d_6$): d: 56.5, 111.3, 120.3, 120.4, 122.4, 128.3 (2), 128.4, 128.7, 129.2 (2), 132.6, 136.7, 138.2, 145.9, 161.1.
- SM (ionspray): 252 m/z $(M+1)^+$
- *Anal.* calculé pour $C_{16}H_{13}NO_2$: C, 76.48; H, 5.21; N, 5.57. Trouvé: C, 76.23; H, 5.14; N, 5.70.

**EXEMPLE 29 : 5,7-Acétoxy-3-(4-acétoxyphényl)-1,2-dihydro-2-quinolinone (composé 39)**

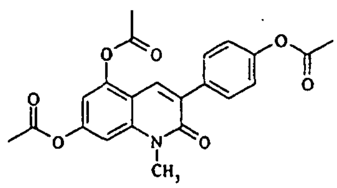

Sous atmosphère d'azote, 200 mg (0,71 mmol) de composé 9 (CRL8321) sont solubilisés dans de l'anhydride acétique et de la pyridine (8 ml, v/v) à 0°C. Le mélange réactionnel est agité à température ambiante pendant 18 h. Le milieu est hydrolysé par addition d'eau (10 ml) puis extrait par du dichlorométhane (deux fois). La phase organique est séchée sur $MgSO_4$, puis évaporée. Le résidu obtenu est purifié par chromatographie sur colonne de silice (AcOEt/$CH_2Cl_2$ 1:9) pour donner 220 mg (76%) du composé **39**.

- PF 206-207°C (AcOEt)
- IR (KBr) : n 1769, 1748, 1638, 1598, 1576, 1508 $cm^{-1}$
- $^1H$ RMN (250 MHz, $CDCl_3$): d 2.31 (s, 3H, $CH_3$), 2.33 (s, 3H, $CH_3$), 2.40 (s, 3H, $CH_3$), 3.72 (s, 3H, $NCH_3$), 6.92 (d, 1H, J = 2.0 Hz, $H_{Ar}$), 7.03 (d, 1H, J = 2.0 Hz, $H_{Ar}$), 7.14 (d, 2H, J = 8.5 Hz, $H_{Ar}$), 7.67 (d, 2H, J = 8.5 Hz, $H_{Ar}$), 7.78 (s, 1H, $H_{Ar}$).
- $^{13}C$ RMN (62.90 MHz, $CDCl_3$): d 20.8, 21.0, 21.2, 30.5, 105.2, 110.2, 111.8, 121.4 (2), 129.7, 130.3 (2), 131.5, 134.2, 141.0, 148.8, 150.7, 151.9, 161.2, 168.6, 168.8, 169.6.
- SM (ionspray): m/z 410 $(M^++1)$
- *Anal.* calculé pour $C_{22}H_{19}NO_7$: C, 64.54; H, 4.68; N, 3.42. Trouvé: C, 64.83; H, 4.85; N, 3.57.

**EXEMPLE 30: 3-[5,7-Diméthoxy-3-(4-méthoxyphényl)-2-oxo-1,2-dihydro-1-quinolinyl] butanenitrile (composé 40)**

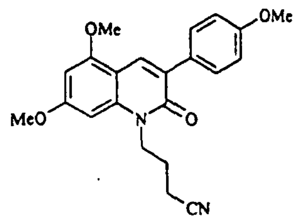

**(CRL8398)**

Sous atmosphère d'azote, 400 mg (1.28 mmol) de composé 1 (CRL8246) sont solubilisés dans 10 ml de N, N-diméthylformamide (DMF) anhydre. A 0°C, 47 mg (1.92 mmol, 1,5 eq) d'hydrure de sodium, préalablement lavé dans de l'éther de pétrole, sont additionnés par petites portions à la solution réactionnelle (réaction exothermique). Le 4-chlorobutyronitrile (0.23 ml, 2.57 mmol, 2 eq) et l'iodure de sodium (20 mg) sont additionnés au milieu. La réaction est chauffée pendant 3 h à 90°C. Après refroidissement et évaporation du DMF, de l'eau est versée sur le résidu. La solution aqueuse est extraite par de l'acétate d'éthyle (deux fois). La phase organique est séchée sur $MgSO_4$, puis évaporée sous pression réduite. Le résidu obtenu est purifié par chromatographie sur colonne de silice ($CH_2Cl_2$/AcOEt 9:1) pour donner 151 mg (31 %) de composé **40a** et 161 mg (33%) de dérivé **40.**

Composé **40:**

- PF 157-158°C (AcOEt)
- IR (KBr) n 1639, 1609, 1597, 1575, 1517 $cm^{-1}$
- $^1H$ RMN (250 MHz, $CDCl_3$): d; 2.10-2.21 (m, 2H, $CH_2$), 2.52 (t, 2H, J = 7.2 Hz, $CH_2CN$), 3.83 (s, 3H, $OCH_3$), 3.92 (s, 3H, $OCH_3$). 3.93 (s, 3H, $OCH_3$), 4.43 (t, 2H, J = 7.2 Hz, $NCH_2$), 6.31 (d, 1H, J = 2.2 Hz, $H_{Ar}$), 6.42 (d, 1H, J = 2.2 Hz, $H_{Ar}$), 6.94 (d, 2H, J = 8.8 Hz, $H_{Ar}$), 7.66 (d, 2H, J = 8.8 Hz, $H_{Ar}$), 8.15 (s, 1H, $H_{Ar}$).
- $^{13}C$ RMN (62.90 MHz, $CDCl_3$): d 15.3, 23.6, 41.8, 55.4, 55.9. 56.0, 89.6, 93.1, 106.4, 113.6 (2), 119.5, 126.7. 129.6, 130.1 (2), 130.8, 140.7, 157.9, 159.3, 161.2, 162.8.
- SM (ionspray): m/z 379 ($M^+$+1)
- *Anal.* calculé pour $C_{22}H_{22}N_2O_4$: C, 69.83; H, 5.86; N, 7.40. Trouvé: C, 69.61; H, 5.97; N. 7.32.

**2-([5,7-Diméthoxy-3-(4-méthoxyphényl)-2-quinolinyl]oxy)butanenitrite (composé 40a)**

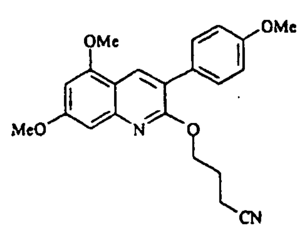

Composé **40a:**

- PF 89-90°C (AcOEt)

- IR (KBr) n 1624, 1607, 1582, 1515 $cm^{-1}$
- $^1H$ RMN (250 MHz, $CDCl_3$): d 2.12-2.22 (m, 2H, $CH_2$), 2.50 (t, 2H, J = 7.5 Hz, $CH_2$), 3.87 (s, 3H, $OCH_3$). 3.94 (s, 6H, $OCH_3$), 4.61 (t, 2H, J = 7.5 Hz, $CH_2$), 6.40 (d, 1H, J = 2.2 Hz, $H_{Ar}$), 6.81 (d, 1H, J = 2.2 Hz, $H_{Ar}$), 6.97 (d, 2H, J = 8.8 Hz, $H_{Ar}$), 7.53 (d, 2H, J = 8.8 Hz, $H_{Ar}$), 8.27 (s, 1 H, $H_{Ar}$).
- $^{13}C$ RMN (62.9 MHz, $CDCl_3$): d 14.6, 25.4, 55.4, 55.7, 55.8, 63.6, 96.2, 98.6, 113.1, 113.7 (2), 119.5, 122.0, 129.5, 130.5 (2), 132.8, 147.9, 156.4, 159.1, 159.7, 161.6.
- SM (ionspray): m/z 379 ($M^+$+1)
- *Anal.* calculé pour $C_{22}H_{22}N_2O_4$: C, 69.83; H, 5.86; N, 7.40. Trouvé: C, 69.99; H, 5.72; N, 7.60.

**EXEMPLE 31:**

**a) *N*-(3,5-Diméthoxyphényl)-2-(4-benzyloxyphényl)acétamide (composé 41)**

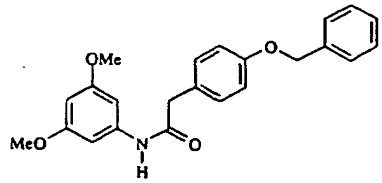

Sous atmosphère d'azote, 238 mg (1,6 mmol) de 3,5-diméthoxyaniline sont solubilisés dans du toluène (7 ml) à 0°C. Une solution de chlorure de 4-benzylphénylacétyle (0,5 ml, 1,7 mmol) dans 5 ml de toluène est ajoutée goutte à goutte au milieu. Le mélange réactionnel est agité à température ambiante pendant 2 h, puis le milieu est hydrolysé par une solution froide d'hydrogénocarbonate de sodium. Le système biphasique est agité vigoureusement pendant 30 min, puis la phase organique est recueillie. La phase aqueuse est extraite par de l'acétate d'éthyle (deux fois). La phase organique est séchée sur $MgSO_4$, puis évaporée. Le résidu obtenu est purifié par chromatographie sur colonne de silice (AcOEt/EP 4:6) pour donner 300 mg (81%) du composé **41.**

- PF 122-123°C (AcOEt/EP)
- IR (KBr) n 291, 1659, 1610, 1595, 1513 $cm^{-1}$
- $^1H$ RMN (250 MHz, $CDCl_3$): d 3.66 (s, 2H, $CH_2$), 3.74 (s, 3H, $OCH_3$), 3.75 (s, 3H, $OCH_3$), 5.08 (s, 2H, $CH_2$), 6.21 (t, 1H, J = 2.2 Hz, $H_{Ar}$), 6.65-6.66 (m, 2H, J = 2.2 Hz, $H_{Ar}$), 7.00 (d, 2H, J = 8.8 Hz, $H_{Ar}$), 7.08 (s, 1H, NH), 7.24 (d, 2H, J = 8.8 Hz, $H_{Ar}$), 7.33-7.46 (m, 5H, $H_{Ar}$).
- $^{13}C$ RMN (62.90 MHz, $CDCl_3$): d 44.2, 55.5 (2), 70.2, 96.9, 98.0 (2), 115.7 (2), 126.6, 127.6 (2), 128.2, 128.8 (2), 130.8 (2), 136.9, 139.6, 158.4, 161.1 (2), 169.7.
- SM (ionspray): 378 ($M^+$+1)
- *Anal.* calculé pour $C_{23}H_{23}NO_4$: C, 73.19; H, 6.14; N, 3.71. Trouvé: C, 72.87; H, 5.97; N, 3.85.

**b) 5,7-Diméthoxy-3-(4-benzyloxyphényl)-1,2-dihydro-2-quinolinone (composé 42)**

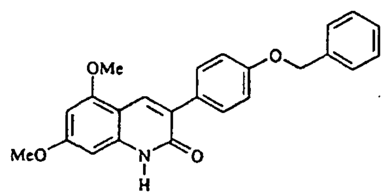

Sous atmosphère d'azote et à -30°C, 0,31 ml (4,0 mmol, 1,5 eq) de N,N diméthylformamide sont additionnés goutte à goutte à 1,75 ml (19 mmol, 7,5 eq) de $POCl_3$. Le milieu est agité pendant 15 min à -30°C, puis 1,02 g d'amide 41 (2,7 mmol) sont additionnés. Sous agitation, le mélange réactionnel est ramené à température ambiante, puis la réaction est chauffée à 75°C pendant 1,5 h. A la fin de la réaction, cette solution est versée sur de la glace pilée,

neutralisée par une solution d'ammoniaque à 30%, puis extraite par du dichlorométhane. La phase organique est séchée sur $MgSO_4$, puis évaporée. Le résidu obtenu est dissous dans 4,75 ml d'acide acétique glacial et 0,15 ml d'eau, puis la solution finale est chauffée à reflux pendant 3 h. L'acide acétique est évaporé. Le résidu est solubilisé dans l'eau, neutralisé par une solution d'hydroxyde de sodium à 25%, puis finalement extrait avec du dichlorométhane. La phase organique est séchée sur $MgSO_4$, puis évaporée sous pression réduite. Le résidu est repris dans de l'acétate d'éthyle, cette opération entraînant la précipitation du produit final. Les cristaux ainsi obtenus sont filtrés pour donner 200 mg (20%) du composé **42.**

- PF 234-235°C (AcOEt)
- IR (KBr): n 1629, 1608, 1569, 1515 $cm^{-1}$
- $^1H$ RMN (250 MHz, DMSO-$d_6$): d 3.81 (s, 3H, $OCH_3$), 3.90 (s, 3H, $OCH_3$), 5.15 (s, 2H, $CH_2$), 6.37 (s, 1H, $H_{Ar}$), 6.45 (s, 1H, $H_{Ar}$), 7.04 (d, 2H, J = 8.8 Hz, $H_{Ar}$), 7.31-7.49 (m, 5H, HAr), 7.69 (d, 2H, J = 7.5 Hz, $H_{Ar}$), 7.97 (s, 1H, $H_{Ar}$), 11.76 (s large, 1H, NH).
- $^{13}C$ RMN (62.90 MHz, DMSO-$d_6$): d 55.4, 55.9, 69.2, 90.0, 93.0, 104.6, 114.3 (2), 126.4, 127.6 (2), 127.8, 128.4 (2) 129.2, 129.6 (2), 130.2, 137.1, 140.5, 156.6, 157.7, 161.5, 161.9.
- SM (ionspray): m/z 388 ($M^+$+1)
- *Anal.* calculé pour $C_{24}H_{21}NO_4$: C, 74.40; H, 5.46; N, 3.62. Trouvé: C, 74.26; H, 5.67; N, 3.52.

**EXEMPLE 32:**

**a) *N*-(4-Méthoxyphényl)-2-phénylacétamide (composé 43)**

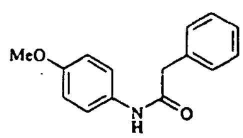

Sous atmosphère d'azote, 1,5 g (12,0 mmol) de p-anisidine sont solubilisés dans du toluène (7 ml) à 0°C. Une solution de chlorure de phénylacétyle (1,61 ml, 12,2 mmol) dans 20 ml de toluène est ajoutée goutte à goutte au milieu. Le mélange réactionnel est agité à température ambiante pendant 2 h, puis le milieu est hydrolysé par une solution froide d'hydrogénocarbonate de sodium. Le système biphasique est agité vigoureusement pendant 30 min, puis la phase organique est recueillie. La phase aqueuse est extraite par de l'acétate d'éthyle (deux fois). La phase organique est séchée sur $MgSO_4$, puis évaporée. Le résidu obtenu est purifié par chromatographie sur colonne de silice (AcOEt/EP 3:7) pour donner 2,6 g (89%) du composé 43.

- PF 118-119°C (AcOEt/EP)
- IR (KBr): n 3290, 1650, 1603, 1545, 1513 $cm^{-1}$
- $^1H$ RMN (250 MHz, $CDCl_3$): d 3.72 (s, 2H, $CH_2$), 3.77 (s, 3H, $OCH_3$), 6.81 (d, 2H, J = 9.0 Hz, $H_{Ar}$), 7.00 (large s, 1 H, NH), 7.28-7.43 (m, 7H, $H_{Ar}$).
- $^{13}C$ RMN (62.90 MHz, $CDCl_3$): dδ. 44.8, 55.6, 114.2 (2), 121.9 (2), 127.7, 129.3 (2), 129.7 (2), 130.8, 134.7, 156.7, 169.1.
- SM (ionspray): 242 ($M^+$+1)
- *Anal.* calculé pour $C_{15}H_{15}NO_2$: C, 74.67; H, 6.27; N, 5.80. Trouvé: C, 74.79; H, 6.14; N, 5.95.

**b) 6 Méthoxy-3-phényl-1,2-dihydro-2-quinolinone (composé 44)**

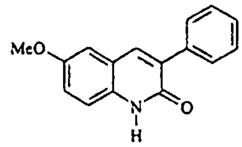

Sous atmosphère d'azote et à -30°C, 0,96 ml (12,4 mmol, 1,5 eq) de *N,N*-diméthylformamide sont additionnés goutte à goutte à 5,4 ml (58 mmol, 7,5 eq) de $POCl_3$. Le milieu est agité pendant 15 min à -30°C, puis 2,0 g d'amide 43 (8,3 mmol) sont additionnés. Sous agitation, le mélange réactionnel est ramené à température ambiante, puis la réaction est chauffée à 75°C pendant 1,5 h. A la fin de la réaction, cette solution est versée sur de la glace pilée, neutralisée par une solution d'ammoniaque à 30%, puis extraite par du dichlorométhane. La phase organique est séchée sur $MgSO_4$, puis évaporée. Le résidu obtenu est dissous dans 12,2 ml d'acide acétique glacial et 0,4 ml d'eau, puis la solution finale est chauffée à reflux pendant 3 h. L'acide acétique est évaporé. Le résidu est solubilisé dans l'eau, neutralisé par une solution d'hydroxyde de sodium à 25%, puis finalement extrait avec du dichlorométhane. La phase organique est séchée sur $MgSO_4$, puis évaporée sous pression réduite. Le résidu est repris dans de l'acétate d'éthyle, cette opération entraînant la précipitation du produit final. Les cristaux ainsi obtenus sont filtrés pour donner 585 mg (28%) du composé 44.

- PF 243-244°C (AcOEt)
- IR (KBr) n 1645, 1618, 1569, 1503 $cm^{-1}$
- $^1H$ RMN (250 MHz, DMSO-$d_6$): d 3.80 (s, 3H, $OCH_3$), 7.16 (dd, 1H, J = 2.5, 8.9 Hz, $H_{Ar}$), 7.28 (d, 1H, J = 8.9 Hz, $H_{Ar}$), 7.29 (d, 1H, J = 2.5 Hz, $H_{Ar}$), 7.34-7.47 (m, 3H, $H_{Ar}$), 7.76 (d, 2H, J = 6.8 Hz, $H_{Ar}$), 8.06 (s, 1H, $H_{Ar}$), 11.85 (s large, 1H, NH).
- $^{13}C$ RMN (62.90 MHz, DMSO-$d_6$): d 55.4, 109.4, 116.0, 119.5, 120.1, 127.8, 127.9 (2), 128.7 (2), 131.9, 132.9, 136.4, 137.2, 154.2, 160.6.
- SM (ionspray): m/z 252 ($M^+$+1)
- *Anal.* calculé pour $C_{16}H_{13}NO_2$: C, 76.48; H, 5.21; N, 5.57. Trouvé: C, 76.16; H, 5.11; N, 5.66.

**<u>EXEMPLE 33 :</u> 5,7-Diméthoxy-3-(4-trifluorométhanesufonylphényl)-1,2-dihydro-2-quinolinone (composé 45)**

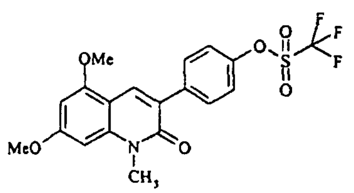

Sous atmosphère d'azote, 170 mg (0,55 mmol) de composé 8 sont solubilisés dans de l'anhydride triflique et de la pyridine (8 ml, v/v) à 0°C. Le mélange réactionnel est agité à température ambiante pendant 2 h. Le milieu est hydrolysé par addition d'eau (10 ml) puis extrait par du dichlorométhane (deux fois). La phase organique est séchée sur $MgSO_4$, puis évaporée. Le résidu obtenu est purifié par chromatographie sur colonne de silice (AcOEt/$CH_2Cl_2$ 1: 9) pour donner 194 mg (80%) du composé 45.

- PF 144-145°C (AcOEt)
- IR (KBr): n 1646, 1618, 1602, 1504 $cm^{-1}$
- $^1H$ RMN (250 MHz, $CDCl_3$): d 3.75 (s, 3H, $NCH_3$), 3.94 (s, 6H, $CH_3$), 6.32 (d, 1H, J = 2.0 Hz, $H_{Ar}$), 6.39 (d, 1H, J = 2.0 Hz, $H_{Ar}$), 7.30 (d, 2H, J = 8.8 Hz, $H_{Ar}$), 7.82 (d, 2H, J = 8.8 Hz, $H_{Ar}$), 8.19 (s, 1 H, $H_{Ar}$).
- $^{13}C$ RMN (62.90 MHz, $CDCl_3$): d 30.5, 55.8, 56.0, 90.4, 93.0, 106.0, 121.0 (2), 125.3, 130.9 (3), 132.1. 138.1, 148.9, 158.0, 161.8, 163.2.

- SM (ionspray): m/z 444 ($M^{+}$+1)

**EXEMPLE 34 : 5,7-Diméthoxy-3-(4-acétylphényl)-1,2-dihydro-2-quinolinone (composé 46)**

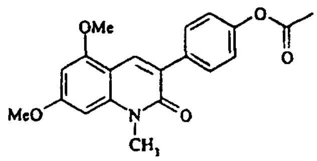

Sous atmosphère d'azote, 200 mg (0,64 mmol) de composé **8** sont solubilisés dans de l'anhydride acétique et de la pyridine (8 ml, v/v) à 0°C. Le mélange réactionnel est agité à température ambiante pendant 18 h. Le milieu est hydrolysé par addition d'eau (10 ml) puis extrait par du dichlorométhane (deux fois). La phase organique est séchée sur $MgSO_4$, puis évaporée. Le résidu obtenu est purifié par chromatographie sur colonne de silice ($AcOEt/CH_2Cl_2$ 1: 9) pour donner 165 mg (73%) du composé **46**.

- PF 148-149°C (AcOEt/EP)
- IR (KBr): n 1751, 1639, 1601, 1508 $cm^{-1}$
- $^1H$ RMN (250 MHz, $CDCl_3$): d 2.31 (s, 3H, $CH_3$), 3.72 (s, 3H, $NCH_3$), 3.90 (s, 6H, $CH_3$), 6.28 (d, 1H, J = 2.0 Hz, $H_{Ar}$), 6.34 (d, 1H, J = 2.0 Hz, $H_{Ar}$), 7.13 (d, 2H, J = 8.8 Hz, $H_{Ar}$), 7.75 (d, 2H, J = 8.8 Hz, $H_{Ar}$), 8.15 (s, 1H, $H_{Ar}$).
- $^{13}C$ RMN (62.90 MHz, $CDCl_3$): d 21.3, 30.4, 55.7, 55.9, 90.3, 92.7, 106.0, 121.1 (2), 126.3, 130.1 (3), 131.4, 142.1, 150.1, 157.8, 162.0, 162.7, 169.6.
- SM (ionspray): m/z 354 ($M^{+}$+1)
- *Anal.* calculé pour $C_{20}H_{19}NO_5$: C, 67.98; H, 5.42; N, 3.96. Trouvé: C, 68.23; H, 5.56; N, 3.79.

**EXEMPLE 35:**

**a) *N*-(4-Méthylphényl)-2-phénytacétamide (47)**

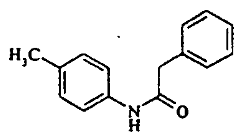

Sous atmosphère d'azote, 1,0 g (9,3 mmol) de 4-méthylaniline sont solubilisés dans du toluène (10 ml) à 0°C. Une solution de chlorure de phénylacétyle (1,25 ml, 9,4 mmol) dans 20 ml de toluène est ajoutée goutte à goutte au milieu. Le mélange réactionnel est agité à température ambiante pendant 2 h, puis le milieu est hydrolysé par une solution froide d'hydrogénocarbonate de sodium. Le système biphasique est agité vigoureusement pendant 30 min, puis la phase organique est recueillie. La phase aqueuse est extraite par de l'acétate d'éthyle (deux fois). La phase organique est séchée sur $MgSO_4$, puis évaporée. Le résidu obtenu est purifié par chromatographie sur colonne de silice (AcOEt/EP 3:7) pour donner 1,9 g (92%) du composé **47**.

- PF 119-120°C (AcOEt)
- IR (KBr) n 3310, 1657, 1604, 1536, 1514 $cm^{-1}$
- $^1H$ RMN (250 MHz, $CDCl_3$): d 2.28 (s, 3H, $CH_3$), 3.70 (s, 2H, $CH_2$), 7.06 (d, 1H, J = 8.5 Hz, $H_{Ar}$), 7.25-7.38 (m, 9H, $H_{Ar}$).
- $^{13}C$ RMN (62.90 MHz, $CDCl_3$): d 21.0, 44.9, 120.1 (2), 127.7, 129.3 (2), 129.5 (2), 129.6 (2), 134.2, 134.7, 135.2, 169.2.
- SM (ionspray): 226 ($M^{+}$+1)
- *Anal.* calculé pour $C_{15}H_{15}NO$: C, 79.97; H, 6.71; N, 6.22. Trouvé: C, 80.23; H, 6.87; N, 6.11.

**b) 6-Méthyl-3-phényl-1,2-dihydro-2-quinolinone (48)**

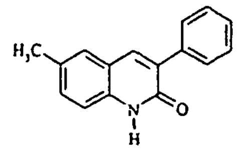

Sous atmosphère d'azote et à -30°C, 0,41 ml (5,3 mmol, 1,5 eq) de N,N-diméthylformamide sont additionnés goutte à goutte à 3,3 ml (25 mmol, 7 eq) de $POCl_3$. Le milieu est agité pendant 15 min à -30°C, puis 800 mg d'amide **47** (3,5 mmol) sont additionnés. Sous agitation, le mélange réactionnel est ramené à température ambiante, puis la réaction est chauffée à 75°C pendant 1,5 h. A la fin de la réaction, cette solution est versée sur de la glace pilée, neutralisée par une solution d'ammoniaque à 30%, puis extraite par du dichlorométhane. La phase organique est séchée sur $MgSO_4$, puis évaporée. Le résidu obtenu est dissous dans 5,4 ml d'acide acétique glacial et 0,2 ml d'eau, puis la solution finale est chauffée à reflux pendant 3 h. L'acide acétique est évaporé. Le résidu est solubilisé dans l'eau, neutralisé par une solution d'hydroxyde de sodium à 25%, puis finalement extrait avec du dichlorométhane. La phase organique est séchée sur $MgSO_4$, puis évaporée sous pression réduite. Le résidu est repris dans de l'acétate d'éthyle, cette opération entraînant la précipitation du produit final. Les cristaux ainsi obtenus sont filtrés pour donner 80 mg (10%) du composé **48**.

- PF 212-213°C (AcOEt)
- IR (KBr) :n 1657, 1570 $cm^{-1}$
- $^1H$ RMN (250 MHz, DMSO-$d_6$): d 2.35 (s, 3H, $CH_3$), 7.25 (d, 1H, J = 8.5 Hz, $H_{Ar}$), 7.33-7.48 (m, 5H, $H_{Ar}$), 7.51 (s large, 1H, $H_{Ar}$), 7.76 (d, 2H, J = 8.9 Hz, $H_{Ar}$), 8.02 (s, 1 H, $H_{Ar}$), 11.87 (s large, 1H, NH).
- $^{13}C$ RMN (62.90 MHz, DMSO-$d_6$): d 20.5, 114.6, 119.5, 127.6, 127.8, 127.9 (2), 128.7 (2), 130.8, 131.5 (2), 136.4 (2), 137.4, 160.9.
- SM (ionspray): m/z 236 ($M^+$+1)
- *Anal.* calculé pour $C_{16}H_{13}NO$: C, 81.68; H, 5.57; N, 5.95. Trouvé: C, 81.78; H, 5.39; N, 6.11.

On donnera ci-après des résultats d'essais pharmacologiques mettant en évidence les propriétés des composés de formule I et I a soit seuls, soit en association avec des agents cytotoxiques.

**1 - Interaction (stimulation ou inhibition de la prolifération) avec la génération de cellules clonogènes (test clonogénique)**

Le test utilisé est celui décrit par Hamburger et al. (Science, 1977;197, 461-463) et Salmon et al. (New England J. Med., 298, 1321-1327). Une cellule est considérée clonogénique si elle possède la capacité de proliférer et de donner naissance à une colonie cellulaire. Les « human tumor stem cells » ou « cellules souches tumorales humaines » sont les cellules qui sont à l'origine des cellules néoplasiques qui constituent une tumeur donnée. Ces cellules souches tumorales sont responsables des processus de récidives observables après résection chirurgicale des tumeurs primaires et sont également responsables de la formation des métastases. Au niveau d'une tumeur ou d'une lignée cellulaire tumorale, ces cellules souches clonogéniques se différencient des autres cellules de la tumeur ou de la lignée cellulaire néoplasique considérée, par le fait qu'elles conservent leur capacité à proliférer en l'absence de tout support solide.

Dans ce test, les cellules tumorales sont mises en culture sur un support semi-solide constitué par de l'agar. Seules les cellules ne nécessitant pas de support solide pour leur croissance (c'est-à-dire les cellules très tumorigéniques appelées "anchorage-independent cells" par M.I. Dawson et al., Cancer Res. 1995 ; 55 : 4446-4451 ; également dénommées cellules clonogènes en référence à "clonal growth") sont capables de se développer sur un tel support à base d'agar. En effet, sur un tel milieu, les cellules normales -qui sont à croissance en "mode adhérent" ("anchorage-dependent cells" selon la terminologie de M.I. Dawson)- comme par exemple les fibroblastes, ne survivent pas. Au sein d'une population cellulaire tumorale, cultivée sur un tel support, ce sont ces cellules clonogènes (associées à un nombre illimité de divisions cellulaires et dont la prolifération est appelée par M.I. Dawson "anchorage-independent [clonal] growth") qui sont capables de croître. Le pourcentage de ces cellules clonogènes au sein d'une tumeur ou d'une lignée cellulaire varie entre 0,1% et 0,001%. Les cellules non-clonogènes (associées à un nombre limité de divisions cellulaires) ne se développent pas dans ce test car elles nécessitent un support solide pour leur croissance

qui doit se faire en "mode adhérent" ("anchorage-dependent [adherent] growth", selon M.I. Dawson et al., Cancer Res. 1995 ; 55 : 4446-51)."

L'influence de composés de formule (I) et (Ia) sur la croissance des colonies cellulaires obtenues en cultivant, par exemple, les lignées tumorales mammaires MCF7 et MXT et la lignée colorectale HT-29 sur le milieu de culture semi-liquide appelé "soft agar" a été mesurée. Sur un tel milieu, seules les cellules clonogènes appelées par M.I. Dawson "anchorage-independent (clonal) cells" survivent et se développent. La croissance de ces cellules en un tel mode "non adhérent" témoigne de leur degré de tumorigénicité. L'inhibition de la croissance de la taille d'une tumeur dans laquelle s'est développé un plus grand nombre de cellules clonogènes devient alors le témoin d'une activité cytotoxique renforcée.

A l'inverse, ce test peut aussi révéler qu'un composé est capable d'inhiber la génération/prolifération de cellules clonogènes, ce qui rend la tumeur moins apte à se développer, donc diminue la population de cellules tumorales.

Les lignées cellulaires tumorales étudiées sont maintenues en culture dans des boîtes falcon de 25 $cm^2$. Elles sont ensuite trypsinisées et les cellules bien dissociées les unes des autres. Le pourcentage de cellules vivantes est déterminé après coloration au bleu trypan. Une suspension cellulaire à la concentration de $5.10^4$ à $15.10^4$ cellules/ml (selon le type cellulaire considéré) est préparée dans une solution d'agar à 0,3%. Ensuite, 200 µl de cette suspension sont ensemencés dans des boîtes de pétri de 35 mm de diamètre, dans lequelles sont déposés 3 ml d'une couche de base constituée d'une solution d'agar à 0,5%. Les 200 µl de suspension cellulaire sont à leur tour recouverts par 1,8 ml d'une couche supérieure constituée d'une solution d'agar à 0,3%. Les boîtes sont ensuite placées dans un incubateur à 37° C, 5% $CO_2$ et 70% d'humidité jusqu'au traitement. Ce dernier est effectué environ 1 à 2 heures après l'ensemencement. Les composés à tester sont préparés à une concentration 100 fois supérieure à la concentration souhaitée et 50 µl de ces solutions traitantes sont déposés sur la couche supérieure d'agar des boîtes correspondantes. Dans la présente étude, la concentration finale des produits testés est $10^{-5}$, $10^{-7}$ et $10^{-9}$ M. Les boîtes sont ensuite maintenues 21 jours dans l'incubateur. Au 21è jour les boîtes sont traitées en déposant sur la couche supérieure 100 µl d'une solution de MTT (bromure de 3-(4,5-dimethylthiazol-2-yl)-2,5-diphényltétrazolinium à 1 mg/ml préparé avec du milieu RPMI 1640 pendant 3 h à 37°C. Après ce laps de temps, les colonies cellulaires sont fixées en ajoutant 2 ml de formol par boîte. Après 24 heures de fixation, le formol est évaporé et le nombre de colonies cellulaires colorées, donc constituées de cellules métaboliquement actives, et dont la surface est supérieure à 100 $\mu m^2$ est déterminé, à l'aide d'un microscope inversé.

Le nombre moyen de clones de cellules clonogénes déterminé pour chaque condition expérimentale étudiée est exprimée en pourcentage par rapport au nombre moyen de clones de cellules clonogènes comptabilisé dans la condition contrôle posée égal à 100%. Ces valeurs, exprimées en pourcentage par rapport à la condition contrôle pour l'ensemble des produits testés, sont consignées dans le Tableau I.

## TABLEAU I

## TESTS CLONOGENIQUES

| LIGNEES | CRL8315 | | | CRL8316 | | | CRL8246 | | |
|---|---|---|---|---|---|---|---|---|---|
| CELLULAIRES | $10^{-5}$ | $10^{-7}$ | $10^{-9}$ | $10^{-5}$ | $10^{-7}$ | $10^{-9}$ | $10^{-5}$ | $10^{-7}$ | $10^{-9}$ |
| MCF7 | 119,6 ± 5,6<br>* | 127 ± 8,8<br>* | 157,1 ± 12,2<br>** | 23,2 ± 1,8<br>*** | 84 ± 5<br>* | 83,4 ± 4,6<br>* | 126,8 ± 9,9<br>* | 145,7 ± 8,9<br>** | 139,1 ± 6,6<br>** |
| HT-29 | 103,5 ± 4,5<br>NS | 111,9 ± 5,4<br>NS | 112,9 ± 2,4<br>* | 50,6 ± 1,8<br>*** | 80,1 ± 2,9<br>** | 101,6 ± 3,2<br>NS | 70,9 ± 2,8<br>** | 103,3 ± 3,6<br>NS | 104 ± 2,7<br>NS |
| MXT | 76 ± 2,3<br>** | 103,9 ± 4,3<br>NS | 102,4 ± 3,9<br>NS | 10,8 ± 0,5<br>*** | 89,1 ± 3,7<br>NS | 95,3 ± 3,8<br>NS | 98,7 ± 7,2<br>NS | 97,7 ± 9,3<br>NS | 95,2 ± 6,8<br>NS |

| LIGNEES | CRL8256 | | | CRL8247 | | | CRL8283 | | |
|---|---|---|---|---|---|---|---|---|---|
| CELLULAIRES | $10^{-5}$ | $10^{-7}$ | $10^{-9}$ | $10^{-5}$ | $10^{-7}$ | $10^{-9}$ | $10^{-5}$ | $10^{-7}$ | $10^{-9}$ |
| MCF7 | 51,6 ± 3,7<br>*** | 83,8 ± 3,4<br>* | 97,9 ± 5,6<br>NS | 51,5 ± 2,8<br>*** | 81,9 ± 1,2<br>** | 98,3 ± 4,2<br>NS | 56,5 ± 4,9<br>*** | 106,2 ± 4,8<br>NS | 97,4 ± 5,8<br>NS |
| HT-29 | 97,1 ± 4,3<br>NS | 100,5 ± 4,1<br>NS | 107,5 ± 3,7<br>NS | 72,7 ± 3,6<br>** | 98,3 ± 4,9<br>NS | 104,5 ± 2,7<br>NS | 88,9 ± 0,2<br>** | 90,9 ± 3,1<br>NS | 106,1 ± 1,4<br>NS |
| MXT | 53 ± 1,9<br>*** | 103,8 ± 3,9<br>NS | 104,5 ± 4,7<br>NS | 65,7 ± 1,7<br>*** | 89,6 ± 4,9<br>NS | 98,4 ± 2,6<br>NS | 23,7 ± 1,4<br>*** | 81,2 ± 3<br>** | 91,4 ± 4<br>NS |

- Concentrations exprimées en mole.$l^{-1}$
- Les résultats récapitulés dans ce tableau représentent les valeurs moyennes ± l'erreur standard sur la moyenne (ESM) établies sur au moins 6 cupules.
- Condition contrôle = 100%.
- (NS : $p > 0{,}05$; * : $p < 0{,}05$; ** : $p < 0{,}01$; *** : $p < 0{,}001$).

Ainsi, les composés de formules (I) et (Ia), parce qu'ils agissent sur le comportement clonogénique de la tumeur :

- soit augmentent (ex : CRL 8315) - par rapport à la situation de référence [milieux de culture non additionnés des composés de formule (I) ou (Ia)] - le nombre moyen de clones de cellules clonogènes rendant, de ce fait, un plus grand nombre de cellules de la tumeur sensibles à l'agent cytotoxique (car les cellules clonogènes sont plus sensibles aux agents cytotoxiques pendant leur phase de prolifération),
- soit diminuent (ex : CRL 8283) le nombre de cellules clonogènes par toxicité directe (d'où, là aussi, régression de la tumeur).

**2 - Activité cytotoxique au niveau des cellules non-clonogènes : "test MTT"**

L'influence des composés de formule (I) et (Ia) sur les cellules non-clonogènes a été évaluée à l'aide du test colorimétrique MTT.

Le principe du test MTT est basé sur la réduction mitochondriale par les cellules vivantes métaboliquement actives du produit MTT (bromure de 3-(4,5-diméthylthiazol-2-yl)-2,5 diphényltétrazolium) de couleur jaune en un produit de couleur bleue, le formazan. La quantité de formazan ainsi obtenue est directement proportionnelle à la quantité de cellules vivantes présentes dans le ou les puits de culture. Cette quantité de formazan est mesurée par spectrophotométrie.

Les lignées cellulaires sont maintenues en culture monocouche à 37° C dans des boîtes de culture à bouchon fermé contenant du milieu de base MEM 25 MM HEPES (Minimum Essential Medium). Ce milieu est bien adapté à la

croissance d'une gamme de cellules variées diploïdes ou primaires de mammifères. Ce milieu est ensuite additionnée :

- d'une quantité de 5% de SVF (Sérum de Veau Foetal) décomplémenté à 56° C pendant 1 heure,
- de 0,6 mg/ml de L-glutamine,
- de 200 IU/ml de pénicilline,
- de 200 µg/ml de streptomycine,
- de 0,1 mg/ml de gentamicine.

Les 12 lignées cellulaires cancéreuses humaines qui ont été utilisées ont été obtenues auprès de *l'American Type Culture Collection* (ATCC, Rockville, MD, USA). Ces 12 lignées cellulaires sont :

- U-373MG (code ATCC : HTB-17) et U-87MG (code ATCC : HTB-14) qui sont deux glioblastomes,
- SW1088 (code ATCC : HTB-12) qui est un astrocytome,
- A549 (code ATCC : CCL-185) et A-427 (code ATCC : HTB-53) qui sont deux cancers du poumon non-à-petites-cellules,
- HCT-15 (code ATCC : CCL-225) et LoVo (code ATCC : CCL-229) qui sont deux cancers colorectaux,
- T-47D (code ATCC : HTB-133) et MCF7 (code ATCC : HTB-22) qui sont deux cancers du sein,
- J82 (code ATCC : HTB-1) et T24 (code ATCC : HTB-4) qui sont deux cancers de la vessie,
- PC-3 (code ATCC : CRL-1435) qui est un cancer de la prostate.

Au plan expérimental : 100 µl d'une suspension cellulaire contenant 20 000 à 50 000 (selon le type cellulaire utilisé) cellules/ml de milieu de culture sont ensemencés en plaques multi-puits de 96 puits à fond plat et sont mis à incuber à 37°C, sous atmosphère comprenant 5% $CO_2$ et 70% d'humidité. Au bout de 24 heures d'incubation, le milieu de culture est remplacé par 100 µl de milieu frais contenant soit les différents composés à tester à des concentrations variant de $10^{-5}$ à $10^{-10}$ M soit le solvant ayant servi à la mise en solution des produits à tester (condition contrôle). Après 72 heures d'incubation dans les conditions précédentes, le milieu de culture est remplacé par 100 µl d'une solution jaunâtre de MTT dissous à raison de 1 mg/ml dans du RPMI 1640. Les microplaques sont remises à incuber pendant 3 heures à 37°C puis centrifugées pendant 10 minutes à 400 g. La solution jaunâtre de MTT est éliminée et les cristaux de formazan bleu formés au niveau cellulaire sont dissous dans 100 µl de DMSO. Les microplaques sont ensuite mises sous agitation pendant 5 minutes. L'intensité de la coloration bleue résultant donc de la transformation du produit MTT jaune en formazan bleu par les cellules encore vivantes au terme de l'expérience est quantifiée par spectrophotométrie à l'aide d'un appareil de type *DYNATECH IMMUNOASSAY SYSTEM* aux longueurs d'onde de 570 nm et 630 nm correspondant respectivement aux longueurs d'ondes d'absorbance maximale du formazan et au bruit de fond. Un logiciel intégré au spectrophotomètre calcule les valeurs moyennes de densité optique ainsi que les valeurs de déviation standard (Dév. Std.) et d'erreur standard sur la moyenne (ESM).

A titre d'exemple, on donnera dans le tableau II les résultats de la densité optique moyenne, exprimés en pourcentage par rapport à la densité optique moyenne mesurée dans la condition contrôle (posée égale à 100%), obtenus à la concentration de $10^{-5}$ M sur les 12 lignées cellulaires tumorales précitées.

## TABLEAU II a

| 2-QUINOLONES | LIGNEES CELLULAIRES | | | | | | | | | | | |
|---|---|---|---|---|---|---|---|---|---|---|---|---|
| | U-87MG | U-373MG | SW1088 | T24 | J82 | HCT-15 | LoVo | MCF7 | T-47D | A549 | A-427 | PC-3 |
| CRL8246 | 92,1 ± 1,5<br>* | 96,6 ± 1,2<br>NS | 107,6 ± 1,1<br>** | 109,4 ± 3,5<br>NS | 87,6 ± 2,2<br>** | 97,2 ± 5,1<br>NS | 108,8 ± 7<br>NS | 98,1 ± 1,4<br>NS | 96 ± 2,6<br>NS | 113 ± 2,1<br>*** | 101 ± 0,9<br>NS | 121,7 ± 1,7<br>*** |
| CRL8284 | 88,1 ± 2,2<br>* | 87,7 ± 1,4<br>*** | 78,3 ± 1,6<br>*** | 68,5 ± 0,9<br>*** | 48,8 ± 0,7<br>*** | 77,3 ± 1,8<br>*** | 101,7 ± 1,3<br>NS | 66,6 ± 2,7<br>*** | 89,9 ± 1,9<br>* | 91,7 ± 1,6<br>** | 96 ± 2,0<br>NS | 83,8 ± 1<br>*** |
| CRL8311 | 91,8 ± 1,3<br>NS | 113,3 ± 2,5<br>** | 80,7 ± 1,7<br>*** | 90 ± 1,9<br>** | 127 ± 1,9<br>*** | 101,2 ± 3,6<br>NS | 77,9 ± 1,7<br>*** | 98,8 ± 2,3<br>NS | 107,6 ± 6,6<br>NS | 106,1 ± 2,4<br>NS | 89,7 ± 2,0<br>** | 122,1 ± 3,5<br>** |
| CRL8271 | 78,5 ± 1,7<br>*** | 96,2 ± 1,6<br>NS | 102,2 ± 0,5<br>NS | 107,4 ± 2,3<br>* | 75,9 ± 1,3<br>*** | 87,2 ± 2,8<br>** | 94,2 ± 2,8<br>NS | 105,5 ± 2,3<br>NS | 90,9 ± 1,7<br>** | 94,2 ± 4,7<br>NS | 84,1 ± 1,9<br>** | 107 ± 2,7<br>NS |
| CRL8244 | 97 ± 1<br>NS | 69 ± 0,9<br>*** | 81,3 ± 1,1<br>*** | 88,7 ± 2,9<br>* | 88,5 ± 2,1<br>** | 76,8 ± 3,0<br>*** | 78,2 ± 1,8<br>*** | 75,9 ± 3,8<br>*** | 105 ± 3<br>* | 98 ± 0,9<br>NS | 93,6 ± 2,3<br>NS | 83,2 ± 3,6<br>** |
| CRL8321 | 96,5 ± 1,2<br>NS | 97,1 ± 2,4<br>NS | 97,2 ± 1,8<br>NS | 103,1 ± 1,7<br>NS | 88,6 ± 0,9<br>*** | 118,3 ± 1,6<br>*** | 107,8 ± 1,1<br>** | 96,6 ± 0,9<br>* | 91,5 ± 1,2<br>** | 99,4 ± 1,3<br>NS | 107 ± 1,4<br>** | 102,2 ± 3,1<br>NS |
| CRL8245 | 58,6 ± 1,7<br>*** | 63,7 ± 2,7<br>*** | 75,1 ± 2<br>*** | 51,1 ± 1,9<br>*** | 31,1 ± 0,6<br>*** | 35,8 ± 1,2<br>*** | 65,5 ± 1,1<br>*** | 46,9 ± 1<br>*** | 59,6 ± 2,9<br>*** | 74,1 ± 2,1<br>*** | 69 ± 1,9<br>*** | 74,4 ± 1,9<br>*** |
| CRL8314 | 74,5 ± 3,4<br>*** | 89,2 ± 2<br>** | 85,4 ± 2,5<br>*** | 61,9 ± 2,5<br>*** | 33,2 ± 1,2<br>*** | 116,5 ± 4,4<br>* | 82,9 ± 2,5<br>** | 72,2 ± 2<br>*** | 113,5 ± 2,3<br>* | 85,2 ± 1,4<br>*** | 104,4 ± 3,1<br>NS | 88,6 ± 1,4<br>*** |
| CRL8318 | 78,1 ± 2,4<br>*** | 89,9 ± 1,3<br>** | 75,2 ± 3<br>*** | 81,8 ± 1,4<br>*** | 72,8 ± 1,2<br>*** | 113 ± 1,7<br>*** | 75,2 ± 2,3<br>*** | 105 ± 2,4<br>NS | 100,3 ± 3,9<br>NS | 86,6 ± 2,5<br>** | 74 ± 3<br>*** | 79,2 ± 2,8<br>*** |
| CRL8317 | 91 ± 4,1<br>NS | 91,2 ± 1,9<br>** | 103,4 ± 2,3<br>NS | 91,4 ± 4,3<br>NS | 83,6 ± 1,8<br>*** | 103,6 ± 2,6<br>NS | 86,8 ± 2,8<br>** | 96 ± 2,1<br>NS | 95 ± 2,5<br>NS | 94,7 ± 2,2<br>NS | 91,3 ± 2<br>** | 97,9 ± 1,1<br>NS |
| CRL8319 | 115 ± 2,9<br>* | 101,7 ± 1,5<br>NS | 89,8 ± 2,7<br>NS | 89,6 ± 2,1<br>** | 80,9 ± 1<br>*** | 96,7 ± 1,6<br>NS | 79,7 ± 2,7<br>** | 101,5 ± 2<br>NS | 104,5 ± 2,5<br>NS | 97,3 ± 1,2<br>NS | 79,1 ± 2,5<br>*** | 90,7 ± 3,6<br>NS |
| CRL8283 | 69,9 ± 3,4<br>*** | 93,4 ± 1,5<br>NS | 84,7 ± 3,1<br>** | 72,9 ± 0,9<br>*** | 71,6 ± 2,2<br>*** | 58,2 ± 4,3<br>*** | 97,3 ± 3,6<br>NS | 76,2 ± 0,9<br>*** | 81 ± 3,1<br>*** | 63,6 ± 4,2<br>*** | 73,1 ± 1,7<br>*** | 92,4 ± 1,7<br>* |

x ± y = valeur moyenne ± erreur standard sur la moyenne    Condition contrôle = 100 %    (NS : $p > 0,05$; * : $p < 0,05$; ** : $p < 0,01$; *** : $p < 0,001$)

# TABLEAU II b

| 2-QUINOLONES | LIGNEES CELLULAIRES | | | | | | | | | | | |
|---|---|---|---|---|---|---|---|---|---|---|---|---|
| | U-87MG | U-373MG | SW1088 | T24 | J82 | HCT-15 | LoVo | MCF7 | T-47D | A549 | A-427 | PC-3 |
| CRL8315 | 87,6 ± 4,5 * | 68,9 ± 2,2 *** | 89,1 ± 0,6 *** | 89,6 ± 2,1 ** | 65,9 ± 1,3 *** | 87,9 ± 3,3 * | 78,9 ± 2,1 *** | 96,2 ± 1,7 NS | 101,3 ± 2,5 NS | 76,1 ± 2,4 *** | 92,6 ± 2,1 * | 93,6 ± 3 NS |
| CRL8254 | 105,1 ± 3,6 NS | 7,6 ± 1,3 *** | 128,3 ± 2,2 *** | 102,6 ± 2,9 NS | 87,8 ± 1 *** | 94,4 ± 3,5 NS | 91,8 ± 2,7 * | 81,9 ± 0,6 *** | 55,2 ± 4,1 *** | 96,9 ± 1,5 NS | 20,2 ± 2,3 *** | 97,9 ± 1,2 NS |
| CRL8255 | 72,5 ± 1,1 *** | 68,5 ± 2,7 *** | 67,5 ± 1,5 *** | 79,5 ± 2,2 *** | 36,2 ± 0,5 *** | 58,7 ± 3 *** | 56,9 ± 1,1 *** | 71,5 ± 1,4 *** | 73,7 ± 3,3 *** | 79,1 ± 2,1 *** | 76,3 ± 1,5 ** | 77,7 ± 1,2 *** |
| CRL8247 | 59 ± 2,3 *** | 68,7 ± 2,8 *** | 85,8 ± 3,6 ** | 77,8 ± 2,7 *** | 55 ± 2,3 *** | 87,4 ± 2,5 * | 66,4 ± 2,5 *** | 78,9 ± 1,1 *** | 58,3 ± 1,4 *** | 81,3 ± 1,9 *** | 57 ± 1,5 *** | 70,5 ± 4,6 *** |
| CRL8256 | 84,7 ± 1,7 *** | 76,1 ± 2,6 *** | 72,1 ± 3,3 *** | 78,2 ± 2,6 *** | 77,8 ± 0,8 *** | 73,9 ± 3,6 *** | 93 ± 2,3 NS | 81,8 ± 1,6 *** | 77,5 ± 3,6 ** | 56,5 ± 3,7 *** | 81,1 ± 2,9 *** | 86 ± 2,3 *** |
| CRL8316 | 74,3 ± 1,4 *** | 89,8 ± 2,9 * | 106,6 ± 2 * | 94 ± 3,2 NS | 26,9 ± 0,9 *** | 79,8 ± 2,9 ** | 73,9 ± 2,1 *** | 78,9 ± 4,0 *** | 68,8 ± 2,4 *** | 84,2 ± 1,9 *** | 79,1 ± 4,8 ** | 84,8 ± 2,3 ** |
| CRL8285 | 85,1 ± 2 ** | 96,9 ± 1,6 NS | 89,8 ± 2,7 * | 72,7 ± 1,8 *** | 68,7 ± 2,9 *** | 90,6 ± 1,6 * | 102,8 ± 3,1 NS | 83,7 ± 2,3 *** | 89,3 ± 1,7 ** | 92,5 ± 3,5 NS | 78,4 ± 1,6 *** | 93,4 ± 1,8 * |
| CRL8270 | 75,6 ± 0,9 *** | 52,2 ± 3,2 *** | 50,7 ± 1,8 *** | 51,1 ± 3,8 *** | 42,5 ± 0,8 *** | 58,8 ± 1,8 *** | 80 ± 3,2 *** | 51,5 ± 2,4 *** | 22,9 ± 4,3 *** | 52,2 ± 2,4 *** | 30,8 ± 2,1 *** | 49,4 ± 1,1 *** |
| CRL8366 | 64,4 ± 1,2 *** | 66,3 ± 1,6 *** | 68,4 ± 1,6 *** | 84,6 ± 3 * | 31,2 ± 0,7 *** | 76,1 ± 1,5 *** | 71,3 ± 4,5 *** | 46,3 ± 2,6 *** | 25 ± 4 *** | 61,6 ± 3,5 *** | 45,2 ± 2,2 *** | 67,2 ± 0,9 *** |
| CRL8336 | 82,3 ± 1,8 *** | 82,3 ± 1,6 *** | 101,1 ± 4,3 NS | 88,2 ± 1,4 *** | 90,9 ± 0,9 ** | 93,1 ± 3,2 NS | 92,6 ± 3,4 NS | 73,9 ± 0,7 *** | 96,2 ± 2,9 NS | 114,2 ± 1,9 ** | 87,9 ± 3,1 ** | 91,7 ± 1,5 *** |
| CRL8330 | 86,6 ± 0,7 *** | 75,5 ± 3,7 *** | 73,3 ± 1,8 *** | 57,4 ± 2,3 *** | 70,5 ± 2,4 *** | 94,7 ± 2,1 NS | 59,9 ± 4,2 *** | 87,2 ± 3 ** | 77,6 ± 3 *** | 76,3 ± 2,4 *** | 59,8 ± 1 *** | 48,5 ± 2,1 *** |
| CRL8339 | 69,1 ± 2,1 *** | 73,6 ± 1,8 *** | 87,1 ± 2,6 ** | 87,4 ± 2 ** | 83,7 ± 0,6 *** | 65,5 ± 2,2 *** | 74,4 ± 1,8 *** | 82,7 ± 3,5 ** | 81 ± 2,8 ** | 49,6 ± 2,2 *** | 52,3 ± 2,3 *** | 84,3 ± 1,8 *** |

x ± y = valeur moyenne ± erreur standard sur la moyenne

Condition contrôle = 100 %

(NS : $p > 0,05$; * : $p < 0,05$; ** : $p < 0,01$; *** : $p < 0,001$)

Il apparaît que plusieurs des composés induisent une faible inhibition pouvant atteindre 20-30 % de la prolifération cellulaire globale des lignées tumorales considérées et que ces composés ne semblent pas présenter de spécificité tissulaire.

**3. - Détermination de la dose maximale tolérée (DMT) :**

L'évaluation de la dose maximale tolérée a été réalisée chez des souris B6D2F1/Jico âgées de 4 à 6 semaines. Les composés ont été administrés par voie intrapéritonéale à des doses croissantes s'échelonnant de 2,5 à 160 mg/kg. La valeur de la DMT (exprimée en mg/kg) est déterminée à partir de l'observation du taux de survie des animaux sur une période de 14 jours après une administration unique du produit considéré. L'évolution pondérale des animaux est également suivie pendant cette période. Lorsque que la valeur de la DMT est supérieure à 160 mg/kg, la valeur de la DMT est assimilée à 160 mg/kg par défaut.

Les résultats de l'estimation de la dose maximale tolérée (DMT) sont rassemblés dans le tableau suivant :

TABLEAU III

| Doses Maximales Tolérées | |
|---|---|
| Composés CRL | DMT (mg/kg) |
| CRL8246 (Exemple 1) | > 160 |
| CRL8284 (Exemple 2) | > 160 |
| CRL8311 (Exemple 3) | > 160 |
| CRL8271 (Exemple 4) | > 160 |
| CRL8244 (Exemple 5) | > 160 |
| CRL8321 (Exemple 7) | > 160 |
| CRL8245 (Exemple 8) | > 160 |
| CRL8314 (Exemple 9) | > 160 |
| CRL8318 (Exemple 10) | > 160 |
| CRL8317 (Exemple 12) | > 160 |
| CRL8319 (Exemple 14) | > 160 |
| CRL8283 (Exemple 15) | > 160 |
| CRL8315 (Exemple 16) | > 160 |
| CRL8255 (Exemple 17) | > 160 |
| CRL8247 (Exemple 18) | > 160 |
| CRL8256 (Exemple 19) | > 160 |
| CRL8254 (Exemple 20) | > 160 |
| CRL8316 (Exemple 21) | > 160 |
| CRL8285 (Exemple 22) | > 160 |
| CRL8270 (Exemple 23) | > 160 |
| CRL8266 (Exemple 24) | > 160 |
| CRL8336 (Exemple 26) | > 160 |
| CRL8330 (Exemple 27) | > 160 |
| CRL8339 (Exemple 28) | > 160 |

Les produits de cette famille ne présentent pas de toxicité directe et peuvent donc être utilisés *in vivo* à des concentrations tissulaires élevées, donc à des posologies fortes.

**4. - Activité antitumorale in vivo en association avec un agent cytotoxique**

Les essais ont été réalisés sur les modèles de :

- adénocarcinome mammaire murin MXT hormonosensible (MXT-HS),
- lymphome P 388,

en présence ou non d'agents cytotoxiques tels que le cyclophosphamide, l'étoposide, la doxorubicine ou la vincristine.
Lorsque la valeur de DMT d'un produit a été déterminée, son activité antitumorale *in vivo* a été caractérisée aux doses de DMT/2, DMT/4 et DMT/8 sur le modèle de l'adénocarcinome mammaire d'origine murine MXT-HS et sur le modèle du lymphome P388. C'est la dose qui a présenté la meilleure activité antitumorale sur ces différents modèles qui a été retenue et utilisée dans le cadre des traitements combinés avec les cytotoxiques.
Dans tous les exemples présentés ci-après, quelque que soit le modèle (adénocarcinome mammaire MXT-HS ou lymphome P 388), la condition contrôle est représentée par un lot de 9 souris auxquelles est administré pendant 5 semaines consécutives et à raison de 5 administrations (lundi, mardi, mercredi, jeudi et vendredi) par semaine un volume de 0,2 ml de sérum physiologique contenant le solvant utilisé pour dissoudre les différents composés de formule (I) et (Ia) utilisés.
Au cours de ces essais, ont été déterminés :

i)- <u>le taux de survie des souris.</u>

Ce taux de survie a été calculé sous forme d'un rapport T/C :

T = (Nombre de jours de survie de la souris médiane du lot de souris traitées) (Souris médiane traitée) - (Nombre de souris mortes dans les jours qui ont précédé celui de la souris médiane traitée)
\+ ------------------------------------------------------------
(Nombre de souris mortes le même jour que la souris médiane traitée)

C = (Nombre de jours de survie de la souris médiane du lot de souris contrôle) (Souris médiane traitée) - (Nombre de souris mortes dans les jours qui ont précédé celui de la souris médiane traitée)
\+ ------------------------------------------------------------
(Nombre de souris mortes le même jour que la souris médiane contrôle)

Ce rapport représente le temps de survie moyen de la souris médiane du lot des souris traitées par rapport au temps de survie moyen de la souris médiane du lot des souris contrôles. Ainsi, une molécule induit une augmentation significative ($P < 0.05$) de la survie des animaux lorsque l'indice T/C excède 130%. Par contre elle présente un effet toxique lorsque cette valeur de T/C est inférieure à 70%.
ii)- <u>La croissance tumorale</u> en mesurant deux fois par semaine (lundi et vendredi) la surface des tumeurs MXT-HS ou P388 greffées. Cette surface est calculée, en effectuant le produit de la valeur des deux plus grands axes perpendiculaires de la tumeur. La valeur de ces axes est mesurée à l'aide d'un pied à coulisse.

4.1. Adénocarcinome mammaire murin (MXT-HS)

Le modèle de l'adénocarcinome mammaire murin MXT hormono-sensible (MXT-HS) greffé sur des souris B6D2F1/Jlco âgées de 4 à 6 semaines est dérivé des canaux galactophores de glande mammaire (Watson C. *et al*. Cancer Res. 1977; 37: 3344-48).
On donnera à titre d'exemple les résultats obtenus en utilisant les composés 1 et 20 soit seuls, soit en association avec les agents cytotoxiques.

***A*** - composé 1 ou CRL 8246 :

Traitements 1 et 1 bis

Le composé 1 est administré seul. La première injection du produit est réalisée au septième jour post-greffe (J7) pendant quatre semaines consécutives à raison de 5 injections par semaine (lundi, mardi, mercredi, jeudi et vendredi) et à la dose de 20 mg/kg.

Traitement 2

Le cyclophosphamide (CPA) est administré seul. La première injection du produit est réalisée au quatorzième jour post-greffe (J14) pendant trois semaines consécutives à raison de 3 injections par semaine (lundi, mercredi et vendredi) et à la dose de 10 mg/kg.

Traitement 3

Le composé 1 est co-administré avec le cyclophosphamide. Dans ce cas, la première injection du composé 1 est réalisée au septième jour post-greffe (J7) pendant quatre semaines consécutives à raison de 5 injections par semaine (lundi, mardi, mercredi, jeudi et vendredi) à la dose de 20 mg/kg et la première injection du cyclophosphamide est réalisée au quatorzième jour post-greffe (J14) pendant trois semaines consécutives à raison de 3 injections par semaine (lundi, mercredi et vendredi) à la dose de 10 mg/kg.

Traitement 4

L'étoposide (ETO) est administré seul. La première injection du produit est réalisée au quatorzième jour post-greffe (J14) pendant trois semaines consécutives à raison de 3 injections par semaine (lundi, mercredi et vendredi) et à la dose de 10 mg/kg.

Traitement 5

La doxorubicine (DOX) est administrée seule. La première injection du produit est réalisée au quatorzième jour post-greffe (J14) pendant trois semaines consécutives à raison de 3 injections par semaine (lundi, mercredi et vendredi) et à la dose de 5 mg/kg.

Traitement 6

Le composé 1 est co-administré avec l'étoposide. Dans ce cas, la première injection du composé 1 est réalisée au septième jour post-greffe (J7) pendant quatre semaines consécutives à raison de 5 injections par semaine (lundi, mardi, mercredi, jeudi et vendredi) à la dose de 20 mg/kg et la première injection de l'étoposide est réalisée au quatorzième jour post-greffe (J14) pendant trois semaines consécutives à raison de 3 injections par semaine (lundi, mercredi et vendredi) à la dose de 10 mg/kg.

Traitement 7

Le composé 1 est co-administré avec la doxorubicine. Dans ce cas, la première injection du composé 1 est réalisée au septième jour post-greffe (J7) pendant quatre semaines consécutives à raison de 5 injections par semaine (lundi, mardi, mercredi, jeudi et vendredi) à la dose de 20 mg/kg et la première injection de l'adriamycine est réalisée au quatorzième jour post-greffe (J14) pendant trois semaines consécutives à raison de 3 injections par semaine (lundi, mercredi et vendredi) à la dose de 5 mg/kg.
On donnera dans les tableaux IV et V les résultats obtenus pour le temps de survie pour le composé 1 :

TABLEAU IV

| Traitements | T/C (exprimé en %) |
|---|---|
| 1 (composé 1) | 100 |
| 2 (CPA) | 122 |
| 3 (Composé 1 + CPA) | 135 |

TABLEAU V

| Traitements | T/C (exprimé en %) |
|---|---|
| 1 bis (composé 1) | 95 |
| 4 (ETO) | 130 |
| 5 (DOX) | 92,5 |
| 6(Composé 1 + ETO) | 150 |
| 7(Composé 1 + DOX) | 145 |

Ces résultats montrent que la co-administration du composé 1 avec les cytotoxiques : cyclophosphamide, étoposide ou doxorubicine, augmente de manière significative le temps de survie moyen de la souris médiane des différents lots de souris ainsi traitées par rapport au temps de survie moyen de la souris médiane du lot des souris contrôles. De plus, cette augmentation du temps de survie moyen de la souris médiane des différents lots de souris traitées avec ces co-administrations est significativement plus long que celui obtenu avec les traitements impliquant ces cytotoxiques utilisés seuls.

L'étude de la croissance tumorale a par ailleurs mis en évidence les résultats suivants pour le composé 1. Dans le tableau VI, ci-dessous, sont indiqués en pourcentage les diminutions (-) ou les augmentations (+) de la surface des tumeurs MXT-HS induites avec les différents traitements 1, 2 et 3 de l'exemple 1 par rapport à la condition contrôle au 31$^{ème}$ jour après la greffe tumorale, soit après 19 administrations du composé 1 et 8 administrations de cyclophosphamide utilisés ou non en co-administration. Au 31$^{ème}$ jour post-greffe, 89% des animaux contrôles sont encore en vie (soit 8 animaux sur 9).

TABLEAU VI

| Traitements | Surface tumorale (exprimée en %) |
|---|---|
| 1 (composé 1) | -19,5 |
| 2 (CPA) | -23,6 |
| 3 (Composé 1+CPA) | - 49.6 |

Les résultats montrent que la co-administration du composé 1 avec le cyclophosphamide induit de manière hautement significative une diminution de la croissance des tumeurs MXT-HS plus importante que celle induite par les traitements impliquant le composé 1 ou le cyclophosphamide utilisés seuls.

***B* -** Composé 21 ou CRL 8256 :

Autre exemple, celui relatif au composé 21 utilisé seul ou en association avec l'étoposide.

Traitement 10

Le composé 21 est administré seul. La première injection du produit est réalisée au septième jour post-greffe (J7) à raison de 5 injections par semaine (lundi, mardi, mercredi, jeudi et vendredi) pendant cinq semaines consécutives et à la dose de 40 mg/kg.

Traitement 20

L'étoposide (ETO) est administré seul. La première injection du produit est réalisée au septième jour post-greffe (J7) à raison de 3 injections par semaine (lundi, mercredi et vendredi) pendant trois semaines consécutives et à la dose de 10 mg/kg.

Traitement 30

Le composé 21 est co-administré avec l'étoposide. Dans ce cas, la première injection du composé 21 est réalisée au septième jour post-greffe (J7) à raison de 5 injections par semaine (lundi, mardi, mercredi, jeudi et vendredi) pendant cinq semaines consécutives à la dose de 40 mg/kg et la première injection de l'étoposide est réalisée au septième jour post-greffe (J7) à raison de 3 injections par semaine (lundi, mercredi et vendredi) pendant trois semaines consécutives à la dose de 10 mg/kg.

Dans le tableau VII sont reportés les résultats du temps de survie obtenus avec le composé 21.

TABLEAU VII

| Traitements | T/C (exprimé en %) |
|---|---|
| 10 (composé 21) | 110 |
| 20 (ETO) | 124 |
| 30 (composé 21 + ETO) | 138 |

Ces résultats montrent que la co-administration du composé 21 avec l'étoposide induit une augmentation significative du temps de survie moyen de la souris médiane du lot de souris ainsi traitées par rapport au temps de survie moyen de la souris médiane du lot des souris contrôles. De plus, cette augmentation du temps de survie moyen de la souris médiane du lot des souris traitées avec cette co-administration est significativement plus long que celui obtenu avec les traitements impliquant cette 2-quinolone ou ce cytotoxique utilisés seuls.

**4.2.Lymphone P 388 :**

Les souris CDF1 âgées de 4 à 6 semaines sont greffées avec un morceau de tumeur P388 (provenant d'une banque de tumeurs maintenues au laboratoire) en sous-cutanée dans le flanc droit au jour J0. Afin de se placer dans une situation proche de la réalité clinique, nous attendons le 5$^{ème}$ jour post-greffe (J5) avant de commencer le traitement. Ceci, car après ce laps de temps les tumeurs P388 sous cutanées sont palpables.

A titre d'exemple les résultats obtenus avec les composés 1 (CRL 8246) et 20 (CRL 8247) seuls ou en association avec la vincrinstine sont reportés ci-après.

Traitement 1

Le composé 1 est administré seul. La première injection du produit est réalisée au cinquième jour post-greffe (J5) à raison de 5 injections par semaine (lundi, mardi, mercredi, jeudi et vendredi) pendant cinq semaines consécutives et à la dose de 40 mg/kg.

Traitement 2

Le composé 20 est administré seul. La première injection du produit est réalisée au cinquième jour post-greffe (J5) à raison de 5 injections par semaine (lundi, mardi, mercredi, jeudi et vendredi) pendant cinq semaines consécutives et à la dose de 40 mg/kg.

Traitement 3

La vincristine (VCR) est administrée seule. La première injection du produit est réalisée au cinquième jour post-greffe (J5) à raison de 3 injections par semaine (lundi, mercredi et vendredi) pendant trois semaines consécutives et à la dose de 0,63 mg/kg.

Traitement 4

Le composé 1 est co-administré avec la vincristine. Dans ce cas, la première injection du composé CRL8246 est réalisée au cinquième jour post-greffe (J5) à raison de 5 injections par semaine (lundi, mardi, mercredi, jeudi et vendredi) pendant cinq semaines consécutives à la dose de 40 mg/kg et la première injection de vincristine est réalisée au cinquième jour post-greffe (J5) à raison de 3 injections par semaine (lundi, mercredi et vendredi) pendant trois semaines consécutives à la dose de 0,63 mg/kg.

Traitement 5

Le composé 20 est co-administré avec la vincristine. Dans ce cas, la première injection du composé CRL8247 est réalisée au cinquième jour post-greffe (J5) à raison de 5 injections par semaine (lundi, mardi, mercredi, jeudi et vendredi) pendant cinq semaines consécutives à la dose de 40 mg/kg et la première injection de vincristine est réalisée au cinquième jour post-greffe (J5) à raison de 3 injections par semaine (lundi, mercredi et vendredi) pendant trois semaines consécutives à la dose de 0,63 mg/kg.

Dans le tableau IX sont présentés les résultats obtenus avec les traitements 1 à 5 mentionnés ci-dessus, sur le temps de survie des souris.

TABLEAU IX

| Traitements | T/C (exprimé en %) |
|---|---|
| 1 (composé 1) | 120 |
| 2 (composé 20) | 125 |
| 3(VCR) | 122 |
| 4 (composé 1 + VCR) | 144 |
| 5 (composé 20 + VCR) | 164 |

Ces résultats montrent que la co-administration des composés 1 et 20 avec la vincristine augmente de manière hautement significative le temps de survie moyen de la souris médiane des différents lots de souris ainsi traitées par rapport au temps de survie moyen de la souris médiane du lot des souris contrôles. De plus, cette augmentation du temps de survie moyen de la souris médiane des différents lots de souris traitées avec ces co-administrations est significativement plus long que celui obtenu avec les traitements impliquant ces deux composés 1 et 20 ou la vincristine utilisés seuls.

On donnera ci-après des exemples de modalité d'utilisation des composés de formule (I) et (Ia) dans des protocoles de mono ou polychimiothérapie par des agents cytotoxiques. Dans ces protocoles, les composés de formule (I) et (Ia) seront appelés, pour simplifier, "2-quinolone".

**A. Tumeurs solides**

**1°/ Cancers du poumon**

**1.1. Non à petites cellules (stade avancé) :**

- au protocole recommandé (T. Le Chevalier et al., J. Clin. Oncol. 1994 ; 12 : 360-367) sont ajoutées les perfusions intraveineuses d'une 2-quinolone :

| | dose | voie | jours |
|---|---|---|---|
| • 2-quinolone | 200-2000 mg/m²/jour<br>ou 5 – 50 mg/kg/jour<br>perfusion de 1 h | i.v. | $J_1$, $J_8$, $J_{15}$, $J_{22}$, $J_{29}$, et $J_{36}$ |
| • navelbine | 30 mg /m²/jour | i.v. | $J_1$, $J_8$, $J_{15}$, $J_{22}$, $J_{29}$, et $J_{36}$ |
| • cisplatine | 120 mg/m² | i.v. | $J_1$ et $J_{29}$ |

Cette cure est à répéter 8 fois.

**1.2. A petites cellules (stade avancé) :**

- au protocole recommandé CAV ou VAC (B.J. Roth et al., J. Clin. Oncol. 1992 ; 10 : 282-291) sont ajoutées les perfusions de 2-quinolone :

| | dose | voie | jours |
|---|---|---|---|
| • 2-quinolone | 200-2000 mg/m²/jour<br>ou 5 – 50 mg/kg/jour<br>perfusion de 1 h | i.v. | $J_1$ |
| • cyclosphophamide | 1000 mg /m²<br>bolus | i.v. | $J_1$ |
| • doxorubicine | 40 à 50 mg/m²<br>bolus | i.v. | $J_1$ |
| • vincristine | 1 à 1,4 mg/m²<br>bolus<br>(max 2 mg) | i.v. | $J_1$ |

Cette cure est à répéter 6 fois tous les 21 jours.

- au protocole recommandé Pt-E (B.J. Roth et al., J. Clin. Oncol. 1992 ; 10 : 282-291) sont ajoutées les perfusions de 2-quinolone.

| | dose | voie | jours |
|---|---|---|---|
| • 2-quinolone | 200-2000 mg/m²/jour <u>ou</u> 5 – 50 mg/kg/jour perfusion de 1 h | i.v. | $J_1$ - $J_5$ |
| • cisplatine | 20 mg mg /m²/jour perfusion de 20 à 60 minutes | i.v. | $J_1$ - $J_5$ |
| • étoposide | 80 mg/m²/jour perfusion de 60 minutes | i.v. | $J_1$ - $J_5$ |

chaque cycle est répété tous les 21 jours et la cure comprend 6 cycles.

**1.3. Cancer bronchique non à petites cellules, localement avancé ou métastatique :**

- monochimiothérapie :

| | dose | voie | jours |
|---|---|---|---|
| • 2-quinolone | 200-2000 mg/m²/jour <u>ou</u> 5 – 50 mg/kg/jour perfusion de 1 h | i.v. | $J_1$, $J_8$, $J_{15}$ puis 1 semaine/repos |
| • gemcitabine | 1000 mg/m²/jour perfusion de 0,5 heure | i.v. | $J_1$, $J_8$, $J_{15}$ puis 1 semaine/repos |

la cure pouvant comporter la répétition de ce cycle de 4 semaines.

- association gemcitabine/cisplatine

| | dose | voie | jours |
|---|---|---|---|
| • 2-quinolone | 200-2000 mg/m²/jour <u>ou</u> 5 – 50 mg/kg/jour perfusion de 1 h | i.v. | $J_1$ - $J_5$, $J_8$ - $J_{15}$ |
| • gemcitabine | 1000 mg/m²/jour perfusion de 0,5 heure | i.v. | $J_1$, $J_8$, $J_{15}$ |
| • cisplatine | 20 mg/m²/jour perfusion de 20-60 minutes | i.v. | $J_1$ |

la cure comportant la répétition de ce cycle tous les 21 jours.

**2°/ <u>Cancers du sein</u>**

- protocole CMF en traitement adjuvant du cancer du sein opérable (G. Bonnadonna et al., N. Engl. J. Med. ;1976 ; 294 : 405-410) :

| | dose | voie | jours |
|---|---|---|---|
| • 2-quinolone | 200-2000 mg/m²/jour <u>ou</u> 5 – 50 mg/kg/jour perfusion de 1 h | i.v. | $J_1$ à $J_{14}$ |
| • cyclophosphamide | 100 mg /m²/jour | orale | $J_1$ à $J_{14}$ |
| • méthotrexate | 40 mg/m² bolus | i.v. | $J_1$ et $J_8$ |
| • 5-FU | 600 mg/m² | i.v. | $J_1$ et $J_8$ |

chaque cycle est répété tous les 28 jours et la cure comporte 6 cycles.

- protocole AC (B. Fisher et al., J. Clin. Oncol. ; 1990 ; 8 : 1483 - 1496) en traitement adjuvant :

| | dose | voie | jours |
|---|---|---|---|
| • 2-quinolone | 200-2000 mg/m²/jour <u>ou</u> 5 – 50 mg/kg/jour perfusion de 1 h | i.v. | $J_1$ |
| • doxorubicine | 60 mg /m² bolus | i.v. | $J_1$ |
| • cyclophosphamide | 600 mg/m² bolus | i.v. | $J_1$ |

chaque cycle est répété tous les 21 jours et la cure comporte 4 cycles.

**- cancers du sein avec métastases :**

- dans le protocole FAC (A.U. Buzdar et al., Cancer 1981 ; 47 : 2537 - 2542) et ses différentes adaptations, les perfusions de 2-quinolone sont ajoutées selon le schéma (non limitatif) suivant :

| | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_5$ et $J_8 - J_{12}$ ou $J_1 - J_5$ |
| • 5-FU | 500 mg /m²/jour bolus | i.v. | $J_1$ et $J_8$ ou $J_1 - J_2$ |
| • doxorubicine | 50 mg/m² bolus | i.v. | $J_1$ ou $J_1$ et $J_2$ |
| • cyclophosphamide | 500 mg/m² | bolus i.v. ou orale | $J_1$ $J_1$ |

chaque cycle est répété toutes les 3 semaines jusqu'au diagnostic d'une nouvelle progression de la maladie.

- dans le protocole CAF (G. Falkson et al., Cancer 1985 ; 56 : 219 - 224) :

| | dose | voie | jours |
|---|---|---|---|
| • 2-quinolone | 200-2000 mg/m²/jour ou 5 – 50 mg/kg/jour perfusion de 1 h | i.v. | $J_1 - J_{14}$ |
| • cyclophosphamide | 100 mg /m²/jour | orale | $J_1 - J_{14}$ |
| • doxorubicine | 30 mg/m² bolus | i.v. | $J_1$ et $J_8$ |
| • 5-FU | 500 mg/m² bolus | i.v. | $J_1$ et $J_8$ |

chaque cycle est répété tous les 28 jours jusqu'au diagnostic d'une nouvelle progression de la maladie.

- dans le protocole CMF :

| | dose | voie | jours |
|---|---|---|---|
| • 2-quinolone | 200-2000 mg/m²/jour ou 5 – 50 mg/kg/jour perfusion de 1 h | i.v. | $J_1 - J_5$ et $J_8$-$J_{12}$ |
| • cyclophosphamide | 600 mg /m²/jour bolus | I.v. | $J_1$ et $J_8$ |
| • méthotrexate | 40 mg/m²/jour bolus | i.v. | $J_1$ et $J_8$ |
| • 5-FU | 600 mg/m²/jour bolus | i.v. | $J_1$ et $J_8$ |

ce cycle est à répéter toutes les 3 à 5 semaines et la cure comporte 6 cycles.

- dans le protocole CMF-VP :

| | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_5$ $J_8 - J_{12}$ $J_{15} - J_{19}$ $J_{22} - J_{26}$ |
| • cyclophosphamide | 2 à 2,5 mg /kg/jour | orale | chaque jour |
| • méthotrexate | 25 à 50 mg/m²/jour | i.v. | $J_1$, $J_8$, $J_{15}$, $J_{22}$ |
| • 5-FU | 300 à 500 mg/m²/jour | i.v. | $J_1$, $J_8$, $J_{15}$, $J_{22}$ |
| • vincristine | 0,6 à 1,2 mg/m²/jour | i.v. | $J_1$, $J_8$, $J_{15}$, $J_{22}$ |
| • prednisone | 30 mg/m²/jour | orale | de $J_1$ à $J_{10}$ |

cette cure est à répéter toutes les 4 semaines.

- dans le protocole FEC :

| | dose | voie | jours |
|---|---|---|---|
| • 2-quinolone | 200-2000 mg/m²/jour<br>ou 5 – 50 mg/kg/jour<br>perfusion de 1 h | i.v. | $J_1 - J_5$<br>et $J_8$ - $J_{12}$ |
| • 5-FU | 600 mg /m²/jour | i.v | $J_1$ et $J_8$ |
| • épirubicine | 50 mg/m² | i.v. | $J_1$ |
| • cyclophosphamide | 600 mg/m² | i.v. | $J_1$ |

cette cure est à répéter toutes les 3 semaines.

- dans le protocole MMC-VBC (C. Brambilla et al., Tumori, 1989; 75 : 141-144)

| | dose | voie | jours |
|---|---|---|---|
| • 2-quinolone | 200-2000 mg/m²/jour<br>ou 5 – 50 mg/kg/jour<br>perfusion de 1 heure | i.v. | $J_1 - J_5$<br>et $J_{15}$ - $J_{19}$ |
| • mitomycine C | 10 mg /m²<br>bolus | i.v | $J_1$ |
| • vinblastine | 50 mg/m²/jour<br>bolus | i.v. | $J_1$ et $J_{15}$ |

cette cure est à répéter tous les 28 jours jusqu'au diagnostic de progression de la maladie.

- dans le protocole NFL (S.E. Jones et al., J. Clin. Oncol. 1991 ; 9 : 1736 - 1739):

| | dose | voie | jours |
|---|---|---|---|
| • 2-quinolone | 200-2000 mg/m²/jour <u>ou</u> 5 – 50 mg/kg/jour perfusion de 1 h. | i.v. | $J_1 - J_5$ |
| • mitoxantrone | 10 mg /m² bolus | i.v | $J_1$ |
| • 5-FU | 1000 mg /m² en perfusion de 24 heures | i.v | $J_1 - J_3$ |
| • leucovorine | 100 mg/m² bolus | i.v. | $J_1$ |

la cure comporte deux cycles espacés de 21 jours puis nécessite une évaluation.

Les perfusions de 2-quinolone peuvent également être associées au traitement des cancers du sein avec métastases lorsque un taxoïde est utilisé, par exemple:

- avec paclitaxel (F.A. Holmes et al., J. Natl Cancer Inst. 1991 ; 83 : 1797 - 1805) dans le traitement des formes avec métastases éventuellement résistantes aux anthracyclines :

| | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_5$ |
| • paclitaxel | 175 mg /m² en perfusion de 3 à 24 heures | i.v | $J_1$ |

Ce cycle est répété tous les 21 jours jusqu'à ce qu'une nouvelle progression de la maladie soit diagnostiquée.

- avec docetaxel (C.A. Hudis et al., J. Clin. Oncol. 1996 ; 14 : 58 -65), dans le cancer du sein localement avancé ou métastatique, résistant ou en rechute après chimiothérapie cytoxique (ayant comporté une anthracycline) ou

en rechute au cours d'un traitement adjuvant :

| | dose | voie | jours |
|---|---|---|---|
| • 2-quinolone | 200-2000 mg/m²/jour <u>ou</u> 5 – 50 mg/kg/jour perfusion de 1 h | i.v. | $J_1$ - $J_5$ |
| • docetaxel | 100 mg /m² ou 60-100 mg/m² en perfusion de 1 heure (ou de 24 heures) | i.v | $J_1$ |

Ce cycle est répété tous les 21 jours pour une cure de 2 cycles ou jusqu'à apparition d'une progression de la maladie.

- dans les protocoles d'intensification de dose, associant une transplantation de cellules médullaires autologues et de cellules-souches du sang périphérique, en consolidation du traitement de première intention, par exemple :

  - protocole CPB (W.P. Peters et al., J. Clin. Oncol. 1993 ; 11 : 132 - 1143), dans lequel la perfusion i.v. de cellules-souches a lieu les jours $J_{-1}$, $J_0$ et $J_1$ :

| | dose | voie | jours |
|---|---|---|---|
| • 2-quinolone | 200-2000 mg/m²/jour <u>ou</u> 5 – 50 mg/kg/jour perfusion de 1 h | i.v. | $J_{-6}$ à $J_{-1}$ |
| • cyclophosphamide | 1875 mg /m² en perfusion de 1 heure | i.v | $J_{-6}$ à $J_{-4}$ |
| • cisplatine | 55 mg/m²/jour en perfusion continue de 24 heures | i.v. | $J_{-6}$ à $J_{-4}$ |
| • carmustine (BCNU) | 600 mg/m²/jour en perfusion de 2 heures | i.v. | $J_{-3}$ |

- protocole CTCb (K. Antman et al., J. Clin. Oncol. 1992 ; 10 : 102 - 110), dans lequel la perfusion i.v. de cellules-souches a lieu le jour $J_0$ :

| | dose | voie | jours |
|---|---|---|---|
| • 2-quinolone | 200-2000 mg/m²/jour <u>ou</u> 5 – 50 mg/kg/jour perfusion de 1 h | i.v. | $J_{-7}$ à $J_{-1}$ |
| • cyclophosphamide | 1500 mg /m² en perfusion continue de 24 heures (4 doses) | i.v | $J_{-7}$ à $J_{-3}$ |
| • thiotepa | 125 mg/m² en perfusion continue de 24 heures(4 doses) | i.v. | $J_{-7}$ à $J_{-3}$ |
| • carboplatine | 200 mg/m² en perfusion continue de 24 heures(4 doses) | i.v. | $J_{-7}$ à $J_{-3}$ |

- protocole CTM (L.E. Damon et al., J. Clin. Oncol. 1989 ; 7 : 560-571 et I.C. Henderson et al., J. Cellular Biochem. 1994 (Suppl 18B) : 95) dans lequel la perfusion i.v, de cellules-souches hématopdietiques a lieu le jour $J_0$

| | dose | voie | jours |
|---|---|---|---|
| • 2-quinolone | 200-2000 mg/m²/jour <u>ou</u> 5 – 50 mg/kg/jour perfusion de 1 h | i.v. | $J_{-6}$ à $J_{-1}$ |
| • cyclophosphamide | 1500 mg /m²/jour en perfusion de 1 heure | i.v | $J_{-6}$ à $J_{-3}$ |
| • thiotepa | 150 mg/m²/jour en perfusion de 2 heures | i.v. | $J_{-6}$ à $J_{-3}$ |
| • mitoxantrone | 10 - 15 mg/m² en perfusion de 1 heure | i.v. | $J_{-6}$ à $J_{-3}$ |

**3°/ Cancers gynécologiques**

**3.1 Cancer de l'ovaire :**

- pour le traitement des carcinomes ovariens, en particulier métastatiques :

  ***i*) protocole PAC** (G. A. Omura et al. J. Clin. Oncol. 1989 ; 7 : 457 - 465) : les perfusions de 2-quinolones sont administrées selon le schéma suivant :

| | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_5$ |
| • cisplatine | 50 mg /m² (ou 40 –90 mg/m²) perfusion de 1 à 2 heures | i.v. | $J_1$ |
| • doxorubicine | 50 mg/m² bolus (ou 30 à 50 mg/m²) | i.v. | $J_1$ |
| • cyclosphosphamide | 1000 mg/m² perfusion de 1 à 2 heures (ou 200 à 600 mg/m²) | i.v. | $J_1$ |

ce cycle est répété tous les 21 à 28 jours et la cure comporte 8 cycles.

*ii*) **protocole altretamine,** d'après A. Marietta et al. (Gynecol. Oncol. 1990 ; 36: 93 -96) :

| | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_5$ $J_8 - J_{12}$ |
| • altretamine | 200 mg /m²/jour divisés en 4 doses | orale | $J_1 - J_{15}$ |

la cure comportant deux cycles, espacés de 28 jours.

***ii*) protocole paclitaxel :** les 2-quinolones peuvent être ajoutées au protocole de paclitaxel tel qu'il a été décrit par W.P. Mc Guire et al. (Ann. Intern. Med. 1989 ; 111 : 273 - 279) :

| | dose | voie | jours |
|---|---|---|---|
| • 2-quinolone | 200-2000 mg/m²/jour<br>ou 5 – 50 mg/kg/jour<br>perfusion de 1 h | i.v. | $J_1 - J_3$ |
| • paclitaxel | 135 mg /m²<br>perfusion de 3 heures ou de 24 heures | i.v. | $J_1$ |

la cure comportant deux de ces cycles, espacés de 28 jours (avec évaluation à l'issue).

- pour le traitement des carcinomes ovariens métastatiques et réfractaires, les 2-quinolones peuvent être ajoutés au protocole de seconde intention, à base de topotécan :

| | dose | voie | jours |
|---|---|---|---|
| • 2-quinolone | 200-2000 mg/m²/jour<br>ou 5 – 50 mg/kg/jour<br>perfusion de 1 h | i.v. | $J_1 - J_5$ |
| • topotecan | 1,5 mg /m²/jour<br>perfusion de 0,5 heure | i.v. | $J_1 - J_5$ |

la cure comportant deux cycles, espacés de 21 jours (avec évaluation à l'issue) d'après A.P. Kudelka et al. (J. Clin. Oncol. 1996 ; 14 : 1552 - 1557).

**3.2 Tumeurs trophoblastiques :**

- chez les patientes à faible risque, les 2-quinolones pourront être associées au protocole décrit par H. Takamizawa et al. (Semin. Surg. Oncol. 1987 ; 3 : 36 - 44) :

| | dose | voie | jours |
|---|---|---|---|
| • 2-quinolone | 200-2000 mg/m²/jour ou 5 – 50 mg/kg/jour perfusion de 1 h | i.v. | $J_1 - J_5$ |
| • methotrexate (MTX) | 20 mg /jour | i.m. | $J_1 - J_5$ |
| • dactinomycine (DACT) | 0,5 mg /jour en bolus | i.v. | $J_1 - J_5$ |

(protocole MTX-DATC).

**3.3 Cancers de l'utérus :**

- les 2-quinolones peuvent également être associées au protocole CAV (ou VAC) selon le schéma ci-après :

| | dose | voie | jours |
|---|---|---|---|
| • 2-quinolone | 200-2000 mg/m²/jour ou 5 – 50 mg/kg/jour perfusion de 1 h | i.v. | $J_1 - J_3$ |
| • cyclophosphamide | 750 – 1200 mg/m² en perfusion | i.v. | $J_1$ |
| • doxorubicine | 45 –50 mg/m² en perfusion | i.v. | $J_1$ |
| • vincristine | 1,4 mg/m² | i.v. | $J_1$ |

la cure comportant la répétition de ce cycle tous les 21 jours.

- dans le protocole FAP :

| | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_5$ |
| • fluorouracile (5-FU) | 600 mg/m²/jour | i.v. | $J_1$, $J_8$ |
| • doxorubicine | 30 mg/m² | i.v. | $J_1$ |
| • cisplatine | 75 mg/m² | i.v. | $J_1$ |

la cure comportant la répétition de ce cycle tous les 21 ou 28 jours.

**4°/ Cancers du testicule**

- les 2-quinolones peuvent également être associées aux protocoles du cancer des testicules :

| Protocole BEP : | dose | voie | jours |
|---|---|---|---|
| • 2-quinolone | 200-2000 mg/m²/jour<br>ou 5 – 50 mg/kg/jour<br>perfusion de 1 h | i.v. | $J_1 - J_5$ |
| • bléomycine | 30 mg/m²<br>en perfusion | i.v. | $J_1$ |
| • étoposide | 100 mg/m²/jour<br>en perfusion | i.v. | $J_1 - J_5$ |
| • cisplatine | 20 mg/m²/jour | i.v. | $J_1 - J_5$ |

la cure comportant 3 cycles, à raison de 1 cycle tous les 21 jours.

5°/ **Cancers de la vessie**

- les 2-quinolones peuvent être associées au protocole CISCA2 (aussi appelé PAC)

| | dose | voie | jours |
|---|---|---|---|
| • 2-quinolone | 200-2000 mg/m²/jour<br>ou 5 – 50 mg/kg/jour<br>perfusion de 1 h | i.v. | $J_1 - J_5$ |
| • cisplatine | 50 mg/m² | i.v. | $J_1$ |
| • cyclophosphamide | 600 mg/m²<br>en perfusion | i.v. | $J_1$ |
| • doxorubicine | 75 mg/m²<br>en perfusion | i.v. | $J_1$ |

le cycle étant à répéter toutes les 3 semaines.

- dans le protocole MVAC (d'après CN Stemberg et l., J. Urol. 1988 ; 139 : 461 - 469) :

| | dose | voie | jours |
|---|---|---|---|
| • 2-quinolone | 200-2000 mg/m²/jour<br>ou 5 – 50 mg/kg/jour<br>perfusion de 1 h | i.v. | $J_1 - J_3$<br>$J_{15} - J_{18}$<br>$J_{22} - J_{25}$ |
| • méthotrexate | 30 mg/m² bolus | i.v. | $J_1$, $J_{15}$, $J_{22}$ |
| • vinblastine | 3 mg/m² | i.v. | $J_2$ ou<br>$J_2$, $J_{15}$, $J_{22}$ |
| • doxorubicine | 30 mg/m² bolus | i.v. | $J_2$ |

| | | | |
|---|---|---|---|
| • cisplatine | 70-100 mg/m² perfusion de 1 h | i.v. | $J_1$ ou $J_2$ |

ce cycle étant répété toutes les 4 à 5 semaines, au minimum pour 2 cycles.

**6°/ Carcinomes naso-pharyngés / Cancers de la tête et du cou**

- Les 2-quinolones peuvent être valablement associées aux protocoles de polychimiothérapie utilisés dans le traitement de ces cancers :

**6.1 Cancers naso-pharyngés :**

- protocole ABVD :

| | Dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_3$ $J_8 - J_{10}$ ou $J_{15} - J_{17}$ |
| • doxorubicine | 30 mg/m²/jour | i.v. | $J_1$ et $J_8$ ou $J_{15}$ |
| • bléomycine | 10 mg/m²/jour | i.v. | $J_1$ et $J_8$ ou $J_{15}$ |
| • vinblastine | 6 mg/m²/jour | i.v. | $J_1$ et $J_8$ ou $J_{15}$ |
| • dacarbazine | 200 mg/m²/jour | i.v. | $J_1$ et $J_8$ ou $J_{15}$ |

la cure comportant 1 à 6 cycles répétés à raison de 1 cycle toutes les 4 semaines.

**6.2 Cancers de la tête et du cou avec métastases :**

- dans le protocole Pt-FU (ex : pour les cancers du pharynx) : d'après le DVAL Study Group (New Engl. J. M. 1991 ; 324 : 1685 - 1690) :

| | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_5$ |
| • cisplatine | 100 mg/m² perfusion de 1 heure | i.v. | $J_1$ |
| • fluorouracile (5-FU) | 1000 mg/m²/jour perfusion continue | i.v. | $J_1 - J_5$ |

la cure comportant deux cycles, à raison de 1 cycle toutes les 3 semaines.

**7°/ <u>Sarcomes des tissus mous</u>**

- Les 2-quinolones peuvent être introduites dans un protocole tel que le protocole CYVADIC :
  - d'après H.M. Pinedo et al. (Cancer 1984 ; 53 : 1825) :

| | dose | voie | jours |
|---|---|---|---|
| • 2-quinolone | 200-2000 mg/m²/jour<br>ou 5 – 50 mg/kg/jour<br>perfusion de 1 h | i.v. | $J_1-J_5$<br>$J_8-J_{10}$<br>$J_{15}-J_{17}$ |
| • cyclophosphamide (Cy) | 500 mg/m² bolus | i.v. | $J_2$ |
| • vincristine (V) | 1,5 mg/m²/jour bolus | i.v. | $J_1$, $J_8$, $J_{15}$ |
| • doxorubicine (A) | 50 mg/m² bolus | i.v. | $J_2$ |
| • dacarbazine (DIC) | 250 mg/m²/jour<br>perfusion de 15 minutes | i.v. | $J_1-J_5$ |

la cure comportant la répétition de ce cycle toutes les 4 semaines, d'abord pour 2 cycles.

**8°/ Cancer de la prostate hormono-refractaire, avec métastases**

- dans le protocole VBL-estramustine, d'après G.R. Hudis et al. (J. Clin. Oncol. 1992 ; 10 : 1754 : 1761):

| | Dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_3$, $J_8 - J_{10}$ $J_{15} - J_{17}$, $J_{22} - J_{24}$ $J_{29} - J_{31}$, $J_{36} - J_{38}$ |
| • vinblastine | 4 mg/m²/jour bolus | i.v. | $J_1$, $J_8$, $J_{15}$, $J_{22}$, $J_{29}$, $J_{36}$ |
| • estramustine | 200 mg/m² tid (600 mg/m²/jour) | orale | chaque jour pendant 6 semaines |

un cycle de traitement durant 6 semaines et étant suivi de 2 semaines d'intervalle libre.

**9°/ <u>Cancers des cellules germinales</u>**

*i)* pour les tumeurs de pronostic favorable :

- protocole Pt-E, d'après G.J. Bosl et al. (J. Clin. Oncol. 1988 ; 6 : 1231 - 1238)

| | Dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_5$ |
| • cisplatin (Pt) | 20 mg/m²/jour perfusion de 20 à 60 minutes | i.v. | $J_1 - J_5$ |
| • étoposide (E) | 100 mg/m² /jour perfusion de 1 heure | i.v. | $J_1 - J_5$ |

la cure comportant 4 cycles, à raison de 1 cycle tous les 21 ou 28 jours.

*ii*) pour les tumeurs avec métastases :

- protocole PEB, d'après S.D. Williams et al. (N. Eng. J. Med. 1987 ; 316 : 1435-1440):

| | Dose | voie | jours |
|---|---|---|---|
| • 2-quinolone | 200-2000 mg/m²/jour <u>ou</u> 5 – 50 mg/kg/jour perfusion de 1 h | i.v. | $J_1-J_5$ $J_9-J_{11}$ $J_{16}-J_{18}$ |
| • cisplatine (P) | 20 mg/m²/jour perfusion de 20 à 60 minutes | i.v. | $J_1-J_5$ |
| • étoposide (E) | 100 mg/m²/jour perfusion de 1 heure | i.v. | $J_2$, $J_9$, $J_{16}$ |
| • bléomycine (B) | 30U (ou mg)/jour bolus | i.v. | $J_1-J_5$ |

la cure comportant 4 cycles, à raison de 1 cycle tous les 21 jours.

10°/ <u>Cancers du rein</u>

- **carcinome rénal métastatique :** les 2-quinolones peuvent être introduites dans le protocole décrit par M. J. Wilkinson et al. (Cancer 1993 ; 71 : 3601-3604):

| | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_5$ $J_8 - J_{15}$ |
| • floxuridine | 0,075 mg/kg/jour perfusion continue | i.v. | $J_1 - J_{14}$ |

la cure comportant deux cycles espacés de 28 jours.

- **néphroblastome** : les 2-quinolones peuvent être introduites dans le protocole DAVE:

| | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_3$ $J_8 - J_{10}$ |
| • dactinomycine | 0,6 mg/m²/jour | i.v. | $J_1$, $J_8$ |
| • doxorubicine | 30 mg/m²/jour | i.v. | $J_1$, $J_8$ |
| • cyclophosphamide | 200 mg/m² /jour perfusion de 1 heure | i.v. | $J_1$, $J_8$ |

à raison d'un cycle toutes les 3 à 4 semaines.

**11°/ <u>Cancers du tube digestif</u>**

**11.1 Cancers de l'oesophage :**

- les 2-quinolones peuvent être introduites dans le protocole FAP selon :

| | dose | voie | jours |
|---|---|---|---|
| • 2-quinolone | 200-2000 mg/m²/jour<br>ou 5 – 50 mg/kg/jour<br>perfusion de 1 h | i.v. | $J_1 - J_3$<br>$J_8 - J_{10}$ |
| • 5-fluorouracile (5-FU) | 600 mg/m² | i.v. | $J_1$, $J_8$ |
| • doxorubicine | 30 mg/m² | i.v. | $J_1$ |
| • cisplatine | 75 mg/m² | i.v. | $J_1$ |

ce cycle étant répété toutes les 3 à 4 semaines.

**11.2 Cancers de l'estomac**

- dans les carcinomes gastriques avancés et/ou avec métastases :
- protocole EAP (d'après P. Preusser et al. , J. Clin. Oncol. 1989 ; 7 : 1310) :

| | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_5$, $J_8 - J_{10}$ |
| • étoposide | 120 mg/m²/jour perfusion de 1 heure | i.v. | $J_3$, $J_4$, $J_5$ ou $J_4 - J_6$ |
| • doxorubicine | 20 mg/m²/jour bolus | i.v. | $J_1$, $J_7$ |
| • cisplatine | 40 mg/m²/jour perfusion de 1 heure | i.v. | $J_2$, $J_8$ |

à raison de 1 cycle tous les 28 jours.

- protocole FAMtx : d'après J.A. Wils et al. (J. Clin. Oncol. 1991 ; 89 : 827):

| | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_3$ |
| • fluorouracile (5-FU) (F) | 1500 mg/m² bolus 1 heure près le méthotrexate | i.v. | $J_1$ |
| • doxorubicine (A) | 30 mg/m² bolus | i.v. | $J_{15}$ |
| • méthotrexate (Mtx) | 1500 mg/m² perfusion de 30 minutes | i.v. | $J_1$ |

la cure comportant d'abord deux cycles, espacés de 28 jours.

- chez certains malades, ce protocole ou sa variante (l'épirubicine remplaçant la doxorubicine) pourront être utilisés selon le schéma suivant :

| | dose | voie | jours |
|---|---|---|---|
| • 2-quinolone | 200-2000 mg/m²/jour<br>ou 5 – 50 mg/kg/jour<br>perfusion de 1 h | i.v. | $J_1-J_3$ |
| • fluorouracile (5-FU) | 1500 mg/m² | i.v. | $J_1$ |
| • doxorubicine (A)<br>ou<br>• épirubicine (A) | 30 mg/m² bolus<br><br>60 mg/m² bolus | i.v.<br><br>i.v. | $J_1$ = FAMTx<br><br>$J_1$ = FEMTx |
| • méthotrexate<br>(à perfuser avant le 5-FU) | 1500 mg/m² | i.v. | $J_1$ |
| • leucovorine | 15 mg/m²/jour | orale | $J_2-J_4$ |

**12°/ Cancers colo-rectaux**

- les 2-quinolones peuvent être introduites dans le protocole de traitement adjuvant FU-Levamizole du cancer colo-rectal (d'après C.G. Moertel et al., N. Eng. J. Med. 1990 ; 322 : 352) :

| | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_5$ $J_{29} - J_{31}$ |
| • 5-fluorouracile | 450 mg/m²/jour bolus | i.v. | $J_1 - J_5$ |
| • 5-fluorouracile | 450 mg/m² bolus | i.v. | $J_{29}$ |
| • lévamisole | 50 mg tid | orale | 3 jours/semaine une semaine sur deux |

le traitement en bolus par le 5-FU étant répété chaque semaine après la phase d'induction $J_1$ - $J_5$, pendant .52 semaines ; celui par une 2-quinolone étant répété sur le même rythme, le jour du bolus de 5-FU puis les 2 jours suivants.

- **pour le traitement du cancer colo-rectal, refractaire au traitement par 5-fluorouracile (5-FU) et avec métastases :**

  - d'après M.L. Rothenberg et al. (J. Clin. Oncol. 1996 ; 14 : 1128-1135) :

| | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_3$, $J_8 - J_{10}$, $J_{15} - J_{17}$, $J_{22} - J_{24}$ |
| • irinotecan | 125 mg/m²/jour | i.v. | $J_1$, $J_8$, $J_{15}$, $J_{22}$ |

la cure comportant deux cycles, espacés de 42 jours.

13°/ Sarcomes de Kaposi

- les 2-quinolones peuvent être associées aux deux protocoles utlisant des antracyclines formulées en liposomes :

*i*) protocole décrit par P.S. Gill et al. (J. Clin. Oncol. 1995 ; 13 : 996-1003) et C.A. Presant et al. (Lancet 1993 ; 341 : 1242-1243) :

| | dose | voie | jours |
|---|---|---|---|
| • 2-quinolone | 200-2000 mg/m²/jour ou 5 – 50 mg/kg/jour perfusion de 1 h | i.v. | $J_1 - J_3$ et $J_{15} - J_{17}$ |
| • daunorubicine liposomale | 20 mg/m²/jour perfusion de 1 heure | i.v. | $J_1$, $J_{15}$ |

la cure comportant deux cycles répétés à 28 jours d'intervalle avant d'évaluer les effets.

*ii)* protocole de M. Harrison et al. (J. Clin. Oncol. 1995 ; 13 : 914-920) :

| | dose | voie | jours |
|---|---|---|---|
| • 2-quinolone | 200-2000 mg/m²/jour ou 5 – 50 mg/kg/jour perfusion de 1 h | i.v. | $J_1 - J_3$ |
| • doxorubicine liposomale | 20 mg/m² perfusion de 30 minutes | i.v. | $J_1$ |

la cure comportant deux cycles répétés à 28 jours d'intervalle avant d'évaluer les effets.

14°/ Mélanomes métastatiques

- les 2-quinolones peuvent également être incorporées aux protocoles combinés de traitement des mélanomes malins métastatiques :

- protocole DTIC/TAM : d'après G. Cocconi et al. (N. Eng. J. Med. 1992 ; 327 : 516), la cure comprenant la répétition de 4 cycles, à raison de 1 cycle tous les 21 jours, selon le schéma ci-après :

| | dose | voie | jours |
|---|---|---|---|
| • 2-quinolone | 200-2000 mg/m²/jour<br>ou 5 – 50 mg/kg/jour<br>perfusion de 1 h | i.v. | $J_1 - J_5$ |
| • dacarbazine (DTIC) | 250 mg/m²/jour<br>perfusion [15 à 30 min. si cathéter central] ou [30 min. si perfusion périphérique dans 250 ml ] | i.v. | $J_1 - J_5$ |
| • tamoxifen (TAM) | 20 mg/m²/jour | orale | $J_1 - J_5$ |

la cure comportant 4 cycles à raison de 1 cycle tous les 21 jours.

**15°/ Carcinome neuroendocrine**

- les 2-quinolones peuvent être associées au protocole décrit par C.G. Moertel et al. (Cancer 1991 ; 68 : 227) :
  - protocole Pt-E :

| | dose | voie | jours |
|---|---|---|---|
| • 2-quinolone | 200-2000 mg/m²/jour ou 5 – 50 mg/kg/jour perfusion de 1 h | i.v. | $J_1 - J_3$ |
| • étoposide | 130 mg/m²/jour perfusion de 1 heure | i.v. | $J_1 - J_3$ |
| • cisplatine | 45 mg/m²/jour perfusion de 1 heure | i.v. | $J_2$, $J_3$ |

la cure comportant deux cycles répétés tous les 28 jours.

**16°/ <u>Cancer du pancréas</u>**

- **adéno-carcinome pancréatique de stade avancé :** les 2-quinolones peuvent être associées au traitement par gemcitabine, selon le protocle de M. Moore et al. (Proc. Am. Soc. Clin. Oncol. 1995 ; 14 : 473) :

| | dose | voie | jours |
|---|---|---|---|
| • 2-quinolone | 200-2000 mg/m²/jour ou 5 – 50 mg/kg/jour perfusion de 1 h | i.v. | $J_1 - J_3$, $J_8 - J_{10}$, $J_{15}$, $J_{22}$,$J_{29}$, $J_{36}$, $J_{43}$, $J_{57}$ |
| • gemcitabine | 1000 mg/m² perfusion de 0,5 heure | i.v. | $J_1$, $J_8$, $J_{15}$, $J_{22}$, $J_{29}$, $J_{36}$,$J_{43}$, puis $J_{57}$ puis une fois/semaine pendant 3 semaines puis 1 semaine repos et évaluation |

**B. Onco-hématologie**

**1°/ Leucémies aigues de l'adulte**

**1.1. Leucémie lymphoblastique aigue :**

1.1.1. Protocole de Linker

Les 2-quinolones peuvent être ajoutées aux protocoles de Linker - Chimiothérapie d'induction et chimiothérapie de consolidation . (voir C.A. Linker et al. Blood 1987 ; 69 : 1242-1248 et C.A. Linker et al. Blood 1991 ; 78 : 2814-2822) selon les schémas suivants :

*i)* chimiothérapie d'induction :

| | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_5$, $J_8 - J_{12}$, $J_{15} - J_{19}$ |
| • daunorubicine | 50 mg/m² bolus toutes les 24 heures (30 mg/m² chez les patients de + de 50 ans) | i.v. | $J_1$, $J_2$, $J_3$ |
| • vincristine | 2 mg bolus | i.v. | $J_1$, $J_8$, $J_{15}$, $J_{22}$ |
| • prednisone | 60 mg/m²/jour | orale | $J_1 - J_{28}$ |
| • L-asparaginase | 6000 U/m² | i.m. | $J_{17} - J_{28}$ |

*ii)* chimiothérapie de consolidation (régime A) :

| | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_5$, $J_8 - J_{12}$ |
| • daunorubicine | 50 mg/m² bolus toutes les 24 heures | i.v. | $J_1$, $J_2$ |
| • vincristine | 2 mg bolus | i.v. | $J_1$, $J_8$ |
| • prednisone | 60 mg/m²/jour divisés en 3 doses | orale | $J_1 - J_{14}$ |
| • L-asparaginase | 12000 U/m² | i.m. | $J_2$, $J_4$, $J_7$, $J_9$ et $J_{14}$ |

la cure de consolidation A comprend 4 cycles consécutifs tels que celui décrit ci-dessus = Cycles 1, 3, 5 et 7.

*iii)* chimiothérapie de consolidation (régimes B et C) :
Les régimes décrits ci-dessous correspondent aux cycles de consolidation 2, 4, 6 et 8 (régime B) et 9 (régime C), décrits par C.A. Linker et al. :

| régime B : | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_5$, $J_8 - J_{12}$ |
| • Ara-C | 300 mg/m² perfusion de 2 heures | i.v. | $J_1$, $J_4$, $J_8$, $J_{11}$ |
| • téniposide | 165 mg/m² perfusion de 2 heures (4 cycles) | i.v. | $J_1$, $J_4$, $J_8$, $J_{11}$ |

| régime C : | dose | voie | jours |
| --- | --- | --- | --- |
| • 2-quinolone | 200-2000 mg/m²/jour <u>ou</u> 5 – 50 mg/kg/jour perfusion de 1 h | i.v. | $J_1 - J_5$ |
| • méthotrexate | 690 mg/m² perfusion continue de 42 heures | i.v. | $J_1 - J_2$ |
| • leucovorin | 15 mg/m² toutes les 6 heures | orale | $J_2 - J_5$ |

1.1.2. Protocole de Hoelzer

Les produits revendiqués pourront être ajoutés aux cytotoxiques de ce protocole de polychimiothérapie (D. Hoelzer et al., Blood 1984 ; 64 : 38-47, D. Hoelzer et al. , Blood 1988 ; 71 : 123-131) selon le schéma suivant :

*i)* chimiothérapie d'induction / Phase 1 :

| | dose | voie | jours |
|---|---|---|---|
| • 2-quinolone | 200-2000 mg/m²/jour <u>ou</u> 5 – 50 mg/kg/jour perfusion de 1 h | i.v. | $J_1-J_5$, $J_8-J_{12}$, $J_{15}-J_{19}$ |
| • daunorubicine | 25 mg/m² | i.v. | $J_1$, $J_8$, $J_{15}$, $J_{22}$ |
| • vincristine | 1,5 mg/m² (maximum 2 mg) | i.v. | $J_1$, $J_8$, $J_{15}$, $J_{22}$ |
| • prednisone | 60 mg/m² | orale | $J_1-J_{28}$ |
| • L-asparaginase | 5000 U/m² (maximum 2 mg) | i.m. | $J_1-J_{14}$ |

*ii*) chimiothérapie d'induction / Phase 2 :

La phase 2 de l'induction pourra être réalisée comme suit :

| | dose | voie | jours |
|---|---|---|---|
| • 2-quinolone | 200-2000 mg/m²/jour <u>ou</u> 5 – 50 mg/kg/jour perfusion de 1 h | i.v. | $J_{29}-J_{33}$, $J_{36}-J_{40}$, $J_{43}-J_{47}$ |
| • cyclosphosphamide | 650 mg/m² (maximum 1000 mg) | i.v. | $J_{29}$, $J_{43}$, $J_{57}$ |
| • cytarabine | 75 mg/m²/jour perfusion de 1 heure | i.v. | $J_{31}-J_{34}$, $J_{38}-J_{41}$, $J_{45}-J_{48}$, $J_{52}-J_{55}$ |
| • mercaptopurine | 60 mg/m² | orale | $J_{29}-J_{57}$ |
| • methotrexate | 10 mg/m²/jour (maximum 15 mg) | i.v. | $J_{31}$, $J_{38}$, $J_{45}$, $J_{52}$ |

*iii)* chimiothérapie de ré-induction / Phase 1 :

| | dose | voie | jours |
|---|---|---|---|
| • 2-quinolone | 200-2000 mg/m²/jour <u>ou</u> 5 – 50 mg/kg/jour perfusion de 1 h | i.v. | $J_1-J_5$, $J_8-J_{12}$, $J_{15}-J_{19}$, $J_{22}-J_{26}$ |
| • doxorubicine | 25 mg/m²/jour | i.v. | $J_1$, $J_8$, $J_{15}$, $J_{22}$ |
| • dexamethasone | 10 mg/m²/jour | orale | $J_1-J_{28}$ |
| • vincristine | 1,5 mg/m²/jour (maximum 2 mg) | orale | $J_1$, $J_8$, $J_{15}$ et $J_{22}$ |

***iv)*** chimiothérapie de ré-induction / Phase 2 :

| | dose | voie | jours |
|---|---|---|---|
| • 2-quinolone | 200-2000 mg/m²/jour <u>ou</u> 5 – 50 mg/kg/jour perfusion de 1 h | i.v. | $J_{31}-J_{35}$, $J_{38}-J_{42}$ |
| • cyclophosphamide | 650 mg/m² (maximum : 1000 mg) | i.v. | $J_{29}$ |
| • cytarabine | 75 mg/m² | i.v. | $J_{31}-J_{34}$, $J_{38}-J_{41}$ |
| • thioguanine | 60 mg/m² | orale | $J_{29}-J_{42}$ |

**1.2. Leucémies myéloïdes aiguës:**

1.2.1. Traitement de l'adulte de tout âge

Les 2-quinolones peuvent être ajoutées, selon le schéma ci-dessous, au traitement incorporant la dose standard de cytarabine antérieurement décrit par R.O. Dilleman et al. (Blood, 1991 ; 78 : 2520-2526), Z.A. Arlin et al. (Leukemia 1990 ; 4 : 177-183) et P.H. Wiernik et al. (Blood 1992 ; 79 : 313-319) :

| | dose | voie | jours |
|---|---|---|---|
| • 2-quinolone | 200-2000 mg/m²/jour<br>ou 5 – 50 mg/kg/jour<br>perfusion de 1 h | i.v. | $J_1 - J_{12}$ |
| • cytarabine | 100-200 mg/m²/jour<br>en perfusion continue | i.v. | $J_1 - J_7$ |
| • daunorubicine | 45 mg/m²/jour en bolus<br>(30 mg/m²/jour si âge ≥ 60 ans) | i.v. | $J_1 - J_3$, ou<br>$J_8 - J_{10}$ |
| ou | | | |
| • mitoxantrone | 12 mg/m²<br>en bolus quotidien | i.v. | $J_1 - J_3$ |
| ou | | | |
| • idarubicine | 13 mg/m²<br>en bolus quotidien | i.v. | $J_1 - J_3$ |

1.2.2. Traitement de l'adulte d'âge inférieur à 60 ans

*i)* chimiothérapie d'induction :

Ce cycle d'induction incorpore l'administration de cytarabine à forte dose selon le schéma suivant :

| | dose | voie | jours |
|---|---|---|---|
| • 2-quinolone | 200-2000 mg/m²/jour<br>ou 5 – 50 mg/kg/jour<br>perfusion de 1 h | i.v. | $J_1 - J_{10}$ |
| • Ara-C (cytarabine) | 2000 mg/m²/jour<br>en perfusion de 2 heures,<br>toutes les 12 heures | i.v. | $J_1 - J_6$ |
| • daunorubicine | 60 mg/m²/jour<br>en perfusion continue de 24 heures | i.v. | $J_4 - J_6$ |
| ou<br>• cytarabine | 3000 mg/m²/jour<br>en perfusion de 1 heure,<br>toutes les 12 heures | i.v. | $J_1 - J_6$ |
| • daunorubicine | 45 mg/m² bolus<br>toutes les 24 heures | i.v. | $J_7 - J_9$ |

(afin de réduire le risque de toxicité S.N.C., en cas d'insuffisance rénale, ajuster la posologie de cytarabine à la clairance de la créatinine)
d'après L.E. Damon et al. (Leukemia 1994 ; 8 : 535-541), G.L. Phillips et al. (Blood 1991 ; 77 : 1429-1435) et G. Smith et al. (J. Clin. Oncol. 1997 ; 15 : 833-839).

*ii)* chimiothérapie de consolidation :

Le cycle, décrit ci-après, sera répété 8 fois, à raison de 1 cycle toutes les 4 à 6 semaines (d'après R.J. Mayer et al., N. Engl J. Med. 1994 ; 331 : 896-903) :

| | dose | voie | jours |
|---|---|---|---|
| • 2-quinolone | 200-2000 mg/m²/jour<br>ou 5 – 50 mg/kg/jour<br>perfusion de 1 h | i.v. | $J_1 - J_5$ |
| • cytarabine | 3000 mg/m²<br>en perfusion de 3 heures<br>toutes les 12 heures<br>(4 cycles) | i.v. | $J_1$, $J_3$, $J_5$ |
| puis<br>• cytarabine | 100 mg/m²/jour<br>toues les 12 heures | s.c. | $J_1 - J_5$ |
| • daunorubicine | 45 mg/m² bolus<br>(4 cycles) | i.v. | $J_1$ |

*iii*) chimiothérapie de consolidation (avec forte dose de cytarabine) :

Le cycle, décrit ci-après, devra être répété 2 fois et est adapté d'après G.L. Phillips et al. (Blood 1991 ; 77 : 1429-1435) ; S.N. Wolff et al. (J. Clin. Oncol. 1989 ; 7 : 1260 -1267) ; R.J. Mayer et al. (N. Engl J. Med. 1994 ; 331 : 896-903) :

| | dose | voie | jours |
|---|---|---|---|
| • 2-quinolone | 200-2000 mg/m²/jour<br>ou 5 – 50 mg/kg/jour<br>perfusion de 1 h | i.v. | $J_1 - J_{10}$ |
| • cytarabine | 3000 mg/m²<br>1 heure toutes les 12 heures | i.v. | $J_1 - J_6$ |
| • daunorubicine | 30-45 mg/m²/jour bolus<br>1 fois/jour | i.v. | $J_7 - J_9$ |

1.2.3. Traitement de l'adulte d'âge égal ou supérieur à 60 ans

Les substances revendiquées pourront être ajoutées aux protocoles de chimiothérapies de consolidation ci-après :

*i)* selon R.O. Dilman et al. (Blood 1991 ; 78 ; 2520-2526), Z.A. Arlin et al. (Leukemia 1990 ; 4 : 177-183), P.H. Wiernik et al. (1992 ; 79 : 313-319) :

| | dose | voie | jours |
|---|---|---|---|
| • 2-quinolone | 200-2000 $mg/m^2$/jour <u>ou</u> 5 – 50 mg/kg/jour perfusion de 1 h | i.v. | $J_1 - J_6$ |
| • cytarabine (Ara-C) | 100-200 $mg/m^2$ perfusion continue de 24 heures | i.v. | $J_1 - J_5$ |
| • daunorubicine | 30-45 $mg/m^2$/jour bolus | i.v. | $J_1, J_2$ |
| ou • mitoxantrone | 12 $mg/m^2$/jour bolus | i.v. | $J_1, J_2$ |
| ou • idarubicine | 13 $mg/m^2$/jour bolus | i.v. | $J_1, J_2$ |

*ii)* selon R.J. Mayer et al. (N. Engl. J. Med. 194 ; 331 : 896-903) :

| | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_6$ |
| • cytarabine | 100 mg/m² perfusion continue de 24 heures (4 cycles) | i.v. | $J_1 - J_5$ |
| puis • cytarabine | 100 mg/m² toutes les 12 heures | s.c. | $J_1, J_5$ |
| • daunorubicine | 45 mg/m²/jour bolus (4 cycles) | i.v. | $J_1$ |

*iii)* selon C.A. Linker et al. (Blood 1993 ; 81 : 311-318), N. Chao et al. (Blood 1993 ; 81 : 319-323) et A.M. Yeager at al. (N. Eng. J. Med. 1986 ; 315 : 145-147) :

Ce protocole comprend une transplantation de moëlle osseuse autologue (pratiquée le jour $J_0$) :

| | dose | voie | jours |
|---|---|---|---|
| • 2-quinolone | 200-2000 mg/m²/jour <u>ou</u> 5 – 50 mg/kg/jour perfusion de 1 h | i.v. | $J_{-7} - J_{-2}$ |
| • busulfan | 1 mg/kg qid (au total 16 doses) | orale | $J_{-7}$ à $J_{-4}$ |
| • étoposide | 60 mg/kg/jour perfusion de 10 heures | i.v. | $J_{-3}$ |

ou

| | dose | voie | jours |
|---|---|---|---|
| • 2-quinolone | 200-2000 mg/m²/jour <u>ou</u> 5 – 50 mg/kg/jour perfusion de 1 h | i.v. | $J_{-9} - J_{-1}$ |
| • busulfan | 1 mg/kg qid | orale | $J_{-9}$ à $J_{-6}$ |
| • cyclophosphamide | 50 mg/kg/jour perfusion de 1 heure | i.v. | $J_{-5}$ à $J_{-2}$ |

*iv)* en cas de transplantation de moëlle osseuse allogène HLA-compatible selon:

P.J. Tutscha et al. Blood 1987 ; 70 : 1382-1388,
F.R. Applebaum et al., Ann. Int. Med. 1984 ; 101 : 581-588 :

| | dose | voie | jours |
|---|---|---|---|
| • 2-quinolone | 200-2000 mg/m²/jour <u>ou</u> 5 – 50 mg/kg/jour perfusion de 1 h | i.v. | $J_{-7} - J_{-1}$ |
| • busulfan | 1 mg/kg qid (au total 16 doses) | orale | $J_{-7}$ à $J_{-4}$ |
| • cyclophosphamide | 60 mg/kg/jour perfusion de 1 heure | i.v. | $J_{-3}$ à $J_{-2}$ |

**2°/ <u>Leucémies chroniques de l'adulte</u>**

**2.1 Leucémie myéloïde chronique**

En phase myéloblastique, les 2-quinolones peuvent être ajoutées au traitement HU-Mith, décrit par C.A. Koller et al. (N. Engl. J. med. 1986 ; 315 : 1433-1438) :

| | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_5$<br>$J_8 - J_{12}$<br>$J_{15} - J_{19}$<br>$J_{22} - J_{26}$ |
| • hydroxyurée | 500 mg/jour | orale | tous les jours |
| • mithramycine | 25µg/kg/jour perfusion de 2-4 heures | i.v. | quotidien pendant 3 semaines puis 3 fois/semaine |

**2.2 Leucémie lymphocytaire chronique**

2.2.1 Protocole FCG-CLL

Les 2-quinolones peuvent être ajoutées aux combinaisons "chlorambucil pulsé" telles que décrites par E. Kimby et al. (Leuk. Lymphoma 1991 ; 5 (Suppl.) 93-96) et par le FCGCLL (Blood 1990 ; 75 : 1422-1425) :

| | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_5$, $J_8 - J_{12}$, $J_{15} - J_{22}$ |
| • chlorambucil | 0,1 mg/kg/jour | orale | 1 fois/jour |
| <u>ou</u> | | | |
| • chlorambucil | 0,4 mg/kg/jour tous les 14 jours | orale | $J_1$ |
| <u>et</u> | | | |
| • prednisone | 75 mg/jour | orale | $J_1 - J_3$ |

2.2.2 Protocole fludarabine-CdA

d'après H.G. Chun et al. (J. Clin. Oncol. 1991 ; 9 : 175-188), M.J. Keating et al. (Blood 1989 ; 74 : 19-25 / J. Clin. Oncol. 1991 ; 9 : 44-49) et A. Saven et al. (J. Clin. Oncol. 1995 ; 13 : 570-574) :

| | dose | voie | jours |
|---|---|---|---|
| • 2-quinolone | 200-2000 mg/m²/jour<br>ou 5 – 50 mg/kg/jour<br>perfusion de 1 h | i.v. | $J_1 - J_8$<br>(1 fois/mois pour 6 à 12 cycles) |
| • fludarabine | 25-30 mg/m²/jour<br>perfusion de 30 minutes<br>[toutes les 4 semaines pour 6 à 12 cycles) | i.v. | $J_1 - J_5$ |
| ou<br>• cladibrine | 0,09 mg/kg/jour<br>en perfusion continue<br>[1 cycle tous les 28 à 35 jours pour 1 à 9 cycles (médiane : 4 cycles)] | i.v. | $J_1 - J_7$ |

**3°/ Maladies lymphoprolifératives**

**3.1 Maladie de Hodgkin**

Les 2-quinolones peuvent être incorporées aux protocoles de polychimiothérapie utilisés classiquement pour le traitement du lymphome de Hodgkin :

3.1.1 Protocole AVDB

d'après G. Bonnadonna et al. (Cancer Clin. Trials 1979 ; 2 : 217-226) et G.P. Canellos et al. (N. Engl. J. Med. 1993 ; 327 : 1478-1484) :

| | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_3$, $J_{15} - J_{18}$ |
| • doxorubicine (A) | 25 mg/m² bolus | i.v. | $J_1$, $J_{15}$ |
| • bléomycine (B) | 10 U/m² bolus | i.v. | $J_1$, $J_{15}$ |
| • vinblastine (V) | 6 mg/m² bolus | i.v. | $J_1$, $J_{15}$ |
| • dacarbazine (D) | 375 mg/m² bolus | i.v. | $J_1$, $J_{15}$ |

la cure comportant 6 à 8 cycles, à raison de 1 cycle tous les 28 jours.

3.1.2 Protocole MOPP/ABVD

d'après G. Bonnadonna et al. (Ann. Intern. Med. 1986 ; 104 : 739-746) et G. P. Canellos et al. (N. Engl. J. Med. 1993 ; 327 : 1478-1484) :

Le protocole MOPP doit être alterné avec le protocole ABVD (cf. § 3.1.1) tous les 28 jours et la cure comporte 6 cycles :

| Protocole MOPP : | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_3$, $J_8 - J_{11}$ et $J_{14} - J_{17}$ |
| • mechlorethamine (M) | 6 mg/m² bolus | i.v. | $J_1$, $J_8$ |
| • vincristine (O) | 1,4 mg/m² bolus (pas de maximum) | i.v. | $J_1$, $J_8$ |
| • procarbazine (P) | 100 mg/m²/jour | orale | $J_1 - J_{14}$ |
| prednisone (P) | 40 mg/m²/jour | orale | $J_1$-$J_{14}$ |

3.1.3 Protocole Stanford V

d'après N.L. Bartlett et al. (J. Clin. Oncol. 1995 ; 13 : 1080-1088) :

| | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_5$ $J_8 - J_{12}$ $J_{15} - J_{19}$ $J_{22} - J_{26}$ |
| • doxorubicine | 25 mg/m² | i.v. | $J_1$, $J_{15}$ |
| • vinblastine | 6 mg/m² bolus (4mg/m² au cours du cycle 3 si âge ≥ 50 ans) | i.v. | $J_1$, $J_{15}$ |
| • mechlorethamine (M) | 6 mg/m² bolus | i.v. | $J_1$ |
| • vincristine | 1,4 mg/m² bolus (dose max : 2 mg) [1 mg/m² au cours du cycle 3 si âge ≥ 50 ans) | i.v. | $J_1$, $J_{22}$ |
| • bléomycine | 5 U/m² | i.v. | $J_8$, $J_{22}$ |
| • étoposide | 60 mg/m² | orale | $J_{15}$, $J_{16}$ |
| • prednisone | 40 mg/m²/jour | orale | 1/fois semaine (semaines 1-9) |

la cure comportant 3 cycles à raison de 1 cycle tous les 28 jours.

3.1.4 Protocole EVA

d'après G.P. Canellos et al. (Proc. Am. Soc. Clin. Oncol. 1991 ; 10 : 273) :

| | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_5$ |
| • étoposide (E) | 100 mg/m² perfusion de 2 heures | orale | $J_1$, $J_2$, $J_3$ |
| • vinblastine (V) | 6 mg/m² bolus | i.v. | $J_1$ |
| • doxorubicine (A) | 50 mg/m² bolus | i.v. | $J_1$ |

la cure comportant 6 cycles, à raison de 1 cycle tous les 28 jours.

3.1.5 Protocole B-CAVe

d'après W.G. Harker et al. (Ann. Intern. Med. 1984 ; 101 : 440-446) :

| | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_3$ |
| • bléomycine (B) | 5 U/m² bolus | i.v. | $J_1$ |
| • lomustine (CCNU) | 100 mg/m² | orale | $J_1$ |
| • doxorubicine (A) | 60 mg/m² bolus | i.v. | $J_1$ |
| • vinblastine (Ve) | 5 mg/m² bolus | i.v. | $J_1$ |

la cure comportant 8 cycles, à raison de 1 cycle tous les 28 jours.

**3.2. Lymphomes non hodgkiniens.**

3.2.1. de bas grade de malignité

**i)- protocole CVP**

- d'après C.M. Bagley et al. (Ann. Intern. Med. 1972 ; 76 : 227 - 234) et C.S. Portlock et al: (Blood 1976 ; 47 : 747 - 756)

| | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_5$ |
| • cyclophosphamide (c) | 300-400 mg/m²/jour | orale | $J_1, J_5$ |
| • vincristine (V) | 1.4 mg/ m² bolus (max : 2 mg) | i.v. | $J_1$ |
| • prednisone (P) | 100 mg/m²/jour | orale | $J_1 - J_5$ |

Ce cycle est répété tous les 21 jours jusqu'à réponse maximale

**ii)- protocole I-COPA**

- d'après RV Smalley et al. (N. Eng. J. Med. 1992 ; 327 : :1336 - 1341)

| | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_5$ |
| • cyclophosphamide (C) | 600 mg/m² jour | i.v. | $J_1$ |
| • vincristine (O) | 1.2 mg/m² bolus (max : 2 mg) | i.v. | $J_1$ |
| • prednisone (P) | 100 mg/m²/jour | i.v. | $J_1 - J_5$ |
| • doxorubicine (A) | 50 mg/m² bolus | i.v. | $J_1$ |
| • interféron-alpha (I) | 6 MU/m² | i.m. | $J_{22} - J_{26}$ |

La cure comprend 8 à 10 cycles, à raison d'un cycle tous les 28 jours.

iii)- **protocole fludarabine-CdA**

- d'après P. Solol-Celigny et al. (Blood 1994 ; 84 (Supp. 1): 383a), H. Hoeschster et al. ; (Blood 1994 ; 84 (Suppl. 1): 564a et A.C. Kay (J. Clin. Oncol. 1992 ; 10 : 371-377)

| | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_7$ |
| • fludarabine | 25 mg/m² jour perfusion de 0.5 heure | i.v. | $J_1 - J_5$ |
| <u>ou</u> • fludarabine | 20 mg/m²/jour | i.v. | $J_1 - J_5$ |
| <u>et</u> cyclophosphamide | 600 - 1000 mg/m²/jour | i.v. | $J_1$ |
| <u>ou</u> cladribine | 0.1 mg/m²/jour perfusion de 24 heures | i.v. | $J_1 - J_7$ |

Pour la fludaribine, chaque cycle est répété tous les 28 jours ; pour la cladribine, chaque cycle est répété tous les 35 jours.

3.2.2. de grade de malignité intermédiaire

**i)- protocole CHOP ou CNOP**

- d'après EM McKelvey et al. (Cancer 1976 ; 38 : 1484 - 1493), J.O Armitage et al. (J. Clin. Oncol. 1984 ; 2 : 898 - 902) , S. Paulovsky et al. (Ann. Oncol. 1992 ; 3 : 205 - 209)

| | dose | voie | jours |
|---|---|---|---|
| • 2-quinolone | 200-2000 mg/m²/jour<br>ou 5 – 50 mg/kg/jour<br>perfusion de 1 h | i.v. | $J_1 - J_5$ |
| • cyclophosphamide (C) | 750 mg/m² jour | i.v. | $J_1$ |
| • doxorubicine (H) | 50 mg/ m² bolus | i.v. | $J_1$ |
| • vincristine (O) | 1.4 mg/ m² bolus<br>(max : 2 mg) | i.v. | $J_1$ |
| • prednisone (P) | 100 mg/m²/jour<br>(en 1 dose/jour) | orale | $J_1 - J_5$ |

pour le protocole CHOP

La mitoxantrone (N) peut être utilisée pour remplacer (protocole CNOP) la doxorubicine chez les patients de plus de 60 ans (dose : 12 mg/m$^2$ en bolus i;v. au jour J1 de chaque cycle).
La cure par le protocole CHOP ou CNOP comprend 6 à 8 cycles à raison de 1 cycle tous les 21 jours.

**ii)- protocole MACOP-B**

- d'après P. Klimo et al. (Ann. Intern. Med. 1985 ; 102 : 596 - 602) et I.A. Cooper et al. (J. Clin. Oncol. 1994 ; 12 : 769 - 778)

| | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_5$, $J_8 - J_{12}$ $J_{15} - J_{22}$, $J_{29} - J_{33}$ $J_{43} - J_{47}$, $J_{57} - J_{61}$ $J_{71} - J_{75}$ |
| • methotrexate (M) | 100 mg/m² bolus puis 300 mg/m² perfusion de 4 heures | i.v. | $J_8$, $J_{36}$, $J_{64}$ |
| • leucovorin | 15 mg qid | orale | $J_9$, $J_{37}$, $J_{65}$ |
| • doxorubicine (A) | 50 mg/m² bolus | i.v | $J_1$, $J_{15}$, $J_{29}$, $J_{43}$ $J_{57}$, $J_{71}$ |
| • cyclophosphamide (c) | 350 mg/m² bolus | i.v | $J_1$, $J_5$, $J_{29}$ $J_{43}$, $J_{57}$, $J_{71}$ |
| • vincristine (O) | 1.4 mg/ m² bolus (max : 2 mg) | i.v. | $J_8$, $J_{22}$, $J_{36}$ $J_{50}$, $J_{64}$, $J_{78}$ |
| • prednisone (P) | 75 mg/jour | orale | Chaque jour pendant 12 semaines |
| • bléomycine (B) | 10 U/ m² bolus | i.v. | $J_{22}$, $J_{50}$, $J_{78}$ |

Ce protocole de traitement s'étale sur 12 semaines et correspond à 1 cycle.

**iii)- protocole VACOP-B**

- d'après J.M. Connors et al. (Proc. Am. Soc. Clin. Oncol. 1990 ; 9 :254) :

| | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_5$, $J_8 - J_{12}$ $J_{15} - J_{22}$, $J_{29} - J_{34}$ $J_{43} - J_{47}$, $J_{57} - J_{61}$ $J_{71} - J_{75}$ |
| • etoposide (V) | 50 mg/m² | i.v. | $J_{15}$, $J_{43}$ $J_{71}$ |
| • etoposide | 100 mg/m² | orale | $J_{16}$, $J_{17}$, $J_{44}$, $J_{45}$ $J_{72}$, $J_{73}$ |
| • doxorubicine (A) | 50 mg/m² bolus | i.v | $J_1$, $J_{15}$, $J_{29}$, $J_{43}$ $J_{57}$, $J_{71}$ |
| • cyclophosphamide (c) | 350 mg/m² jour bolus | i.v | $J_8$, $J_{22}$, $J_{36}$ $J_{50}$, $J_{64}$, $J_{78}$ |
| • vincristine (O) | 1.2 mg/ m² bolus | i.v. | $J_8$, $J_{22}$, $J_{36}$ $J_{50}$, $J_{64}$, $J_{78}$ |
| • prednisone (P) | 45 mg/m²/jour | orale | 1/jour pendant 1 semaine, puis 4/jour les 11 semaines suivantes |

Chaque cycle durant 12 semaines.

**iv)- protocole m-BACOD / M-BACOD**

- d'après M.A. Shipp et al. (Ann. Int. Med. 1986 ; 140 : 757 - 765) et A.T. Skarin et al. (J. Clin. Oncol. 1983 ; 1 : 91 - 98)

| | dose | voie | jours |
|---|---|---|---|
| • 2-quinolone | 200-2000 mg/m²/jour<br>ou 5 – 50 mg/kg/jour<br>perfusion de 1 h | i.v. | $J_1 - J_5$, $J_8 - J_{12}$<br>$J_{15} - J_{19}$ |
| • methotrexate<br>(m)<br>ou<br>(M) | 200 mg/m²<br>perfusion de 4 heures<br><br>3000 mg/m²<br>perfusion de 4 heures | i.v.<br><br>i.v. | $J_8$, $J_{15}$<br>ou<br>$J_{15}$ |
| • leucovorin | 10 mg/m² qid<br>(6 doses au total)) | orale | $J_9$, $J_{16}$<br>ou $J_{16}$ |
| • bléomycine (B) | 4 U/m² bolus | i.v | $J_1$ |
| • doxorubicine (A) | 45 mg/m² bolus | i.v | $J_1$ |
| • cyclophosphamide (C) | 600 mg/m² bolus | i.v | $J_1$ |
| • vincristine (O) | 1.mg/ m² bolus | i.v. | $J_1$ |
| • dexaméthasone (D) | 6 mg/m²/jour | orale | $J_3 - J_5$ |

La cure comportant 10 cycles, à raison de 1 cycle tous les 21 jours.

**v)- protocole ProMACE/CytaBOM**

- d'après D.L. Longo et al. (J. Clin. Oncol. 1991 ; 9 : 25 - 38) :

| | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_5, J_8 - J_{12}$ |
| • cyclophosphamide (C) | 650 mg/m² perfusion de 0.5 heure | i.v | $J_1$ |
| • doxorubicine (A) | 25 mg/m² bolus | i.v | $J_1$ |
| • étoposide | 120 mg/m² perfusion de 1 heure | i.v | $J_1$ |
| • prednisone (P) | 60 mg/jour | orale | $J_1 . J_{14}$ |
| • cytarabine | 300 mg/m² bolus | i.v | $J_8$ |
| • bléomycine (B) | 5 U/m² bolus | i.v | $J_8$ |
| • vincristine (O) | 1,4 mg/ m² bolus | i.v | $J_8$ |

| | | | |
|---|---|---|---|
| • methotrexate | 120 mg/m²bolus | i.v | $J_8$ |
| • leucovorin | 25 mg/m² qid<br>(4 doses au total) | orale | $J_9$ |

La cure comportant 6 à 8 cycles, à raison de 1 cycle tous les 14 jours.

3.2.3. de grade de malignité bas ou intermédiaire

**i)- protocole de sauvetage ESHAP**

- en cas de récidive ou en cas d'échec du traitement de première ligne, d'après W.S. Velasquez et al. (J. Clin. Oncol. 1994 ; 12 : 1169 - 1176)

| | dose | voie | jours |
|---|---|---|---|
| • 2-quinolone | 200-2000 mg/m²/jour<br>ou 5 – 50 mg/kg/jour<br>perfusion de 1 h | i.v. | $J_1 – J_5$ |
| • etoposide (E) | 40 mg/m²<br>perfusion de 2 heures | i.v | $J_1 – J_4$ |
| • méthylprednisolone (S) | 500 mg/jour<br>perfusion de 15 minutes | i.v | $J_1$, $J_4$ |
| • cytarabine (HA) | 2000 mg/m²<br>perfusion de 3 heures | i.v | $J_5$ |
| • cisplatine (P) | 25 mg/ m²/jour bolus<br>perfusion continue<br>de 24 heures | i.v. | $J_1 – J_4$ |

La cure comportant 6 cycles, à raison de 1 cycle tous les 28 jours.

ii)- **protocole de sauvetage** MINE

- en cas de récidive ou en cas d'échec du traitement de première ligne, d'après F. Cabanillas et al. (Semin. Oncol. 1990 ; 17 (Suppl. 10) : 28 - 33)

| | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_5$ |
| • ifosfamide (I) | 1330 mg/ m² perfusion de 1 heure | i.v. | $J_1 - J_3$ |
| • mesna (M) | 1330 mg/ m² dans la perfusion de ifosfamide puis 266 mg/ m² bolus 4 et 8 heures après chaque dose de ifosfamide | i.v. | $J_1 - J_3$ |
| • mitoxantrone (M) | 8 mg/ m² perfusion de 15 minutes | i.v. | $J_1$ |
| • étoposide (E) | 65 mg/m²/jour perfusion de 1 heure | i.v | $J_1 - J_3$ |

Ce cycle étant à répéter tous les 21 jours.

**3.3. Lymphomes non hodgkiniens : lymphome de Burkitt, lymphome à petites cellules, lymphome lymphoblastique.**

3.3.1. Protocole de Magrath

- Les produits revendiqués pourront être associés aux protocoles de Magrath selon les schémas suivants :

i)- cycle 1

- d'après I.T. Magrath et al. (Blood 1984 ; 63 : 1102 - 1111)

| | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_5$. $J_8 - J_{12}$ |
| • cytarabine | 30 mg/m² | intra-thécale | $J_1$, $J_2$, $J_3$, $J_7$ |
| • cyclophosphamide | 1200 mg/ m² bolus | i.v. | $J_1$ |
| • methotrexate | 12.5 mg/m² (max : 12.5 mg) | Intra-thécale | $J_{10}$ |
| • methotrexate | 300 mg/m²/jour perfusion de 1 heure puis 60 mg/m²/h perfusion de 41 heures | i.v | $J_{10} - J_{11}$ |
| • leucovorin | 15 mg/m² bolus qid (8 doses successives) | i.v | A commencer 42 heures après le début de l'administration de méthotrexate |

**ii)- cycles 2 à 15**

- d'après I.T. Magrath et al. (1984) également

| | dose | voie | jours |
|---|---|---|---|
| • 2-quinolone | 200-2000 mg/m²/jour<br>ou 5 – 50 mg/kg/jour<br>perfusion de 1 h | i.v. | $J_1 - J_3$<br>$J_{10} - J_{11}$ |
| • cytarabine | 45 mg/m² | Intra-thécale | $J_1$, $J_2$<br>(cycles 2 et 3)<br>$J_1$<br>(cycles 4 et 6) |
| • Cyclophosphamide | 1200 mg/m² bolus | i.v. | $J_1$ |
| • doxorubicine | 40 mg/m² bolus | i.v. | $J_1$ |
| • vincristine | 1.4 mg/m² bolus<br>(max : 2 mg) | i.v. | $J_1$ |
| • méthotrexate | 12.5 mg/m²<br>(max : 12.5 mg) | Intra-thécale | $J_3$, $J_{10}$<br>(cycles 2 et 3)<br>$J_{10}$<br>(cycles 4, 5, 6) |

| | | | |
|---|---|---|---|
| • méthotrexate | 300 mg/m² perfusion de 1 heure puis 60 mg/m² perfusion continue de 41 heures | i.v. | $J_{10}$, $J_{11}$ (cycles 2 et 6 $J_{14}$, $J_{15}$ (cycles 7 – 15) |
| • leucovorin | 15 mg/m² bolus qid (8 doses consécutives) | i.v. | Commencer à la 42^e^ heure du traitement par méthotrexate |

la cure comportant 14 cycles, à raison d'un cycle tous les 28 jours.

**3.4 Macroglobulinémie de Waldenström**

3.4.1 Protocole CVP

d'après le protocole CVP décrit par M.A. Dimopoulous et al. (Blood 1994 ; 83 : 1452-1459) et C.S. Portlock et al. (Blood 1976 ; 47 : 747-756) :

| | dose | voie | jours |
|---|---|---|---|
| • 2-quinolone | 200-2000 mg/m²/jour ou 5 – 50 mg/kg/jour perfusion de 1 h | i.v. | $J_1 - J_5$ |
| • cyclophosphamide (C) | 300-400 mg/m²/jour | orale | $J_1 - J_5$ |
| • vincristine (V) | 1,4 mg/m²/jour bolus (max : 2 mg) | i.v. | $J_1$ |
| • prednisone (P) | 100 mg/m²/jour | orale | $J_1 - J_5$ |

la cure étant à poursuivre indéfiniment (1 cycle tous les 21 jours).

3.4.2 Protocole Fludarabine-CdA

d'après H.M. Kantarjian et al. (Blood 1990 ; 75 : 1928-1931) et M.A. Dinopoulous et al. (Ann. Intern. Med. 1993 ; 118 : 195-198) :

| | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_5$ |
| • fludarabine | 25-30 mg/m² perfusion de 0,5 heure | i.v. | $J_1 - J_5$ |

<u>ou</u>

| | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_7$ |
| • cladribine (CdA) | 0,09 mg/m²/jour perfusion continue | i.v. | $J_1 - J_7$ |

la cure comportant 6 à 12 cycles espacés de 28 jours dans le cas de la fludarabine et 2 cycles espacés de 28 jours également dans le cas de la cladribine.

**3.5 Myélome multiple**

3.5.1 Protocole MP

d'après R. Alexanian et al. (JAMA 1969 ; 208 : 1680-1685), A. Belch et al. (Br. J. Cancer 1988 ; 57 : 94-99) et F. Mandelli et al. (N. Engl. J. med. 1990 ; 322 : 1430-1434):

| | dose | voie | jours |
|---|---|---|---|
| • 2-quinolone | 200-2000 mg/m²/jour ou 5 – 50 mg/kg/jour perfusion de 1 h | i.v. | $J_1 - J_5$ |
| • melphalan (M) | 0,25 mg/kg/jour | orale | $J_1 - J_4$ |
| • prednisone (P) | 100 mg/jour | orale | $J_1 - J_4$ |

ou

| | dose | voie | jours |
|---|---|---|---|
| • 2-quinolone | 200-2000 mg/m²/jour ou 5 – 50 mg/kg/jour perfusion de 1 h | i.v. | $J_1 - J_5$ |
| • melphalan (M) | 9 mg/m²/jour | orale | $J_1 - J_4$ |
| • prednisone (P) | 100 mg/jour | orale | $J_1 - J_4$ |

la cure comportant au moins 12 cycles, à raison de 1 cycle toutes les 4 à 6 semaines.

3.5.2 Protocole VAD

d'après B. Barlogie et al. (N. Engl. J. Med. 1984 ; 310 : 1353-1356) :

| | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_5$ |
| • vincristine (V) | 0,4 mg/jour perfusion continue de 24 heures | i.v. | $J_1 - J_4$ |
| • doxorubicine (A) | 9 mg/m²/jour perfusion continue de 24 heures | i.v. | $J_1 - J_4$ |
| • dexaméthasone (D) | 40 mg/jour | i.v. | $J_1 - J_4$, $J_9 - J_{12}$, $J_{17} - J_{20}$ |

3.5.3 Protocole MP-interferon α

d'après O. Osterborg et al. (Blood 1993 ; 81 : 1428-1434) :

| | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_5$ |
| • melphalan (M) | 0,25 mg/kg/jour | orale | $J_1 - J_4$ |
| • prednisone (P) | 2 mg/kg/jour | orale | $J_1 - J_4$ |
| • interféron-alpha | 7 MU/m²/jour | s.c. | $J_1 - J_5$, et $J_{22} - J_{26}$ |

la cure comportant la répétion indéfinie de ce cycle, à raison de 1 cycle tous les 42 jours.

3.5.4 Protocole VCAP ou VBAP

d'après S.E. Salmon et al. (J. Clin. Oncol. 1983 ; 1 : 453-461) :

protocole VCAP :

| | dose | voie | jours |
|---|---|---|---|
| • 2-quinolone | 200-2000 mg/m²/jour <u>ou</u> 5 – 50 mg/kg/jour perfusion de 1 h | i.v. | $J_1 - J_5$ |
| • vincristine (V) | 1 mg/m² bolus (max : 1,5 mg) | i.v. | $J_1$ |
| • doxorubicine (A) | 30 mg/m² bolus | i.v. | $J_1$ |
| • prednisone (P) | 60 mg/m²/jour | orale | $J_1 - J_4$ |
| • cyclophosphamide (C) | 125 mg/m² perfusion de 1 heure | orale | $J_1 - J_4$ |

protocole VBAP : le cyclophosphamide est remplacé par la carmustine (BCNU), le reste étant identique :

| | dose | voie | jours |
|---|---|---|---|
| • carmustine | 30 mg/m² perfusion de 1 heure | i.v. | $J_1$ |

## C. TUMEURS DE L'ENFANT - Oncologie pédiatrique

Les isoflavones peuvent également être incorporés aux protocoles polychimiothérapeutiques de traitement des tumeurs pédiatriques afin d'améliorer l'efficacité antitumorale tout en réduisant la sévérité des effets secondaires grâce à l'action sur le recrutement et la mobilisation des cellules clonogènes et à la possibilité de réduire les doses actives.

### 1°/ Sarcome d'Ewing / Tumeur neuroectodermale primitive

Les 2-quinolones peuvent être introduites dans le protocole VCR-Doxo-CY -Ifos-Mesna-E (E. D. Berger et al., J. Clin. Oncol. 1990 ; 8 : 1514 - 1524 ; W.H. Meyer et al., J. Clin. Oncol. 1992 ; 10 : 1737 - 1742) :

| | dose | voie | jours |
|---|---|---|---|
| • 2-quinolone | 100-200 mg/m²/jour<br>ou 2 – 50 mg/kg/jour<br>perfusion de 1 heure | i.v. | $J_1$ - $J_5$ et<br>$J_{22}$ - $J_{27}$ et<br>$J_{43}$ - $J_{48}$ et<br>$J_{63}$ - $J_{68}$ et |
| • vincristine | 2 mg/m² bolus<br>(dose maximale = 2 mg) | i.v | $J_1$, $J_8$, $J_{15}$, $J_{43}$ |
| • doxorubicine | 30 mg/m²/jour<br>en perfusion de 24 heures | i.v. | $J_1$ - $J_3$,<br>$J_{43}$ - $J_{45}$ |
| • cyclophos-phamide | 2,2 g/m²<br>en perfusion de 0,5 heure | i.v. | $J_1$ , $J_{43}$ |
| • ifosfamide | 1800 mg/m²/jour<br>en perfusion de 1 heure | i.v. | $J_{22}$ - $J_{26}$<br>$J_{63}$ - $J_{67}$ |

| | | | |
|---|---|---|---|
| • mesna | 360 mg/m²<br>en perfusion de 15 minutes à raison de 5 doses toutes les 3 heures | i.v. | administré avec cyclophosphamide et ifosfamide |
| • étoposide | 100 mg/m²<br>en perfusion de 1 heure | i.v. | $J_{22}$ - $J_{26}$<br>$J_{63}$ - $J_{67}$ |

la cure comprend 6 à 10 de ces cycles en fonction de la sévérité initiale du sarcome et de l'amplitude de la réponse.

**2°/ Leucémie lymphoblastique aigue de l'enfant**

2.1. Chimiothérapie d'induction (jours $J_1$ - $J_{-30}$)

Les 2-quinolones peuvent être ajoutées aux protocoles recommandés (P.S. Gaynon et al., J. Clin. Oncol., 1993, 11, 2234-2242 ; J. Pullen et al., J. Clin. Oncol. 1993 ; 11 : 2234 -2242 ; J. Pullen et al., J. Clin. Oncol. 1993 ; 11 : 839 -849 ; V.J. Land et al., J. Clin. Oncol. 1994 ; 12 :1939 -1945) :

| | dose | voie | jours |
|---|---|---|---|
| • 2-quinolone | 100-200 mg/m²/jour<br>ou 2 – 50 mg/kg/jour<br>perfusion de 1 heure | i.v. | $J_1$ - $J_5$,<br>$J_8$-$J_{11}$, $J_{15}$ -$J_{18}$,<br>$J_{22}$ - $J_{27}$ |
| • vincristine | 1,5 mg/m² bolus<br>(dose maximale ≅ 2 mg) | i.v | $J_1$, $J_8$, $J_{15}$, $J_{22}$ |
| • L-asparaginase | 6000 IU/m² | i.m. | 3 fois/semaine<br>pendant 3 semaines |
| • prednisone | 60 mg/m²<br>en 3 doses/jour | orale | $J_1$ à $J_{28}$ |
| • daunorubicine | 25 mg/m²/jour<br>en perfusion de 15 minutes | i.v. | $J_1$, $J_8$, $J_{15}$ et $J_{22}$ |
| • méthotrexate | fonction de l'âge | intrathécale | $J_{15}$, $J_{28}$ |
| • cytarabine | fonction de l'âge | intrathécale | $J_1$ |

en fonction du résultat de l'examen de la moëlle osseuse, le passage à la phase de consolidation se fait le jour $J_{28}$ du protocole de traitement.

2.2. Chimiothérapie de consolidation / maintenance

Les 2-quinolones peuvent être introduites dans le protocole de maintenance (P.S. Gaynon et al., J. Clin. Oncol. 1993 ; 11 : 2234 -2242 ; J. Pullen et al., J. Clin. Oncol. 1993 ; 11 : 839 -849 ; V.J. Land et al., J. Clin. Oncol. 1994 ; 12 :1939 -1945) selon le schéma suivant :

| | dose | voie | jours |
|---|---|---|---|
| • 2-quinolone | 100-200 mg/m²/jour<br>ou 2 – 50 mg/kg/jour<br>perfusion de 1 heure | i.v. | $J_1$ - $J_5$, $J_{15}$ - $J_{20}$ et<br>$J_{94}$ - $J_{99}$, $J_{101}$ - $J_{106}$<br>$J_{108}$ - $J_{113}$, $J_{122}$ - $J_{127}$ |
| • cyclophosphamide | 1000 mg/m²<br>en perfusion de 0,5 heure | i.v | $J_1$, $J_{15}$, $J_{122}$ |
| • L-asparaginase | 6000 U/m² | i.m. | 3 fois/semaine<br>entre $J_{97}$ et $J_{122}$ |
| • cytarabine | 75 mg/m²/jour<br>en perfusion de 15<br>minutes | i.v./s.c. | une séquence de 4<br>jours démarrant<br>$J_2$, $J_9$, $J_{16}$ $J_{23}$, $J_{123}$, $J_1$ |
| • doxorubicine | 25 mg/m²/jour<br>en perfusion de 15<br>minutes | i.v. | $J_{94}$, $J_{101}$, $J_{108}$ |
| • mercaptopurine | 60 mg/m²/jour | orale | $J_1$-$J_{93}$, $J_{143}$ à fin de<br>traitement |
| • méthotrexate | 20 mg/m²/jour | orale | 1 fois/semaine entre<br>$J_{36}$ et $J_{72}$ et entre $J_{143}$<br>et la fin du traitement |
| • prednisone | 40 mg/m²/jour<br>(divisés en 3 doses/jour) | orale | 5 jours consécutifs<br>par mois entre $J_{143}$ et<br>la fin du traitement |

| | | | |
|---|---|---|---|
| • thioguanine | 60 mg/m²/jour | orale | $J_{122}$ - $J_{135}$ |
| • vincristine | 1,5 mg/m² bolus (dose maximale = 2 mg) | i.v | $J_{94}$, $J_{101}$, $J_{108}$, ensuite 1 fois/mois entre $J_{143}$ et la fin du traitement |
| • méthotrexate | fonction de l'âge | intra-thécale | $J_1$, $J_8$, $J_{15}$ $J_{22}$, $J_{123}$, $J_{130}$ puis 1 fois/3mois entre $J_{143}$ et la fin du traitement |

**3°/ Leucémie myéloïde aigue de l'enfant**

Les 2-quinolones sont ajoutées aux protocoles d'induction et de consolidation / maintenance selon les schémas suivants :

3.1. Chimiothérapie d'induction

D'après Y. Ravindranath et al., J. Clin. Oncol. 1991 ; 9 : 572 -580 ; M.E. Nesbit et al., J. Clin. Oncol. 1994 ; 12 : 127 - 135 ; RJ Wells et al., J. Clin. Oncol. 1994 ; 12 : 2367 - 2377) :

| | dose | voie | jours |
|---|---|---|---|
| • 2-quinolone | 100-200 mg/m²/jour<br>ou 2 – 50 mg/kg/jour<br>perfusion de 1 heure | i.v. | $J_1$ - $J_5$, $J_{10}$ - $J_{13}$ |
| • cytarabine | selon l'âge | intrathécale | $J_1$ |
| • daunorubicine | 20 mg/m²/jour<br>en perfusion de 24 heures | i.v. | $J_1$ - $J_4$, $J_{10}$ - $J_{13}$ |
| • cytarabine | 200 mg/m²/jour<br>en perfusion de 24 heures | i.v. | $J_1$ - $J_4$, $J_{10}$ - $J_{13}$ |
| • thioguanine | 100 mg/m²/jour<br>divisés en 2 doses/jour | orale | $J_1$ - $J_4$, $J_{10}$ - $J_{13}$ |
| • étoposide | 100 mg/m²/jour<br>en perfusion de 24 heures | i.v. | $J_1$ - $J_4$, $J_{10}$ - $J_{13}$ |
| • déxaméthasone | 6 mg/m²<br>divisés en 3 doses/jour | i.v./orale | $J_1$ - $J_4$, $J_{10}$ - $J_{13}$ |

ce cycle étant répété à partir de $J_{28}$.

3.2. Chimiothérapie de consolidation / maintenance

D'après Y. Ravidranath et al., J. Clin. Oncol. 1991 ; 9 : 572 -580 ; M.E. Nesbit et al., J. Clin. Oncol. 1994 ; 12 : 127 - 135 ; R.J. Wells et al, J. Clin. Oncol. 1994 ; 12 : 2367 - 2377) :

| | dose | voie | jours |
|---|---|---|---|
| • cytarabine | selon l'âge | intrathécale | $J_1$, $J_{28}$, $J_{56}$ |
| • 2-quinolone | 100-200 mg/m²/jour<br>ou 2 – 50 mg/kg/jour<br>perfusion de 1 heure | i.v. | $J_1$ - $J_5$, $J_8$ - $J_{13}$<br>$J_{28}$ - $J_{33}$, $J_{56}$ - $J_{51}$<br>$J_{89}$ - $J_{94}$ |
| • cytarabine | 3000 mg/m²<br>en perfusion de 3heures<br>toutes les 12 heures | i.v. | $J_1$ - $J_2$, et $J_8$ - $J_9$ |
| • L-asparaginase | 6000 IU/m²<br>3 heures après la cytarabine | i.m. | $J_2$, $J_9$ |
| • vincristine | 1,5 mg/m² bolus<br>(dose maximale = 2 mg) | i.v. | $J_{28}$, $J_{56}$ |
| • thioguanine | 75 mg/m²/jour | orale | $J_{28}$ - $J_{84}$ |
| • cytarabine | 75 mg/m²/jour<br>bolus | i.v. | $J_{28}$ – $J_{31}$, $J_{56}$ – $J_{59}$ |
| • cyclophosphamide | 75 mg/m²/jour<br>en perfusion de 0,5 heure | i.v. | $J_{28}$ – $J_{31}$, $J_{56}$ – $J_{59}$ |
| • cytarabine | 25 mg/m²/jour<br>bolus | sc/i.v. | $J_{89}$ - $J_{93}$ |
| • thioguanine | 50 mg/m²/jour | orale | $J_{89}$ - $J_{93}$ |

| | | | |
|---|---|---|---|
| • étoposide | 100 mg/m²/jour<br>en perfusion de 1 heure | i.v. | $J_{89}$ , $J_{92}$ |
| • dexaméthasone | 2 mg/m²/jour | orale | $J_{89}$ - $J_{92}$ |
| • daunorubicine | 30 mg/m²<br>en perfusion de 15 minutes | i.v. | $J_{89}$ |

**4°/ Maladie de Hodgkin de l'enfant**

Les 2-quinolones peuvent être ajoutées au protocole MOPP-ABVD selon EA Gehan et al. (Cancer 1990 ; 65 : 1429 - 1437), SP Hunger et al. (J. Clin. Oncol. 1994 ; 12 : 2160 - 2166) et MM Hudson et al. (J. Clin. Oncol. 1993 ; 11 : 100 - 108) :

| | dose | voie | jours |
|---|---|---|---|
| • 2-quinolone | 100-200 mg/m²/jour<br>ou 2 – 50 mg/kg/jour<br>perfusion de 1 heure | i.v. | $J_1$ – $J_5$ et $J_8$ – $J_{12}$ |
| • mechloréthamine (M) | 6 mg/m² bolus | i.v. | $J_1$, $J_8$ |
| • vincristine (O) | 1,5 mg/m² bolus<br>(maximum 2 mg) | i.v. | $J_1$, $J_8$ |
| • procarbazine (P) | 100 mg/m²/jour | orale | $J_1$ – $J_{14}$ |
| • prednisone (P) | 40 mg/m²/jour<br>(divisés en 3 doses/j) | orale | $J_1$ – $J_{14}$ |

| | | | |
|---|---|---|---|
| • doxorubicine (A) | 25 mg/m²/jour en perfusion de 15 minutes | i.v. | $J_{29}$, $J_{43}$ |
| • bléomycine (B) | 10 U/m² en perfusion de 15 minutes | i.v. | $J_{29}$, $J_{43}$ |
| • vinblastine (V) | 6 mg/m² bolus (maximum 2 mg) | i.v. | $J_{29}$, $J_{43}$ |
| • dacarbazine (D) | 375 mg/m² en perfusion de 15 minutes | i.v. | $J_{29}$, $J_{43}$ |

Ce cycle doit être répété 6 fois à raison de 1 cycle toutes les 8 semaines, la cure comportant 6 cycles.

Si une transplantation de moëlle osseuse autologue (autogreffe) est prescrite, le protocole CVB décrit par R. Chopra et al. (Blood 1993 ; 81 : 1137 - 1145), C. Wheeler et al. (J. Clin. Oncol. 1990 ; 8 : 648 - 656) et RJ Jones et al (J. Clin. Oncol. 1990, 8, 527-537) pourra être mis en oeuvre selon le schéma suivant (l'allogreffe ayant lieu le jour $J_0$) :

| | dose | voie | jours |
|---|---|---|---|
| • 2-quinolone | 100-200 mg/m²/jour<br>ou 2 – 50 mg/kg/jour<br>perfusion de 1 heure | i.v. | $J_{-7}$, $J_{-1}$ |
| • cyclophosphamide | 1800 mg/m²/jour<br>en 2 perfusions de 1 heure | i.v. | $J_{-7}$, $J_{-6}$<br>$J_{-5}$, $J_{-4}$ |
| • carmustine (BCNU) | 112 mg/m²/jour<br>en perfusion de 0,5 heure | i.v. | $J_{-7}$, $J_{-6}$<br>$J_{-5}$, $J_{-4}$ |
| • étoposide | 500 mg/m²/jour<br>en 2 perfusions de 1 heure | i.v. | $J_{-7}$, $J_{-6}$<br>$J_{-5}$, $J_{-4}$ |

**5°/ Lymphome lymphoblastique de l'enfant**

Les composés revendiqués pourront également être associés aux protocoles de chimiothérapie d'induction (A.T. Meadows et al., J. Clin. Oncol. 1989 ; 7 : 92 - 99 - C. Patte et al., Med. Ped. Oncol. 1992 ; 20 : 105 - 113 et A. Reiter et al., J. Clin. Oncol. 1995 ; 13 : 359 - 372) et de chimiothérapie de maintenance :

5.1 Chimiothérapie d'induction

| | dose | voie | jours |
|---|---|---|---|
| • 2-quinolone | 100-200 mg/m²/jour<br>ou 2 – 50 mg/kg/jour<br>perfusion de 1 heure | i.v. | $J_1 - J_5$, $J_{17} - J_{22}$, $J_{24} - J_{29}$ |
| • cyclophosphamide | 1200 mg/m²<br>en perfusion de 0,5 heure | i.v. | $J_1$ |
| • cytarabine | selon l'âge | intra-thécale | $J_1$ |
| • vincristine | 1,5 mg/m² bolus<br>(maximum 2 mg) | i.v. | $J_3$, $J_{10}$, $J_{17}$, $J_{24}$ |
| • prednisone | 60 mg/m²/jour<br>divisés en 3 doses/jour | orale | $J_3 - J_{28}$ |
| • daunorubicin | 60 mg/m²<br>en perfusion de 15 minutes | i.v. | $J_{17}$ |
| • L-asparaginase | 6000 U/m²/jour<br>en perfusion de 15 minutes | im | $J_{17} - J_{35}$<br>3 fois/semaine |
| • méthotrexate | selon l'âge | intra-thécale | $J_{17}$, $J_{31}$ |

5.2 Chimiothérapie de maintenance :

selon le schéma suivant :

| | dose | voie | jours |
|---|---|---|---|
| • 2-quinolone | 100-200 mg/m²/jour<br>ou 2 – 50 mg/kg/jour<br>perfusion de 1 heure | i.v. | $J_1 - J_5$, $J_{15} - J_{20}$, $J_{29} - J_{34}$ |
| • cyclophosphamide | 1000 mg/m²<br>en perfusion de 0,5 heure | i.v. | $J_1$ |
| • vincristine | 1,5 mg/m² bolus<br>(maximum 2 mg) | orale | $J_1$, $J_5$,<br>(des cycles 2 à 10) |
| • méthotrexate | 300 mg/m²/jour<br>(60% en perfusion de 15 minutes et 40% en perfusion de 4 heures) | i.v. | $J_{15}$ |
| • leucovorin | 10 mg/m²/toutes les 4 h | orale | $J_{16}$ |
| • daunorubicine | 30 mg/m²<br>en perfusion de 0,5 heure | i.v. | $J_{29}$ |
| • methotrexate | selon l'âge | intra-thécale | $J_1$, $J_8$, $J_{15}$<br>(cycle 1), puis<br>1 fois/mois<br>(cycles 2 à 10) |

la cure comportant 10 cycles

**6°/ Neuroblastome pédiatrique**

Le protocole de polychimiothérapie recommandé Doxo-E-Cy-Pt est adapté de R.P. Castleberry et al. (J. Clin.

Oncol. 1992 ; 10 : 1299 -1304), A. Garaventa et al. (J. Clin. Oncol. 1993 ; 11 : 1770 - 1779) et D.C. West et al. (J. Clin. Oncol. 1992 ; 11 : 84 - 90) :

L'évaluation de la réponse thérapeutique est faite après 9 semaines afin de décider de l'attitude : résection chirurgicale, radiothérapie ou nouvelle chimiothérapie.

| | dose | voie | jours |
|---|---|---|---|
| • 2-quinolone | 100-200 mg/m²/jour<br>ou 2 – 50 mg/kg/jour<br>perfusion de 1 heure | i.v. | $J_1 - J_5$,<br>$J_{28} - J_{35}$,<br>$J_{58} - J_{65}$ |
| • doxorubicine | 25 mg/m²/jour<br>en perfusion de 15 minutes | i.v. | $J_2$, $J_{30}$, $J_{58}$ |
| • étoposide | 100 mg/m²<br>en perfusion de 1 heure | orale/<br>nasogas-<br>trique | $J_2$, $J_5$, $J_{30}$, $J_{33}$,<br>$J_{58}$, $J_{61}$ |
| • cyclosphosphamide | 1000 mg/m²<br>en perfusion de 0,5 heure | i.v. | $J_3$, $J_4$, $J_{31}$, $J_{32}$, $J_{59}$,<br>$J_{60}$ |
| • cisplatine | 60 mg/m²<br>en perfusion de 6 heures | i.v. | $J_1$ , $J_{28}$, $J_{56}$ |

L' évaluation de la réponse thérapeutique est faite après 9 semaines afin de décider de l'attitude : résection chirurgicale, radiothérapie ou nouvelle chimiothérapie.

**7°/ Ostéosarcome pédiatrique**

Les 2-quinolones peuvent être ajoutés au protocole Doxo-Pt-Mtx-Lcv tel qu'il est décrit par M. Hudson et al. (J. Clin. Oncol. 1990 ; 8 : 1988 - 1997), PA Meyers (J. Clin. Oncol. 1992 ; 10 : 5 -15), et V.H.C. Bramwell et al. (J. Clin. Oncol. 1992 ; 10: 1579-1591):

| | dose | voie | jours |
|---|---|---|---|
| • 2-quinolone | 100-200 mg/m²/jour ou 2 – 50 mg/kg/jour perfusion de 1 heure | i.v. | $J_1 - J_5$, $J_{21} - J_{26}$, $J_{28} - J_{33}$ |
| • doxorubicine | 25 mg/m²/jour en perfusion de 24 heures | i.v. | $J_1$ - $J_3$ |
| • cisplatine | 120 mg/m² en perfusion de 6 heures | i.v. | $J_1$ |
| • methotrexate | 12 mg/m²/jour en perfusion de 1 heure | i.v. | $J_{21}$, $J_{28}$ |
| • leucovorin | 100 mg/m² toutes les 6 heures | orale | $J_{22}$, $J_{29}$ |

**8°/ Rhabdomyosarcome de l'enfant**

Le protocole Vcr-Dact-CY-Mesna (H. Maurer et al., Cancer 1993 ; 71 : 1904 - 1922 et LR Mandell et al., Oncology 1993 ; 7 : 71 - 83) peut inclure la perfusion i.v. des composés revendiqués selon le schéma suivant :

| | dose | voie | jours |
|---|---|---|---|
| • 2-quinolone | 100-200 mg/m²/jour ou 2 – 50 mg/kg/jour perfusion de 1 heure | i.v. | $J_1 - J_5$, $J_8 - J_{12}$, $J_{22} - J_{27}$, $J_{43} - J_{47}$ |
| • vincristine | 1,5 mg/m² bolus (max. 2 mg) | i.v. | $J_1$, $J_8$, $J_{15}$, $J_{22}$, $J_{29}$, $J_{36}$, $J_{43}$, $J_{50}$ et $J_{57}$ |
| • dactinomycin | 0,015 mg/kg bolus (dose journalière max : 0,5 mg) | i.v. | $J_1 - J_5$, $J_{22} - J_{27}$, $J_{43} - J_{47}$ |

| | | | |
|---|---|---|---|
| • cyclophosphamide | 2,2 g/m² en perfusion de 1 heure | i.v. | $J_1$, $J_{22}$, $J_{43}$ |
| • mesna | 360 mg/m² en perfusion de 1 heure toutes les 3 heures pour 5 doses | i.v. | $J_1$, $J_{22}$, $J_{43}$ |

A la fin de la 9ème semaine de traitement, l'efficacité doit être évaluée pour décider des suites (chirurgie, radiothérapie, poursuite de la chimiothérapie).

**9°/ Tumeur de Wilms chez l'enfant**

Dans le protocole Vcr - Dact tel qu'il est décrit par GJ D'Angio et al. (Cancer, 1989 ; 64 : 349 - 360) et DM Green et al. (J. Clin. Oncol. 1993 ; 11 : 91 - 95) :

| | dose | voie | jours |
|---|---|---|---|
| • 2-quinolone | 100-200 mg/m²/jour ou 2 – 50 mg/kg/jour perfusion de 1 heure | i.v. | $J_1 - J_5$, $J_8 - J_{12}$ puis chaque semaine |
| • vincristine | 2 mg/m² bolus (dose max : 2 mg) | i.v. | $J_7$ puis chaque semaine |
| • dactinomycine | 0,045 mg/kg bolus (P≤ 30 kg) 1,35 mg/m² (P>30 kg) (dose max : 3 mg) | i.v. | $J_1$, puis toutes les 3 semaines |

Ce protocole étant démarré après la résection chirurgicale.

En cas de transplantation de moëlle osseuse autologue (auto-greffe) selon A. Garaventar, et al. (Med. Pediatr. Oncol. 1994 ; 22 : 11 - 14), le protocole E-Thio-Cy pourra être modifié comme suit

| | dose | voie | jours |
|---|---|---|---|
| • 2-quinolone | 100-200 mg/m²/jour<br>ou 2 – 50 mg/kg/jour<br>perfusion de 1 heure | i.v. | $J_{-8} - J_{-1}$ |
| • étoposide | 1800 mg/m²<br>(perfusion de 24 heures) | i.v. | $J_{-8}$ |
| • thiotepa | 300 mg/m²/jour<br>en perfusion de 2 heures | i.v. | $J_{-7}$, $J_{-6}$, $J_{-5}$ |
| • cyclosphophamide | 50 mg/kg/jour<br>en perfusion de 1 heure | i.v. | $J_{-4}$, $J_{-3}$, $J_{-2}$, $J_{-1}$ |

la transplantation de moëlle osseuse ayant lieu à $J_0$.

**Revendications**

**1.** Utilisation d'un composé choisi parmi les composés de formule :

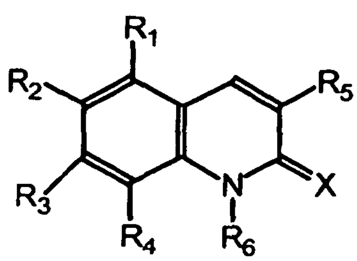

(I) et

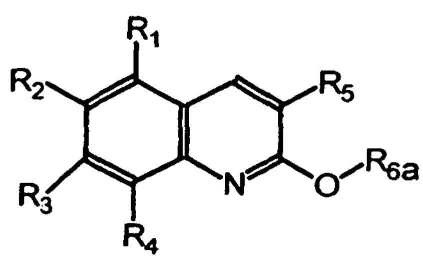

(Ia)

dans laquelle :

X est choisi parmi =O, =S et =N-NH-$R_7$, $R_7$ étant un groupe phényle ou pyridinyle,
$R_1$, $R_2$, $R_3$ et $R_4$ sont choisis indépendamment l'un de l'autre parmi H, OH, un groupe alkyle en $C_1$-$C_4$, un groupe alkoxy en $C_1$-$C_4$, un groupe -OCO-$R_8$, $R_8$ étant un groupe alkyle en $C_1$-$C_4$, au moins l'un des substituants $R_1$, $R_2$, $R_3$ ou $R_4$ étant autre que $H_1$ et $R_2$ et $R_3$ pouvant former ensemble un groupe méthylènedioxy,
$R_5$ est un groupe phényle ou un groupe phényle 1 à 3 fois substitué par des groupes choisis parmi H, OH, un groupe alkoxy en $C_1$-$C_4$, un groupe -OCO$R_8$, un groupe phényl(alkoxy en $C_1$-$C_4$), un groupe -O-$SO_2$-$R'_8$, $R'_8$ étant un groupe alkyle en $C_1$-$C_4$ ou un groupe $CF_3$,
$R_6$ est choisi parmi H, un groupe alkyle en $C_1$-$C_4$, un groupe -CO-$R_9$ et un groupe -A-$R_{10}$,
$R_{6a}$ est choisi parmi un groupe alkyle en $C_1$-$C_4$, un groupe -CO-$R_9$, et un groupe -A-$R_{10}$,
$R_9$ étant un groupe alkyle en $C_1$-$C_4$,

A étant un groupe alkylène en $C_1$-$C_4$,
$R_{10}$ étant choisi parmi les groupes hétérocycliques à 5 ou 6 chaînons ayant 1 à 4 hétéroatomes choisis parmi l'oxygène, le soufre et l'azote, le groupe CN, un groupe - $COOR_{11}$, -$CONR_{12}R_{13}$, un groupe -$NR_{14}R_{15}$, et un groupe -$COR_{16}$,
$R_{11}$, $R_{12}$, $R_{13}$, $R_{14}$, $R_{15}$ et $R_{16}$ étant indépendamment choisis parmi un atome d'hydrogène, un groupe alkyle en $C_1$-$C_4$ et un groupe phényl(alkyle en $C_1$-$C_4$),
$R_4$ et $R_6$ pouvant en outre former ensemble un groupe -CO-$CH_2$-$CH_2$-, pour la fabrication d'un médicament destiné à interférer avec la génération de cellules clonogènes dans les tumeurs lors d'un traitement de ces tumeurs par au moins un agent cytotoxique.

**2.** Utilisation selon la revendication 1, dans laquelle le composé est un composé de formule (I) dans laquelle:

- $R_1$ est un groupe alkoxy en $C_1$-$C_4$
- $R_2$ est un atome d'hydrogène
- $R_3$ est un groupe alkoxy en $C_1$-$C_4$
- $R_4$ est un atome d'hydrogène.

**3.** Utilisation selon la revendication 2, selon laquelle le composé est un composé de formule (I) dans laquelle :

- $R_5$ est un groupe 4-(alkoxy en $C_1$-$C_4$)phényle.

**4.** Utilisation selon la revendication 3, dans laquelle:

- $R_1$ est un groupe méthoxy,
- $R_3$ est un groupe méthoxy, et
- $R_5$ est un groupe 4-méthoxyphényle.

**5.** Utilisation selon la revendication 4 dans laquelle le composé est la 5,7-diméthoxy-3-(4-méthoxyphényl)-1,2-dihydro-2-quinolinone.

**6.** Utilisation selon la revendication 4 dans laquelle le composé est la 3-[5,7-diméthoxy-3-(4-méthoxyphényl)-2-oxo-1,2-dihydro-1-quinolinyl]propanenitrile.

**7.** Utilisation selon la revendication 4 dans laquelle le composé est la 1-[2-(1H-1,2,3,4-tétrazol-5-yl)éthyl]-5,7-diméthoxy-3-(4-méthoxyphényl)-1,2-dihydro-2-quinolinone.

**8.** Utilisation selon la revendication 4 dans laquelle le composé est le N,N-diéthyl-3-[5,7-diméthoxy-3-(4-méthoxyphényl)-2-oxo-1,2-dihydro-1-quinolinyl]-propanamide.

**9.** Composition pharmaceutique ayant une activité sur la prolifération de cellules clonogènes dans les tumeurs et qui comprend une quantité efficace d'un composé choisi parmi les composés de formule :

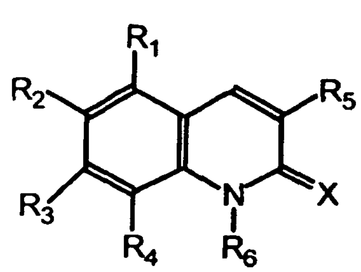
(I) et
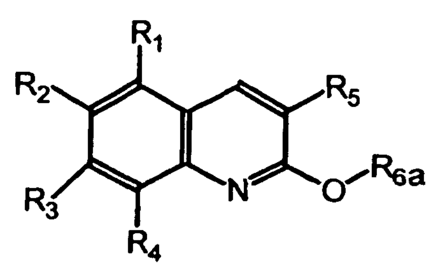
(Ia)

dans laquelle :

X est choisi parmi =O, =S et =N-NH-$R_7$, $R_7$ étant un groupe phényle ou pyridinyle, $R_1$, $R_2$, $R_3$ et $R_4$ sont choisis indépendamment l'un de l'autre parmi H, OH, un groupe alkyle en $C_1$-$C_4$, un groupe alkoxy en $C_1$-$C_4$, un

groupe -OCO-$R_8$, $R_8$ étant un groupe alkyle en $C_1$-$C_4$, au moins l'un des substituants $R_1$, $R_2$, $R_3$ ou $R_4$ étant autre que H, et $R_2$ et $R_3$ pouvant former ensemble un groupe méthylènedioxy,
$R_5$ est un groupe phényle ou un groupe phényle 1 à 3 fois substitué par des groupes choisis parmi H, OH, un groupe alkoxy en $C_1$-$C_4$, un groupe -$OCOR_8$, un groupe phényl(alkoxy en $C_1$-$C_4$), un groupe -O-$SO_2$-$R'_8$, $R'_8$ étant un groupe alkyle en $C_1$-$C_4$ ou un groupe $CF_3$,
$R_6$ est choisi parmi H, un groupe alkyle en $C_1$-$C_4$, un groupe -CO-$R_9$ et un groupe -A-$R_{10}$,
$R_{6a}$ est choisi parmi un groupe -CO-$R_9$, et un groupe -A-$R_{10}$,
$R_9$ étant un groupe alkyle en $C_1$-$C_4$,
A étant un groupe alkylène en $C_1$-$C_4$,
$R_{10}$ étant choisi parmi les groupes hétérocycliques à 5 ou 6 chaînons ayant 1 à 4 hétéroatomes choisis parmi l'oxygène, le soufre et l'azote, le groupe CN, un groupe - $COOR_{11}$, -$CONR_{12}R_{13}$, un groupe -$NR_{14}R_{15}$, et un groupe -$COR_{16}$,
$R_{11}$, $R_{12}$, $R_{13}$, $R_{14}$, $R_{15}$ et $R_{16}$ étant indépendamment choisis parmi un atome d'hydrogène, un groupe alkyle en $C_1$-$C_4$ et un groupe phényl(alkyle en $C_1$-$C_4$),
$R_4$ et $R_6$ pouvant en outre former ensemble un groupe -CO-$CH_2$-$CH_2$-.

**10.** Composition selon la revendication 9, dans laquelle le composé est un composé de formule (I) dans laquelle :

- $R_1$ est un groupe alkoxy en $C_1$-$C_4$
- $R_2$ est un atome d'hydrogène
- $R_3$ est un groupe alkoxy en $C_1$-$C_4$
- $R_4$ est un atome d'hydrogène.

**11.** Composition selon la revendication 10, dans laquelle le composé est un composé de formule (I) dans laquelle :

- $R_5$ est un groupe 4-(alkoxy en $C_1$-$C_4$)phényle.

**12.** Composition selon la revendication 11, dans laquelle le composé est un composé de formule (I), $R_1$ est un groupe méthoxy, $R_3$ est un groupe méthoxy et $R_5$ est un groupe 4-méthoxyphényle.

**13.** Composition selon la revendication 12 dans laquelle le composé est la 5,7-diméthoxy-3-(4-méthoxyphényl)-1,2-dihydro-2-quinolinone.

**14.** Composition selon la revendication 12 dans laquelle le composé est le 3-[5,7-diméthoxy-3-(4-méthoxyphényl)-2-oxo-1,2-dihydro-1-quinolinyl]propanenitrile.

**15.** Composition selon la revendication 12 dans laquelle le composé est la 1-[2-(1*H*-1,2,3,4-tétrazol-5-yl)éthyl]-5,7-diméthoxy-3-(4-méthoxyphényl)-1,2-dihydro-2-quinolinone.

**16.** Composition selon la revendication 12 dans laquelle le composé est le *N,N*-Diéthyl-3-[5,7-diméthoxy-3-(4-méthoxyphényl)-2-oxo-1,2-dihydro-1-quinolinyl]-propanamide.

**17.** Composés de formule :

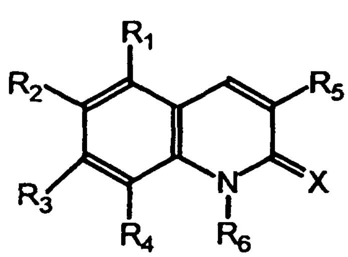

(I) et

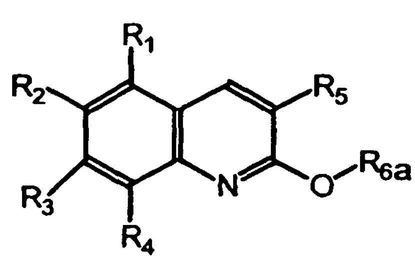

(Ia)

dans laquelle :

X est choisi parmi =O, =S et =N-NH-$R_7$, $R_7$ étant un groupe phényle ou pyridinyle, $R_1$, $R_2$, $R_3$ et $R_4$ sont choisis

indépendamment l'un de l'autre parmi H, OH, un groupe alkyl en $C_1$-$C_4$, un groupe alkoxy en $C_1$-$C_4$, un groupe -OCO-$R_8$, $R_8$ étant un groupe alkyle en $C_1$-$C_4$, au moins l'un des substituants $R_1$, $R_2$, $R_3$ ou $R_4$ étant autre que H, et $R_2$ et $R_3$ pouvant former ensemble un groupe méthylènedioxy,
$R_5$ est un groupe phényle 1 à 3 fois substitué par des groupes choisis parmi OH, un groupe alkoxy en $C_1$-$C_4$, un groupe -OCOR$_8$, un groupe phényl(alkoxy en $C_1$-$C_4$), un groupe -O-$SO_2$-R'$_8$, R'$_8$ étant un groupe alkyle en $C_1$-$C_4$ ou un groupe $CF_3$,
$R_6$ est choisi parmi un groupe alkyle en $C_1$-$C_4$, un groupe -CO-$R_9$ et un groupe -A-$R_{10}$.
$R_{6a}$ est choisi parmi un groupe -CO-$R_9$, et un groupe A-$R_{10}$,
$R_9$ étant un groupe alkyle en $C_1$-$C_4$,
A étant un groupe alkylène en $C_1$-$C_4$,
$R_{10}$ étant choisi parmi les groupes hétérocycliques à 5 ou 6 chaînons ayant 1 à 4 hétéroatomes choisis parmi l'oxygène, le soufre et l'azote, le groupe CN, un groupe - COOR$_{11}$, -CONR$_{12}$R$_{13}$, un groupe -NR$_{14}$R$_{15}$, et un groupe -COR$_{16}$,
$R_{11}$, $R_{12}$, $R_{13}$, $R_{14}$, $R_{15}$ et $R_{16}$ étant indépendamment choisis parmi un atome d'hydrogène, un groupe alkyle en $C_1$-$C_4$ et un groupe phényl(alkyle en $C_1$-$C_4$),
$R_4$ et $R_6$ pouvant en outre former ensemble un groupe -CO-$CH_2$-$CH_2$-,

**18.** Composé selon la revendication 17 qui est le 3-[5,7-diméthoxy-3-(4-méthoxyphényl)-2-oxo-1,2-dihydro-1-quinolinyl]propanenitrile.

**19.** Composé selon la revendication 17 qui est la 1-[2-(1*H*-1,2,3,4-tétrazol-5-yl)éthyl]-5,7-diméthoxy-3-(4-méthoxyphényl)-1,2-dihydro-2-quinolinone.

**20.** Composé selon la revendication 17 qui est le *N*,*N*-diéthyl-3-[5,7-diméthoxy-3-(4-méthoxyphényl)-2-oxo-1,2-dihydro-1-quinolinyl]propanamide.

**21.** Composés de formule :

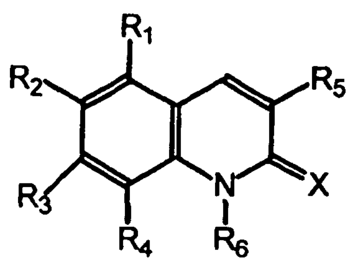

**(I)**

dans laquelle :

X est choisi parmi =O, =S et =N-NH-$R_7$, $R_7$ étant un groupe phényle ou pyridinyle, $R_1$, $R_2$, $R_3$ et $R_4$ sont choisis indépendamment l'un de l'autre parmi H, OH, un groupe alkyl en $C_1$-$C_4$, un groupe alkoxy en $C_1$-$C_4$, un groupe -OCO-$R_8$, $R_8$ étant un groupe alkyle en $C_1$-$C_4$, au moins l'un des substituants $R_1$, $R_2$, $R_3$ ou $R_4$ étant autre que H, et $R_2$ et $R_3$ pouvant former ensemble un groupe méthylènedioxy,
$R_5$ est un groupe phényle ou un groupe phényle 1 à 3 fois substitué par des groupes choisis parmi H, OH, un groupe alkoxy en $C_1$-$C_4$, un groupe -OCOR$_8$, un groupe phényl(alkoxy en $C_1$-$C_4$), un groupe -O-$SO_2$-R'$_8$, R'$_8$ étant un groupe alkyle en $C_1$-$C_4$ ou un groupe $CF_3$,
$R_6$ est choisi parmi saveun groupe -A-$R_{10}$,
A étant un groupe alkylène en $C_1$-$C_4$,
$R_{10}$ étant choisi parmi les groupes hétérocycliques à 5 ou 6 chaînons ayant 1 à 4 hétéroatomes choisis parmi l'oxygène, le soufre et l'azote, le groupe CN, un groupe - COOR$_{11}$, -CONR$_{12}$R$_{13}$, un groupe -NR$_{14}$R$_{15}$, et un groupe -COR$_{16}$,
$R_{11}$, $R_{12}$, $R_{13}$, $R_{14}$, $R_{15}$ et $R_{16}$ étant indépendamment choisis parmi un atome d'hydrogène, un groupe alkyle en $C_1$-$C_4$ et un groupe phényl(alkyle en $C_1$-$C_4$).

**22.** Composés de formule :

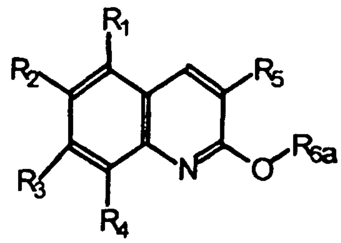

(Ia)

dans laquelle :

$R_1$, $R_2$, $R_3$ et $R_4$ sont choisis indépendamment l'un de l'autre parmi H, OH, un groupe alkyl en $C_1$-$C_4$, un groupe alkoxy en $C_1$-$C_4$, un groupe -OCO-$R_8$, $R_8$ étant un groupe alkyle en $C_1$-$C_4$, au moins l'un des substituants $R_1$, $R_2$, $R_3$ ou $R_4$ étant autre que H, et $R_2$ et $R_3$ pouvant former ensemble un groupe méthylènedioxy,
$R_5$ est un groupe phényle ou un groupe phényle 1 à 3 fois substitué par des groupes choisis parmi H, OH, un groupe alkoxy en $C_1$-$C_4$, un groupe -OCO$R_8$, un groupe phényl(alkoxy en $C_1$-$C_4$), un groupe -O-$SO_2$-$R'_8$, $R'_8$ étant un groupe alkyle en $C_1$-$C_4$ ou un groupe $CF_3$,
$R_{6a}$ est choisi parmi un groupe -CO-$R_9$, et un groupe -A-$R_{10}$,
$R_9$ étant un groupe alkyle en $C_1$-$C_4$,
A étant un groupe alkylène en $C_1$-$C_4$,
$R_{10}$ étant choisi parmi les groupes hétérocycliques à 5 ou 6 chaînons ayant 1 à 4 hétéroatomes choisis parmi l'oxygène, le soufre et l'azote, le groupe CN, un groupe - COO$R_{11}$, -CON$R_{12}R_{13}$, un groupe -N$R_{14}R_{15}$, et un groupe -CO$R_{16}$,
$R_{11}$, $R_{12}$, $R_{13}$, $R_{14}$, $R_{15}$ et $R_{16}$ étant indépendamment choisis parmi un atome d'hydrogène, un groupe alkyle en $C_1$-$C_4$ et un groupe phényl(alkyle en $C_1$-$C_4$).

**23.** Composé selon la revendication 17, **caractérisé en ce que** $R_6$ est choisi parmi un groupe alkyle en $C_1$-$C_4$, un groupe -CO-$R_9$ et un groupe A-$R_{10}$,

$R_9$ étant tel que définis dans la revendication 17,
$R_{10}$ étant choisi parmi les groupes hétérocycliques à 5 ou 6 chaînons ayant 1 à 4 hétéroatomes choisis parmi l'oxygène, le soufre et l'azote, le groupe CN, un groupe - COO$R_{11}$, -CON$R_{12}R_{13}$, un groupe -N$R_{14}R_{15}$, et un groupe -CO$R_{16}$,
$R_{11}$ étant un groupe phényl(alkyle en $C_1$-$C_4$),
$R_{12}$, $R_{13}$, $R_{14}$, $R_{15}$ et $R_{16}$ étant indépendamment choisis parmi un atome d'hydrogène, un groupe alkyle en $C_1$-$C_4$ et un groupe phényl(alkyle en $C_1$-$C_4$).

**24.** Composition pharmaceutique ayant une activité sur la prolifération de cellules clonogènes dans les tumeurs et qui comprend une quantité efficace d'un composé choisi parmi les composés selon l'une quelconque des revendications 17 et 23.

**Patentansprüche**

**1.** Verwendung einer Verbindung ausgewählt aus den Verbindungen der Formeln:

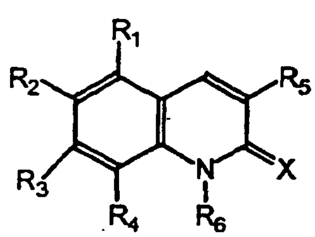

(I) und

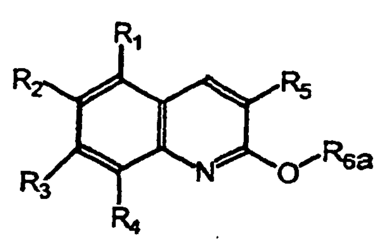

(Ia)

in denen:

X ausgewählt ist aus =O, =S und =N-NH-$R_7$, worin $R_7$ eine Phenyl- oder Pyridinylgruppe bedeutet,
$R_1$, $R_2$, $R_3$ und $R_4$ unabhängig voneinander ausgewählt sind aus H, OH, einer $C_1$-$C_4$-Alkylgruppe, einer $C_1$-$C_4$-Alkoxygruppe, einer Gruppe -OCO-$R_8$, worin $R_8$ eine $C_1$-$C_4$-Alkylgruppe bedeutet, wobei mindestens einer der Substituenten $R_1$, $R_2$, $R_3$ oder $R_4$ von H verschieden ist und $R_2$ und $R_3$ gemeinsam eine Methylendioxygruppe bilden können,
$R_5$ eine Phenylgruppe oder eine Phenylgruppe, die 1- bis 3-mal mit Gruppen ausgewählt aus H, OH, einer $C_1$-$C_4$-Alkoxygruppe, einer Gruppe -OCOR$_8$, einer Phenyl-($C_1$-$C_4$-alkoxy)-gruppe, einer Gruppe -O-$SO_2$-$R'_8$, worin $R'_8$ eine $C_1$-$C_4$-Alkylgruppe darstellt, oder einer $CF_3$-Gruppe substituiert ist, bedeutet,
$R_6$ ausgewählt ist aus H, einer $C_1$-$C_4$-Alkylgruppe, einer Gruppe -CO-$R_9$ und einer Gruppe -A-$R_{10}$,
$R_{6a}$ ausgewählt ist aus einer $C_1$-$C_4$-Alkylgruppe, einer Gruppe -CO-$R_9$ und einer Gruppe -A-$R_{10}$.
$R_9$ eine $C_1$-$C_4$-Alkylgruppe bedeutet,
A eine $C_1$-$C_4$-Alkylengruppe bedeutet,
$R_{10}$ ausgewählt ist aus heterocyclischen Gruppen mit 5 oder 6 Kettengliedern und 1 bis 4 Heteroatomen ausgewählt aus Sauerstoff, Schwefel und Stickstoff, der Gruppe CN, einer Gruppe -COOR$_{11}$, -CONR$_{12}$R$_{13}$, einer Gruppe -NR$_{14}$R$_{15}$ und einer Gruppe -COR$_{16}$,
$R_{11}$, $R_{12}$, $R_{13}$, $R_{14}$, $R_{15}$ und $R_{16}$ unabhängig voneinander ausgewählt sind aus einem Wasserstoffatom, einer $C_1$-$C_4$-Alkylgruppe und einer Phenyl-($C_1$-$C_4$-alkyl)-gruppe, wobei
$R_4$ und $R_6$ weiterhin gemeinsam eine Gruppe -CO-$CH_2$-$CH_2$- bilden können, für die Herstellung eines Arzneimittels zur Beeinflussung der Erzeugung von clonogenen Zellen in Tumoren bei einer Behandlung dieser Tumore mit mindestens einem cytotoxischen Mittel.

**2.** Verwendung nach Anspruch 1, worin die Verbindung eine Verbindung der Formel (I) ist, in der:

- $R_1$ eine $C_1$-$C_4$-Alkoxygruppe bedeutet,
- $R_2$ ein Wasserstoffatom bedeutet,
- $R_3$ eine $C_1$-$C_4$-Alkoxygruppe bedeutet und
- $R_4$ ein Wasserstoffatom bedeutet.

**3.** Verwendung nach Anspruch 2, worin die Verbindung eine Verbindung der Formel (I) ist, in der:

- $R_5$ eine 4-($C_1$-$C_4$-Alkoxy)-phenylgruppe ist.

**4.** Verwendung nach Anspruch 3, worin:

- $R_1$ eine Methoxygruppe bedeutet,
- $R_3$ eine Methoxygruppe bedeutet und
- $R_5$ eine 4-Methoxyphenylgruppe bedeutet.

**5.** Verwendung nach Anspruch 4, worin die Verbindung 5,7-Dimethoxy-3-(4-methoxyphenyl)-1,2-dihydro-2-chinolinon ist.

**6.** Verwendung nach Anspruch 4, worin die Verbindung 3-[5,7-Dimethoxy-3-(4-methoxyphenyl)-2-oxo-1,2-dihydro-1-chinolinyl]-propannitril ist.

**7.** Verwendung nach Anspruch 4, worin die Verbindung 1-[2-(1H-1,2,3.4-Tetrazol-5-yl)-ethyl]-5,7-dimethoxy-3-(4-methoxyphenyl)-1,2-dihydro-2-chinolinon ist.

**8.** Verwendung nach Anspruch 4, worin die Verbindung N,N-Diethyl-3-[5,7-dimethoxy-3-(4-methoxyphenyl)-2-oxo-1,2-dihydro-1-chinolinyl]-propanamid ist.

**9.** Pharmazeutische Zubereitung mit einer Wirkung gegen die Proliferation von clonogenen Zellen in Tumoren und die eine wirksame Menge einer Verbindung enthält, ausgewählt aus den Verbindungen der Formeln:

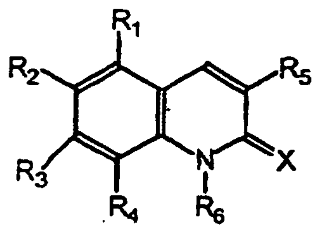 (I) und 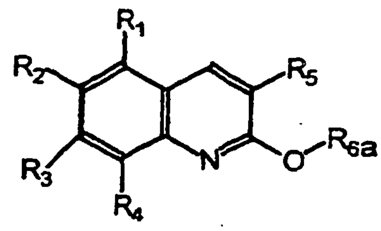 (Ia)

in denen:

X ausgewählt ist aus =O, =S und =N-NH-$R_7$, worin $R_7$ eine Phenyl- oder Pyridinylgruppe bedeutet,
$R_1$, $R_2$, $R_3$ und $R_4$ unabhängig voneinander ausgewählt sind aus H, OH, einer $C_1$-$C_4$-Alkylgruppe, einer $C_1$-$C_4$-Alkoxygruppe, einer Gruppe -OCO-$R_8$, worin $R_8$ eine $C_1$-$C_4$-Alkylgruppe bedeutet, wobei mindestens einer der Substituenten $R_1$, $R_2$, $R_3$ oder $R_4$ von H verschieden ist und $R_2$ und $R_3$ gemeinsam eine Methylendioxygruppe bilden können,
$R_5$ eine Phenylgruppe oder eine Phenylgruppe, die 1- bis 3-mal mit Gruppen ausgewählt aus H, OH, einer $C_1$-$C_4$-Alkoxygruppe, einer Gruppe -OCO$R_8$, einer Phenyl-($C_1$-$C_4$-alkoxy)-gruppe, einer Gruppe -O-$SO_2$-$R'_8$, worin $R'_8$ eine $C_1$-$C_4$-Alkylgruppe darstellt, oder einer $CF_3$-Gruppe substituiert ist, bedeutet,
$R_6$ ausgewählt ist aus H, einer $C_1$-$C_4$-Alkylgruppe, einer Gruppe -CO-$R_9$ und einer Gruppe -A-$R_{10}$,
$R_{6a}$ ausgewählt ist aus einer Gruppe -CO-$R_9$ und einer Gruppe -A-$R_{10}$,
$R_9$ eine $C_1$-$C_4$-Alkylgruppe bedeutet,
A eine $C_1$-$C_4$-Alkylengruppe bedeutet,
$R_{10}$ ausgewählt ist aus heterocyclischen Gruppen mit 5 oder 6 Kettengliedern und 1 bis 4 Heteroatomen ausgewählt aus Sauerstoff, Schwefel und Stickstoff, der Gruppe CN, einer Gruppe -COO$R_{11}$, -CON$R_{12}R_{13}$, einer Gruppe -N$R_{14}R_{15}$ und einer Gruppe -CO$R_{16}$,
$R_{11}$, $R_{12}$, $R_{13}$, $R_{14}$, $R_{15}$ und $R_{16}$ unabhängig voneinander ausgewählt sind aus einem Wasserstoffatom, einer $C_1$-$C_4$-Alkylgruppe und einer Phenyl-($C_1$-$C_4$-alkyl)-gruppe, und
$R_4$ und $R_6$ weiterhin gemeinsam eine Gruppe -CO-$CH_2$-$CH_2$- bilden können.

**10.** Zubereitung nach Anspruch 9, worin die Verbindung eine Verbindung der Formel (I) ist, in der:

- $R_1$ eine $C_1$-$C_4$-Alkoxygruppe bedeutet,
- $R_2$ ein Wasserstoffatom bedeutet,
- $R_3$ eine $C_1$-$C_4$-Alkoxygruppe bedeutet und
- $R_4$ ein Wasserstoffatom bedeutet.

**11.** Zubereitung nach Anspruch 10, worin die Verbindung eine Verbindung der Formel (I) ist, in der:

- $R_5$ eine 4-($C_1$-$C_4$-Alkoxy)-phenylgruppe ist.

**12.** Zubereitung nach Anspruch 11, worin die Verbindung eine Verbindung der Formel (I) ist, in der $R_1$ eine Methoxygruppe, $R_3$ eine Methoxygruppe und $R_5$ eine 4-Methoxyphenylgruppe bedeuten.

**13.** Zubereitung nach Anspruch 12, worin die Verbindung 5,7-Dimethoxy-3-(4-methoxyphenyl)-1, 2-dihydro-2-chinolinon ist.

**14.** Zubereitung nach Anspruch 12, worin die Verbindung 3-[5,7-Dimethoxy-3-(4-methoxyphenyl)-2-oxo-1,2-dihydro-1-chinolinyl]-propannitril ist.

**15.** Zubereitung nach Anspruch 12, worin die Verbindung 1-[2-(1*H*-1,2,3.4-Tetrazol-5-yl)-ethyl]-5,7-dimethoxy-3-(4-methoxyphenyl)-1,2-dihydro-2-chinolinon ist.

**16.** Zubereitung nach Anspruch 12, worin die Verbindung *N*,*N*-Diethyl-3-[5,7-dimethoxy-3-(4-methoxyphenyl)-2-oxo-1,2-dihydro-1-chinolinyl]-propanamid ist.

**17.** Verbindungen der Formeln:

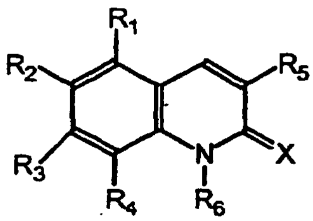 (I) und 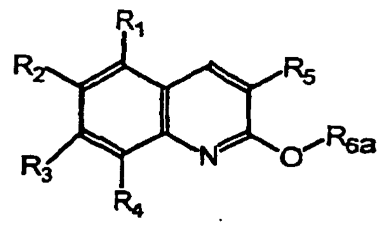 (Ia)

in denen:

X ausgewählt ist aus =O, =S und =N-NH-$R_7$, worin $R_7$ eine Phenyl- oder Pyridinylgruppe bedeutet,
$R_1$, $R_2$, $R_3$ und $R_4$ unabhängig voneinander ausgewählt sind aus H, OH, einer $C_1$-$C_4$-Alkylgruppe, einer $C_1$-$C_4$-Alkoxygruppe, einer Gruppe -OCO-$R_8$, worin $R_8$ eine $C_1$-$C_4$-Alkylgruppe bedeutet, wobei mindestens einer der Substituenten $R_1$, $R_2$, $R_3$ oder $R_4$ von H verschieden ist und $R_2$ und $R_3$ gemeinsam eine Methylendioxygruppe bilden können,
$R_5$ eine Phenylgruppe bedeutet, die 1- bis 3-mal mit Gruppen ausgewählt aus OH, einer $C_1$-$C_4$-Alkoxygruppe, einer Gruppe -OCO$R_8$, einer Phenyl-($C_1$-$C_4$-alkoxy)-gruppe, einer Gruppe -O-$SO_2$-$R'_8$, worin $R'_8$ eine $C_1$-$C_4$-Alkylgruppe darstellt, oder einer $CF_3$-Gruppe substituiert ist,
$R_6$ ausgewählt ist aus einer $C_1$-$C_4$-Alkylgruppe, einer Gruppe -CO-$R_9$ und einer Gruppe -A-$R_{10}$.
$R_{6a}$ ausgewählt ist aus einer Gruppe -CO-$R_9$ und einer Gruppe A-$R_{10}$,
$R_9$ eine $C_1$-$C_4$-Alkylgruppe bedeutet,
A eine $C_1$-$C_4$-Alkylengruppe bedeutet,
$R_{10}$ ausgewählt ist aus heterocyclischen Gruppen mit 5 oder 6 Kettengliedern und 1 bis 4 Heteroatomen ausgewählt aus Sauerstoff, Schwefel und Stickstoff, der Gruppe CN, einer Gruppe -COO$R_{11}$, -CON$R_{12}R_{13}$, einer Gruppe -N$R_{14}R_{15}$ und einer Gruppe -CO$R_{16}$, und
$R_{11}$, $R_{12}$, $R_{13}$, $R_{14}$, $R_{15}$ und $R_{16}$ unabhängig voneinander ausgewählt sind aus einem Wasserstoffatom, einer $C_1$-$C_4$-Alkylgruppe und einer Phenyl-($C_1$-$C_4$-alkyl)-gruppe, wobei
$R_4$ und $R_6$ weiterhin gemeinsam eine Gruppe -CO-$CH_2$-$CH_2$- bilden können.

**18.** Verbindung nach Anspruch 17, nämlich 3-[5,7-Dimethoxy-3-(4-methoxyphenyl)-2-oxo-1, 2-dihydro-1-chinolinyl]-propannitril.

**19.** Verbindung nach Anspruch 17, nämlich 1-[2-(1H 1,2,3,4-Tetrazol-5-yl)-ethyl]-5,7-dimethoxy-3-(4-methoxyphenyl)-1,2-dihydro-2-chinolinon.

**20.** Verbindung nach Anspruch 17, nämlich *N*,*N*-Diethyl-3-[5,7-dimethoxy-3-(4-methoxyphenyl)-2-oxo-1,2-dihydro-1-chinolinyl]-propanamid.

**21.** Verbindungen der Formel:

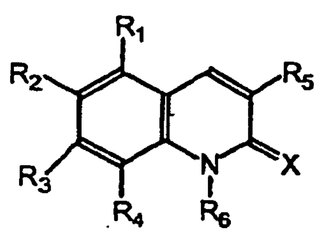 (I)

in der:

X ausgewählt ist aus =O, =S und =N-NH-$R_7$, worin $R_7$ eine Phenyl- oder Pyridinylgruppe bedeutet,
Ri, $R_2$, $R_3$ und $R_4$ unabhängig voneinander ausgewählt sind aus H, OH, einer $C_1$-$C_4$-Alkylgruppe, einer $C_1$-$C_4$-Alkoxygruppe, einer Gruppe -OCO-$R_8$, worin $R_8$ eine $C_1$-$C_4$-Alkylgruppe bedeutet, wobei mindestens einer der Substituenten $R_1$, $R_2$, $R_3$ oder $R_4$ von H verschieden ist und $R_2$ und $R_3$ gemeinsam eine Methylendioxy-

gruppe bilden können,
$R_5$ eine Phenylgruppe oder eine Phenylgruppe, die 1- bis 3-mal mit Gruppen ausgewählt aus H, OH, einer $C_1$-$C_4$-Alkoxygruppe, einer Gruppe -$OCOR_8$, einer Phenyl-($C_1$-$C_4$-alkoxy)-gruppe, einer Gruppe -O-$SO_2$-$R'_8$, worin $R'_8$ eine $C_1$-$C_4$-Alkylgruppe darstellt, oder einer $CF_3$-Gruppe substituiert ist, bedeutet,
$R_6$ ausgewählt ist aus einer Gruppe -A-$R_{10}$,
A eine $C_1$-$C_4$-Alkylengruppe bedeutet,
$R_{10}$ ausgewählt ist aus heterocyclischen Gruppen mit 5 oder 6 Kettengliedern und 1 bis 4 Heteroatomen ausgewählt aus Sauerstoff, Schwefel und Stickstoff, der Gruppe CN, einer Gruppe -$COOR_{11}$, -$CONR_{12}R_{13}$, einer Gruppe -$NR_{14}R_{15}$ und einer Gruppe -$COR_{16}$, und
$R_{11}$, $R_{12}$, $R_{13}$, $R_{14}$, $R_{15}$ und $R_{16}$ unabhängig voneinander ausgewählt sind aus einem Wasserstoffatom, einer $C_1$-$C_4$-Alkylgruppe und einer Phenyl-($C_1$-$C_4$-alkyl)-gruppe.

**22.** Verbindungen der Formel:

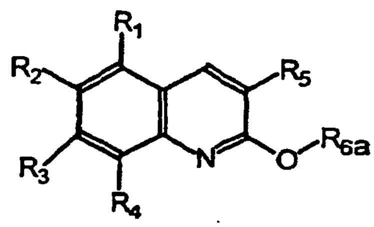

(Ia)

in der:

$R_1$, $R_2$, $R_3$ und $R_4$ unabhängig voneinander ausgewählt sind aus H, OH, einer $C_1$-$C_4$-Alkylgruppe, einer $C_1$-$C_4$-Alkoxygruppe, einer Gruppe -OCO-$R_8$, worin $R_8$ eine $C_1$-$C_4$-Alkylgruppe bedeutet, wobei mindestens einer der Substituenten $R_1$, $R_2$, $R_3$ oder $R_4$ von H verschieden ist und $R_2$ und $R_3$ gemeinsam eine Methylendioxygruppe bilden können,
$R_5$ eine Phenylgruppe oder eine Phenylgruppe, die 1- bis 3-mal mit Gruppen ausgewählt aus H, OH, einer $C_1$-$C_4$-Alkoxygruppe, einer Gruppe -$OCOR_8$, einer Phenyl-($C_1$-$C_4$-alkoxy)-gruppe, einer Gruppe -O-$SO_2$-$R'_8$, worin $R'_8$ eine $C_1$-$C_4$-Alkylgruppe darstellt, oder einer $CF_3$-Gruppe substituiert ist, bedeutet,
$R_{6a}$ ausgewählt ist aus einer Gruppe -CO-$R_9$ und einer Gruppe -A-$R_{10}$,
$R_9$ eine $C_1$-$C_4$-Alkylgruppe bedeutet,
A eine $C_1$-$C_4$-Alkylengruppe bedeutet,
$R_{10}$ ausgewählt ist aus heterocyclischen Gruppen mit 5 oder 6 Kettengliedern und 1 bis 4 Heteroatomen ausgewählt aus Sauerstoff, Schwefel und Stickstoff, der Gruppe CN, einer Gruppe -$COOR_{11}$, -$CONR_{12}R_{13}$, einer Gruppe -$NR_{14}R_{15}$ und einer Gruppe -$COR_{16}$, und
$R_{11}$, $R_{12}$, $R_{13}$, $R_{14}$, $R_{15}$ und $R_{16}$ unabhängig voneinander ausgewählt sind aus einem Wasserstoffatom, einer $C_1$-$C_4$-Alkylgruppe und einer Phenyl-($C_1$-$C_4$-alkyl)-gruppe.

**23.** Verbindung nach Anspruch 17, **dadurch gekennzeichnet, daß** $R_6$ ausgewählt ist aus einer $C_1$-$C_4$-Alkylgruppe, einer Gruppe -CO-$R_9$ und einer Gruppe -A-$R_{10}$,

$R_9$ die in Anspruch 17 angegebenen Bedeutungen besitzt,
$R_{10}$ ausgewählt ist aus heterocyclischen Gruppen mit 5 oder 6 Kettengliedern und 1 bis 4 Heteroatomen ausgewählt aus Sauerstoff, Schwefel und Stickstoff. der Gruppe CN, einer Gruppe -$COOR_{11}$, -$CONR_{12}R_{13}$, einer Gruppe -$NR_{14}R_{15}$ und einer Gruppe -$COR_{16}$,
$R_{11}$ eine Phenyl-($C_1$-$C_4$-alkyl)-gruppe bedeutet, und
$R_{12}$, $R_{13}$, $R_{14}$, $R_{15}$ und $R_{16}$ unabhängig voneinander ausgewählt sind aus einem Wasserstoffatom, einer $C_1$-$C_4$-Alkylgruppe und einer Phenyl($C_1$-$C_4$-alkyl)-gruppe.

**24.** Pharmazeutische Zubereitung, die eine Wirkung auf die Proliferation von clonogenen Zellen in Tumoren ausübt und eine wirksame Menge einer Verbindung enthält, ausgewählt aus den Verbindungen gemäß einem der Ansprüche 17 und 23.

## Claims

1. Use of a compound selected from among the compounds of formula:

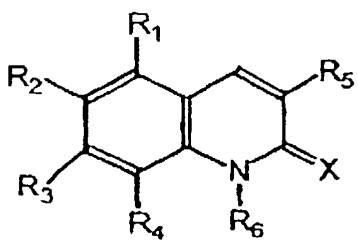 (I) and 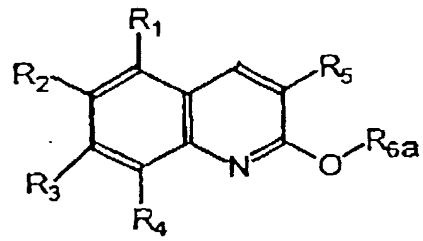 (Ia)

wherein:

X is selected from among =O, =S and =N-NH-$R_7$, where $R_7$ is a phenyl or pyridinyl group,
$R_1$, $R_2$, $R_3$ and $R_4$ are selected independently of one another from among H, OH, a $C_{1-4}$ alkyl group, a $C_{1-4}$ alkoxy group, an -OCO-$R_8$ group, where $R_8$ is a $C_{1-4}$ alkyl group, at least one of the substituents $R_1$, $R_2$, $R_3$ or $R_4$ being other than H, and $R_2$ and $R_3$ together may form a methylenedioxy group, $R_5$ is a phenyl group or a phenyl group mono- to tri- substituted by groups selected from among H, OH, a $C_{1-4}$ alkoxy group, an -OCOR$_8$ group, a phenyl ($C_{1-4}$ alkoxy) group, an -O-$SO_2$-R'$_8$ group, where R'$_8$ is a $C_{1-4}$ alkyl group or a $CF_3$ group,
$R_6$ is selected from among H, a $C_{1-4}$ alkyl group, a -CO-$R_9$ group and an -A-$R_{10}$ group,
$R_{63}$ is selected from among a $C_{1-4}$ alkyl group, a -C-O-$R_9$ group and an -A-$R_{10}$ group,
$R_9$ being a $C_{1-4}$ alkyl group,
A being a $C_{1-4}$ alkylene group,
$R_{10}$ being selected from among the 5- or 6- membered heterocyclic groups having 1 to 4 heteroatoms selected from among oxygen, sulphur and nitrogen, the group CN, a group -COOR$_{11,}$ -CONR$_{12}$R$_{13}$, a group -NR$_{14}$R$_{15}$ and a group -COR$_{16}$,
$R_{11}$, $R_{12}$, $R_{13}$, $R_{14}$, $R_{15}$ and $R_{16}$ being selected independently from among a hydrogen atom, a $C_{1-4}$ alkyl group and a phenyl ($C_{1-4}$ alkyl) group, while $R_4$ and $R_6$ may together also form a group -CO-$CH_2$-$CH_2$-, for the manufacture of a drug intended to interfere with the production of clonogenic cells in tumours during treatment of these tumours with at least one cytotoxic agent.

2. Use according to claim 1, wherein the compound is a compound of formula I wherein:

$R_1$ is a $C_{1-4}$ alkoxy group
$R_2$ is a hydrogen atom
$R_3$ is a $C_{1-4}$ alkoxy group
$R_4$ is a hydrogen arom.

3. Use according to claim 2, wherein the compound is a compound of formula I wherein:

$R_5$ is a 4($C_{1-4}$ alkoxy) phenyl group.

4. Use according to claim 3, wherein:

$R_1$ is a methoxy group,
$R_3$ is a methoxy group, and
$R_5$ is a methoxy phenyl group.

5. Use according to claim 4, wherein the compound is 5,7-dimethoxy-3-(4-methoxyphenyl)-1,2-dihydro-2-quinolinone,

6. Use according to claim 4, wherein the compound is 3-[5,7-dimethoxy-3-(4-methoxyphenyl)-2-oxo-1,2-dihydro-1-quinolinyl]propane nitrile.

7. Use according to claim 4, wherein the compound is 1-[2-(1H-1)2,3,4-tetrazol-5-yl)ethyl]-5,7-dimethoxy-3-(4-methoxyphenyl)-1,2-dihydro-2-quinolinone.

8. Use according to claim 4, wherein the compound is N,N-diethyl-3-[5,7-dimethoxy-3-(4-methoxyphenyl)-2-oxo-1,2-dihydro-1-quinolinyl]-propanamide.

9. Pharmaceutical composition having an effect on the proliferation of clonogenic cells in rumours and comprising an effective amount of a compound selected from among the compounds of formula:

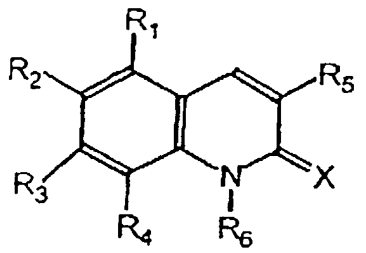

(I) and

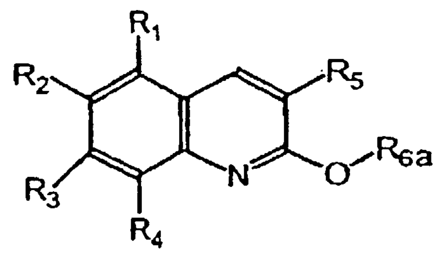

(Ia)

wherein:

X is selected from among =O, =S and =N-NH-$R_7$, where $R_7$ is a phenyl or pyridinyl group,
$R_1$, $R_2$, $R_3$ and $R_4$ are selected independently of one another from among H, OH, a $C_{1-4}$ alkyl group, a $C_{1-4}$ alkoxy group, an -OCO-$R_8$ group, where $R_8$ is a $C_{1-4}$ alkyl group, at least one of the substituents $R_1$, $R_2$, $R_3$ or $R_4$ being other than H, and $R_2$ and $R_3$ together may form a methylenedioxy group,
$R_5$ is a phenyl group or a phenyl group mono- to tri- substituted by groups selected from among H, OH, a $C_{1-4}$ alkoxy group, an -OCOR$_8$ group, a phenyl ($C_{1-4}$ alkoxy) group, an -O-$SO_2$-$R'_8$ group, where $R^1_8$ is a $C_{1-4}$alkyl group or a $CF_3$ group,
$R_6$ is selected from among H, a $C_{1-4}$ alkyl group, a -CO-$R_9$ group and an -A-$R_{10}$ group;
$R_{6a}$ is selected from among a -C-O-$R_9$ group and an -A-$R_{10}$ group,
$R_9$ being a $C_{1-4}$alkyl group,
A being a $C_{1-4}$ alkylene group,
$R_{10}$ being selected from among the 5- or 6- membered heterocyclic groups having 1 to 4 heteroatoms selected from among oxygen, sulphur and nitrogen, the group CN, a group -COOR$_{11}$, -CONR$_{12}$R$_{13}$, a group -NR$_{14}$R$_{15}$ and a group -COR)$_{16}$,
$R_{11}$, $R_{12}$, $R_{13}$, $R_{14}$, $R_{15}$ and $R_{16}$ being selected independently from among a hydrogen atom, a $C_{1-4}$ alkyl group and a phenyl ($C_{1-4}$ alkyl) group, while $R_4$ and $R_6$ may together also form a group -CO-$CH_2$-$CH_2$-.

10. Composition according to claim 9, wherein the compound is a compound of formula I wherein:

$R_1$ is a $C_{1-4}$ alkoxy group
$R_2$ is a hydrogen atom
$R_3$ is a $C_{1-4}$ alkoxy group
$R_4$ is a hydrogen atom.

11. Composition according to claim 10, wherein the compound is a compound of formula I wherein:

$R_5$ is a 4($C_{1-4}$ alkoxy) phenyl group.

12. Composition according to claim 11, wherein the compound is a compound of formula I, $R_1$ is a methoxy group, $R_3$ is a methoxy group and $R_5$ is a 4-methoxyphenyl group.

13. Composition according to claim 12, wherein the compound is 5,7-dimethoxy-3-(4-methoxyphenyl)-1,2-dihydro-2-quinolinone.

14. Composition according to claim 12, wherein the compound is 3-[5,7-dimethoxy-3-(4-methoxyphenyl)-2-oxo-1,2-dihydro-1-quinolmyl]propane nitrile.

**15.** Composition according to claim 12, wherein the compound is 1-[2-(1H-1,2,3,4-tetrazol-5-yl)ethyl]-5,7-dimethoxy-3-(4-methoxyphenyl)-1,2-dihydro-2-quinolinone.

**16.** Composition according to claim 12, wherein the compound is N,N-diethyl-3-[5,7-dimethoxy-3-(4-methoxyphenyl)-2-oxo-1,2-dihydro-1-quinolinyl]-propanamide.

**17.** Compounds of formula:

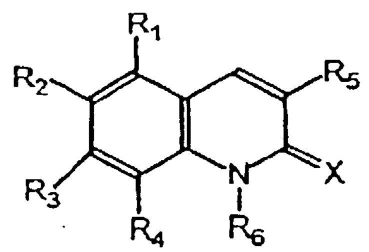

(I) and

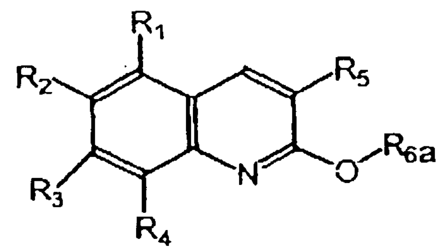

(Ia)

wherein:

X is selected from among =O, =S and =N-NH-$R_7$, where $R_7$ is a phenyl or pyridinyl group,
$R_1$, $R_2$, $R_3$ and $R_4$ are selected independently of one another from among H, OH, a $C_{1-4}$ alkyl group, a $C_{1-4}$ alkoxy group, an -OCO-$R_8$ group, where $R_8$ is a $C_{1-4}$ alkyl group, at least one of the substituents $R_1$, $R_2$, $R_3$ or $R_4$ being other than H, and $R_2$ and $R_3$ together may form a methylenedioxy group,
$R_5$ is a phenyl group mono- to tri- substituted by groups selected from among OH, a $C_{1-4}$ alkoxy group, an -OCO$R_8$ group, a phenyl ($C_{1-4}$ alkoxy) group, an -O-$SO_2$-$R'_8$ group, where $R'_8$ is a $C_{1-4}$ alkyl group or a $CF_3$ group,
$R_6$ is selected from among H, a $C_{1-4}$ alkyl group, a -CO-$R_9$ group and an -A-$R_{10}$ group,
$R_{6a}$ is selected from among a -C-O-$R_9$ group and an -A-$R_{10}$ group,
$R_9$ being a $C_{1-4}$ alkyl group,
A being a $C_{1-4}$ alkylene group,
$R_{10}$ being selected from among the 5- or 6- membered heterocyclic groups having 1 to 4 heteroatoms selected from among oxygen, sulphur and nitrogen, the group CN, a group -COO$R_{11}$, -CON$R_{12}R_{13}$, a group -N$R_{14}R_{15}$ and a group -CO$R_{16}$,
$R_{11}$, $R_{12}$, $R_{13}$, $R_{14}$, $R_{15}$ and $R_{16}$ being selected independently from among a hydrogen atom, a $C_{1-4}$ alkyl group and a phenyl ($C_{1-4}$ alkyl) group, while $R_4$ and $R_6$ may together also form a group -CO-$CH_2$-$CH_2$-.

**18.** Compound according to claim 17 which is 3-[5,7-dimethoxy-3-(4-methoxyphenyl)-2-oxo-1,2-dihydro-1-quinolinyl] propane nitrile.

**19.** Compound according to claim 17 which is 1-[2-(1H-1,2,3,4-tetrazol-5-yl)ethyl]-5,7-dimethoxy-3-(4-methoxyphenyl)-1,2-dihydro-2-quinolinone.

**20.** Compound according to claim 17 which is N,N-diethyl-3-[5,7-dimethoxy-3-(4-methoxyphenyl)-2-oxo-1,2-dihydro-1-quinolinyl]-propanamide.

**21.** Compounds of formula:

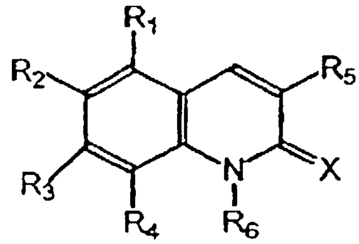

(I)

wherein:

X is selected from among =O, =S and =N-NH-$R_7$, where $R_7$ is a phenyl or pyridinyl group,
$R_1$, $R_2$, $R_3$ and $R_4$ are selected independendy of one another from among H, OH, a $C_{1-4}$ alkyl group, a $C_{1-4}$ alkoxy group, an -OCO-$R_6$ group, where $R_8$ is a $C_{1-4}$ alkyl group, at least one of the substituents $R_1$, $R_2$, $R_3$ or $R_4$ being other than H, and $R_2$ and $R_3$ together may form a methylenedioxy group,
$R_5$ is a phenyl group or a phenyl group mono- to tri- substituted by groups selected from among H, OH, a $C_{1-4}$ alkoxy group, an -OCOR$_6$ group, a phenyl ($C_{1-4}$ alkoxy) group, an -O-$SO_2$-R'$_8$ group, where R'$_8$ is a $C_{1-4}$ alkyl group or a $CF_3$ group,
$R_6$ is selected from among an -A-$R_{10}$ group,
A being a $C_{1-4}$ alkylene group,
$R_{10}$ being selected from among the 5- or 6- membered heterocyclic groups having 1 to 4 heteroatoms selected from among oxygen, sulphur and nitrogen, the group CN, a group -COOR$_{11}$, -CONR$_{12}$R$_{13}$, a group -NR$_{14}$R$_{15}$ and a group -COR$_{16}$,
$R_{11}$, $R_{12}$, $R_{13}$, $R_{14}$, $R_{15}$ and $R_{16}$ being selected independently from among a hydrogen atom, a $C_{1-4}$ alkyl group and a phenyl ($C_{1-4}$ alkyl) group.

**22.** Compounds of formula:

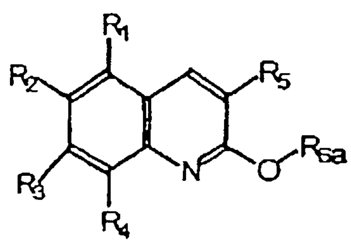

(Ia)

wherein:

$R_1$, $R_2$, $R_3$ and $R_4$ are selected independently of one another from among H, OH, a $C_{1-4}$ alkyl group, a $C_{1-4}$ alkoxy group, an -OCO-$R_8$ group, where $R_8$ is a $C_{1-4}$ alkyl group, at least one of the substituents $R_1$, $R_2$, $R_3$ or $R_4$ being other than H, and $R_2$ and $R_3$ together may form a methylenedioxy group,
$R_6$ is a phenyl group or a phenyl group mono- to tri- substituted by groups selected from among H, OH, a $C_{1-4}$ alkoxy group, an -OCOR$_8$ group, a phenyl ($C_{1-4}$ alkoxy) group, an -O-$SO_2$-R'$_8$ group, where R'$_6$ is a $C_{1-4}$ alkyl group or a $CF_3$ group,
$R_{6a}$ is selected from among a -C-O-$R_9$ group and an -A-$R_{10}$ group,
$R_9$ being a $C_{1-4}$ alkyl group,
A being a $C_{1-4}$ alkylene group,
$R_{10}$ being selected from among the 5- or 6- membered heterocyclic groups having 1 to 4 heteroatoms selected from among oxygen, sulphur and nitrogen, the group CN, a group -COOR$_{11}$, -CONR$_{12}$R$_{13}$, a group -NR$_{14}$R$_{15}$ and a group -COR$_{16}$,
$R_{11}$, $R_{12}$, $R_{13}$, $R_{14}$, $R_{15}$ and $R_{16}$ being selected independently from among a hydrogen atom, a $C_{1-4}$ alkyl group and a phenyl ($C_{1-4}$ alkyl) group.

**23.** Compound according to claim 17, **characterised in that** $R_6$ is selected from among a $C_{1-4}$ alkyl group, a -CO-$R_9$

group and an -A-$R_{10}$ group,

$R_9$ being defined as in claim 17,
$R_{10}$ being selected from among the 5- or 6- membered heterocyclic groups having 1 to 4 heteroatoms selected from among oxygen, sulphur and nitrogen, the group CN, a group -$COOR_{11}$, -$CONR_{12}R_{13}$, a group -$NR_{14}R_{15}$ and a group -$COR_{16}$,
$R_{11}$ being a phenyl ($C_{1-4}$ alkyl) group,
$R_{12}$, $R_{13}$, $R_{14}$, $R_{15}$ and $R_{16}$ being selected independently from among a hydrogen atom, a $C_{1-4}$ alkyl group and a phenyl ($C_{1-4}$ alkyl) group.

**24.** Pharmaceutical composition having an effect on the proliferation of clonogenic cells in tumours and comprising an effective amount of a compound selected from among the compounds according to any one of claims 17 and 23.